# EUROPEAN PATENT APPLICATION

(11) **EP 4 450 494 A1**
(43) Date of publication of application: **23.10.2024**
(21) Application number: 23382368.1
(22) Date of filing: 20.04.2023
(51) Int. Cl.: C07D 401/12, C07D 403/12, A61K 31/496, A61K 31/454, A61K 31/404, A61P 35/00

(54) **INDOLE DERIVATIVES AS HISTONE DEACETYLASE (HDAC) INHIBITORS FOR THE TREATMENT OF CANCER**

(71) Applicant: Consejo Superior De Investigaciones Científicas, 28006 Madrid (ES); Universidad de Alcalá, 28801 Alcala de Henares (Madrid) (ES); Universidad Camilo José Cela, 28692 Villanueva de la Cañada (ES); Administración General De La Communidad Autónoma De Euskadi, 01010 Vitoria-Gateiz (Álava) (ES)
(72) Inventor: MARCO CONTELLES, Josè Luis, Madrid (ES); TOLEDANO PINEDO, Mireira, Madrid (ES); PORRO PÉREZ, Alicia, Madrid (ES); ALMENDROS REQUENA, Pedro, Madrid (ES); DIEZ IRIEPA, Daniel, Alcalá de Henares, Madrid (ES); IRIEPA CANALDA, Isabel, Alcalá de Henares, Madrid (ES); LÓPEZ MUÑOZ, Francisco, Madrid (ES); MATHEU FERNÁNDEZ, Ander, 01010 San Sebastián (ES); ARTETXE ZURUTUZA, Aizpea, 01010 San Sebastián (ES); ITURRIOZ RODRÍGUEZ, Nerea, 01010 San Sebastián (ES); MONCHO AMOR, Verónica, 01010 San Sebastián (ES); SAMPRÓN LEBED, Nicolas, 01010 San Sebastián (ES); CARRASCO GARCÍA, Estefania, 01010 San Sebastián (ES)
(74) Representative: Pons

(57) **Abstract**

Indole derivatives of the formulae I to V: 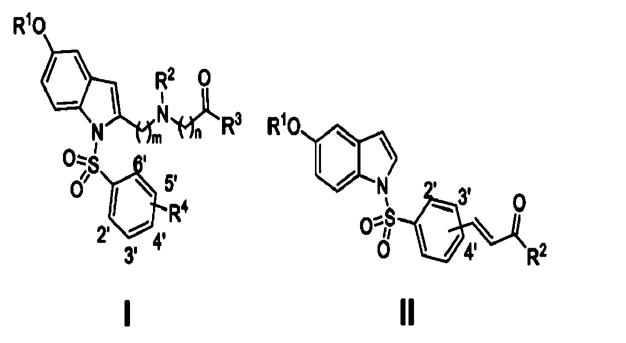 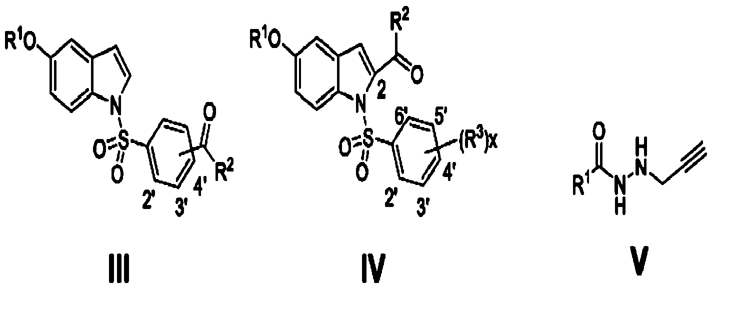 as histone deacetylase (HDAC) inhibitors for the treatment of cancer.

## Description

The present invention relates to a new series of histone deacetylase derivatives, as well as to pharmaceutical compositions comprising them and to their use in therapy, particularly to their use for the treatment of cancer.

### BACKGROUND ART

Histone deacetylases (HDACs) are enzymes that remove acetyl groups from lysine residues in histone and other non-histone substrates and trigger the transcription of transcriptionally silent chromatin. Eighteen HDACs have been identified and are divided into four groups according to phylogenetic sequence and function: class I (HDACs 1, 2, 3, and 8), class Ila (HDACs 4, 5, 7, and 9), class IIb (HDACs 6 and 10), and class IV (HDAC11). Since the epigenetic modification is considered a new promising frontier for the study and treatment of AD (Hwang, J. Y. et al. Nat. Rev. Neurosci. 2017, 18, 347), the interest in regulating histone acetylation through HDAC is rising up. The emerging role of HDAC as AD target is also supported by the evidence of the role of histone acetylation in rescuing learning and memory impairment. Among all the HDAC, particularly, it is worth noting that elevated HDAC6 activity increases tau phosphorylation interfering with its propensity to aggregate, and that the HDAC6 selective inhibitor tubacin attenuates tau phosphorylation. Fan et al. demonstrated that HDAC6 inhibition ameliorates tau phosphorylation and cognitive deficits in an AD model (Zhang, L. et al. Enzyme Inhib. Med. Chem. 2018, 33, 714***).*** The biological functions of HDACs have an extraordinary impact in diseases like cancer (Ellis, L.; Pili, R. Parmaceuticals 2010, 3, 2441; Stimson, L. et al. Ann. Oncology 2009, 20, 1293), central nervous system disorders (cf. Kazantsev, A. G.; Thomson, L. N. Nat. Rev. Drug Discov. 2008, 7, 854), inflammation and immunity (cf. Shakespear, M. R. et al. Trends Immunol. 2011, 32, 335) and HIV-1 latency (cf. Archin, N. M. et al. Nature 2012, 487, 482). Within this context, HDAC inhibitors (HDACi) have emerged as very relevant and promising drugs for the treatment of cancer (cf. Johnstone, R. W. Nat. Rev. Drug. Discov. 2002, 1, 287; Dokmanovic, M. et al. Mol. Cancer Res. 2007, 5, 981; Ropero, S.; Esteller, M. Mol. Oncology 2007, 1, 19). Although many of the actual mechanisms of anticancer activity of HDACi are not completely understood, there is evidence that these compounds alter the biological machinery affecting the hallmarks of cancer like apoptosis (HDAC 1 and 2) differentiation (HDAC 3, 4, 5 and 8), angiogenesis (HDAC 4, 6, 7 and 10), migration (HDAC 6), resistance to chemotherapy (HDAC 1) and proliferation (HDAC 1, 2, 3 and 8) (cf. Witt, O. et al. Cancer Lett. 2009, 277, 8).

Vorinostat (suberoylanilide hydroxamic acid, SAHA) was the first HDACi approved in 2006 by the US FDA for the treatment of cutaneous T-cell lymphoma (CTCL) (cf. Marks, P. A. Oncogene 2007, 26, 1351; Marks, P. A.; Breslow, R. Nat. Biotechnol. 2007, 25, 2084). In 2009, disulfide FK228 gained approval by the same American agency (cf. Grant, C. et al. Expert Rev. Anticancer Ther. 2010, 10, 997*;* Bertino, E. M. et al. Expert Opin. Investig. Drugs 2011, 20, 1151). Nowadays, many HDACi have been prepared, some of them being in clinical development (cf. Miller, T. A. et al. J. Med. Chem. 2003, 46, 5097; Paris, M. et al. J. Med. Chem. 2008, 51, 1505).

Belinostat is the first of four FDA-approved HDAC inhibitors for the treatment of relapsed/refractory peripheral T-cell lymphoma (Poole, R.M. Drugs 2014, 74, 1543). Moreover, HDAC inhibitors (including belinostat) have been found to augment the response to PD-1 immunotherapy in lung adenocarcinoma (Zheng, H. et al. Clin. Cancer Res. 2016, 22, 4119) and melanoma (Woods, D.M. et al. Cancer Immunol. Res. 2015, 3, 1375), as stand-alone agents or in combination with immunotherapeutic approaches (Banik, D. et al. Int. J. Mol. Sci. 2019, 20, 2241). However, belinostat and other HDAC inhibitors have very limited therapeutic outcome for the treatment of nonhematological cancers in completed clinical trials (Mottamal, M. et al. Molecules 2015, 20, 3898).

Tubastatin A is a highly selective HDAC6 inhibitor able to suppress the degeneration of cultivated neurons of cerebral cortex under conditions of the oxidative stress (Butler, K.V. et al. J. Am. Chem. Soc. 2010, 132, 10842).

Considering the above, it would be desirable to have new compounds for the treatment of cancer that have improved efficacy and do not have side effects.

### SUMMARY OF THE INVENTION

A first aspect of the invention relates to a compound of formula **I:** 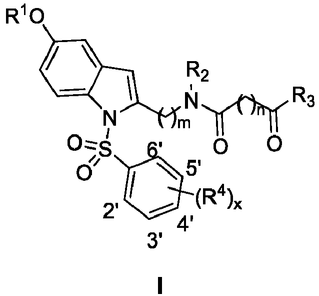 or a pharmaceutical salt thereof, wherein:
R¹ represents H, -C₁₋₄ alkyl, phenyl, benzyl, or Cy₁(C₂₋₄ alkyl);
Cy₁ represents a 5- to 8-membered saturated ring, which contains from 1 to 3 heteroatoms selected from N, O, Se and S; wherein Cy₁ is attached to rest of the molecule through any C or N atom available, and wherein Cy₁ is optionally substituted by one R⁵ group;
R² represents H, Me, CH₂C≡CH, CH₂CH=C=CH₂ or CH₂CH=CH₂;
R³ represents OH, -O C₁₋₄ alkyl, NHOH, NHNR⁶R⁷ or *orto*-NH₂(C₆H₄)NH;
R⁴ represents H, C₁-C₅ alkyl, -O-C₁-C₅ alkyl, -OH, -NO₂ halogen, CN, CO₂Et or SO₂Ph, wherein R⁴ is located in any available position C2', C3', C4', C5', C6';
R⁵ represents H or -C₁₋₆ alkyl;
R⁶ represents H, -OH or -C₁₋₆ alkyl, preferably H or -C₁₋₄ alkynyl;
R⁷ represents H, -OH or -C₁₋₆ alkyl;
x represents 1, 2, 3 and/or 4;
n represents 2, 3, 4, 5 or 6; and
m represents 0, 1 or 2.

In another embodiment the invention relates to the compound of formula **I** as defined above, wherein R¹ is CH₃, -CH₂C₆H₅-CH₂, N-piperidinepropoxy or N-(propargyl)piperazinepropoxy.

In another embodiment the invention relates to the compound of formula I as defined above, wherein R³ is NHOH or *orto*-NH₂(C₆H₄)NH.

In another embodiment the invention relates to the compound of formula **I** as defined above, wherein each R⁶ and/or R⁷ are independently H.

In another embodiment the invention relates to the compound of formula **I** as defined above, wherein:
R¹ is CH₃ or -CH₂C₆H₅-CH₂; and
R³ is NHOH or *orto*-NH₂(C₆H₄)NH.

In another embodiment the invention relates to the compound of formula **I** as defined above, wherein:
R¹ is a *N*-piperidinepropoxy or *N*-(propargyl)piperazinepropoxy; and
R³ is NHOH or *orto*-NH₂(C₆H₄)NH.

Another aspect of the invention relates to a compound of formula **II:** 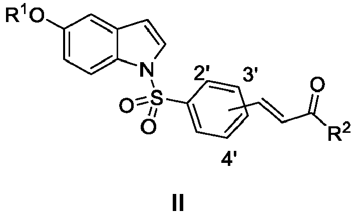 or a pharmaceutical salt thereof, wherein:
R¹ represents -C₁₋₄ alkyl, phenyl, benzyl, or Cy₁(C₂₋₄ alkyl);
Cy₁ represents a 5- to 8-membered saturated ring, which contains from 1 to 3 heteroatoms selected from N, O, Se and S; wherein Cy₁ is attached to rest of the molecule through any C or N atom available, and wherein Cy₁ is optionally substituted by one R³ group;
R² represents -OH, -OC₁₋₄ alkyl, -NHOH, -NHNR⁴R⁵ or *orto*-NH₂(C₆H₄)NH group installed in the α,β-unsaturated group located in any available position C2', C3' or C4' of the molecule;
R³ represents H or -C₁₋₄ alkynyl;
R⁴ represents H, -OH or -C₁₋₆ alkyl, preferably R⁴ represents H or -C₁₋₆ alkyl; and
R⁵ independently represents H or -C₁₋₆ alkyl.

In another embodiment the invention relates to the compound of formula **II** as defined above, wherein R¹ is CH₃, -CH₂C₆H₅-CH₂, N-piperidinepropoxy or N-(propargyl)piperazinepropoxy.

In another embodiment the invention relates to the compound of formula **II** as defined above, wherein R² is NHOH or *orto*-NH₂(C₆H₄)NH, wherein R² is installed in the α,β-unsaturated group located in position C3' of the molecule.

In another embodiment the invention relates to the compound of formula **II** as defined above, wherein:
R¹ is CH₃ or -CH₂C₆H₅-CH₂; and
R² is NHOH or *orto*-NH₂(C₆H₄)NH, wherein R² is installed in the α,β-unsaturated group located in position C3' of the molecule.

In another embodiment the invention relates to the compound of formula **II** as defined above, wherein:
R¹ is *N*-piperidinepropoxy or *N*-(propargyl)piperazinepropoxy; and
R² is NHOH or *orto*-NH₂(C₆H₄)NH, wherein R² is installed in the α,β-unsaturated group located in position C3' of the molecule.

Another aspect of the invention relates to a compound of formula **III:** 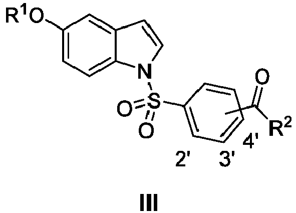 or a pharmaceutical salt thereof, wherein:
R¹ represents -C₁₋₄ alkyl, phenyl, benzyl, or Cy₁(C₂₋₄ alkyl);
Cy₁ represents a 5 to 8-membered saturated ring, which contains from 1 to 3 heteroatoms selected from N, O, Se and S; wherein Cy₁ is attached to rest of the molecule through any C or N atom available, and wherein Cy₁ is optionally substituted by one R³ group;
R² represents -OH, -O C₁₋₄ alkyl, NHOH, NHNR⁴R⁵ or *orto*-NH₂(C₆H₄)NH group installed in the carboxylate group located in any available position C2', C3' or C4' of the molecule;
R³ represents H or -C₁₋₄ alkynyl;
R⁴ represents H, -OH or -C₁₋₆ alkyl, preferably R⁴ represents H or -C₁₋₆ alkyl; and
R⁵ represents H or -C₁₋₆ alkyl.

In another embodiment the invention relates to the compound of formula **III** as defined above, wherein R¹ is CH₃, -CH₂C₆H₅-CH₂, *N*-piperidinepropoxy or *N-*(propargyl)piperazinepropoxy.

In another embodiment the invention relates to the compound of formula **III** as defined above, wherein R² is NHOH or NHNH₂, wherein R² is installed in the carboxylate group located in position C4' of the molecule.

In another embodiment the invention relates to the compound of formula **III** as defined above, wherein:
R¹ is CH₃ or -CH₂C₆H₅-CH₂; and
R² is NHOH or NHNH₂, wherein R² is installed in the carboxylate group located in position C4' of the molecule.

In another embodiment the invention relates to the compound of formula **III** as defined above, wherein:
R¹ is a *N*-piperidinepropoxy or *N*-(propargyl)piperazinepropoxy; and
R² is NHOH or NHNH₂, wherein R² is installed in the carboxylate group located in position C4' of the molecule.

Another aspect of the invention relates to a compound of formula **IV:** 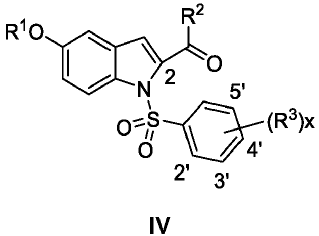 or a pharmaceutical salt thereof, wherein:
R¹ represents -C₁₋₄ alkyl, phenyl, benzyl, or Cy₁(C₂₋₄ alkyl);
Cy₁ represents a 5 to 8-membered saturated ring, which contains from 1 to 3 heteroatoms selected from N, O, Se and S; Cy₁ wherein Cy₁ is attached to rest of the molecule through any C or N atom available, and wherein Cy₁ is optionally substituted by one R⁴ group;
R² represents OH, -OC₁₋₄ alkyl, NHOH, NHNR⁵R⁶ or *orto*-NH₂(C₆H₄)NH group installed in the carboxylic group located in position C2' of the molecule;
each R³ independently represents H; halogen, Me, -C₃H₇, -OMe or -CF₃; or
two R³ groups form a fused benzene ring resulting in a 1-naphthyl or 2-naphthyl group; x represents 1, 2, 3 and/or 4;
R⁴ represents H or -C₁₋₄ alkynyl;
R⁵ represents H or -C₁₋₄ alkynyl; and
R⁶ represents H, -OH or -C₁₋₆ alkyl, preferably R⁶ independently represents H or -C₁₋₆ alkyl.

In another embodiment the invention relates to the compound of formula **IV** as defined above, wherein R¹ is CH₂C₆H₅, N-piperidinepropoxy or *N-*(propargyl)piperazinepropoxy

In another embodiment the invention relates to the compound of formula **IV** as defined above, wherein R² is NHOH, NHNH₂, and *orto*-NH₂(C₆H₄)NH.

In another embodiment the invention relates to the compound of formula **IV** as defined above, wherein each R⁵ and/or R⁶ are independently H.

In another embodiment the invention relates to the compound of formula **IV** as defined above, wherein:
R¹ is CH₂C₆H₅;
R² is NHOH, NHNH₂, and *orto*-NH₂(C₆H₄)NH; and
each R⁵ and/or R⁶ are independently H.

In another embodiment the invention relates to the compound of formula **IV** as defined above, wherein:
R¹ is a *N*-piperidinepropoxy or *N*-(propargyl)piperazinepropoxy; and
R² is NHOH; and
each R⁵ and/or R⁶ are independently H.

Another aspect of the invention relates to a compound of formula **V:** 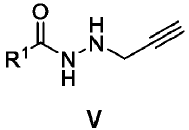 or a pharmaceutical salt thereof, wherein:
R¹ represents 5-hydroxy-1*H*-indol-2-yl, 5-methoxy-1*H*-indol-2-yl, 5-benzyloxy-1*H-*indol-2-yl, 5-(3-(piperidin-1-yl)propoxy)-1*H-*indol-2-yl, 5-(3-(4-(prop-2-yn-1-yl)piperazin-1-yl)propoxy)-1*H*-indol2-yl; C₆H₅, 2'-BrC₆H₄, 3'-BrC₆H₄, 4'-BrC₆H₄; 2'-ClC₆H₄, 3'-ClC₆H₄, 4'-ClC₆H₄; 2'-FC₆H₄, 3'- FC₆H₄, 4'-FC₆H₄; 2'-IC₆H₄, 3'-IC₆H₄, 4'-IC₆H₄; 2'-MeC₆H₄, 3'-MeC₆H₄, 4'-MeC₆H₄; 2'-C₂H₅C₆H₄, 3'-C₂H₅C₆H₄, 4'-C₂H₅C₆H₄; 2'-C₃H₇C₆H₄, 3'-C₃H₇C₆H₄, 4'-C₃H₇C₆H₄; 2'-OMeC₆H₄, 3'-OMeC₆H₄, 4'-OMeC₆H₄; 2'-OHC₆H₄, 3'-OHC₆H₄, 4'-OHC₆H₄; 2',5'-di-FC₆H₃, 3',5'-di-FC₆H₃; 2'-CF₃C₆H₄, 3'-CF₃C₆H₄, 4'-CF₃ C₆H₄; 1-naphthyl, 2-naphthyl; furan, pyrrole, tiophene; pyridine, pyrimidine, pyrazine, C₆H₅NHCO(CH₂)ₙ, 2'-BrC₆H₄NHCO(CH₂)ₙ, 3'-BrC₆H₄NHCO(CH₂)n, 4'-BrC₆H₄ NHCO(CH₂)ₙ; 2'-ClC₆H₄NHCO(CH₂)ₙ, 3'-ClC₆H₄NHCO(CH₂)ₙ, 4'-ClC₆H₄NHCO(CH₂)ₙ; 2'-FC₆H₄NHCO(CH₂)ₙ, 3'-FC₆H₄NHCO(CH₂)ₙ, 4'-FC₆H₄NHCO(CH₂)ₙ; 2'-IC₆H₄NHCO(CH₂)ₙ, 3'-IC₆H₄NHCO(CH₂)ₙ, 4'-IC₆H₄NHCO(CH₂)ₙ; 2'-MeC₆H₄NHCO(CH₂)ₙ, 3'-MeC₆H₄NHCO(CH₂)ₙ, 4'-MeC₆H₄NHCO(CH₂)ₙ; 2'-C₂H₅C₆H₄NHCO(CH₂)ₙ, 3'-C₂H₅C₆H₄NHCO(CH₂)ₙ, 4'-C₂H₅C₆H₄NHCO(CH₂)ₙ; 2'-C₃H₇C₆H₄NHCO(CH₂)ₙ, 3'-C₃H₇C₆H₄NHCO(CH₂)ₙ, 4'-C₃H₇C₆H₄NHCO(CH₂)ₙ; 2'-OMeC₆H₄NHCO(CH₂)ₙ, 3'-OMeC₆H₄NHCO(CH₂)ₙ, 4'-OMeC₆H₄NHCO(CH₂)ₙ; 2'-OHC₆H₄NHCO(CH₂)ₙ, 3'-OHC₆H₄NHCO(CH₂)ₙ, 4'-OHC₆H₄NHCO(CH₂)ₙ; 2',5'-di-FC₆H₃NHCO(CH₂)₁, 3',5'-di-FC₆H₃NHCO(CH₂)ₙ; 2'-CF₃C₆H₄NHCO(CH₂)ₙ, 3'-CF₃C₆H₄NHCO(CH₂)ₙ, 4'-CF₃C₆H₄ NHCO(CH₂)ₙ; 1-naphthylNHCO(CH₂)ₙ, 2-naphthyl NHCO(CH₂)ₙ; furanNHCO(CH₂)ₙ, pyrroleNHCO(CH₂)ₙ, tiophenNHCO(CH₂)ₙ; pyridineNHCO(CH₂)ₙ, pyrimidine NHCO(CH₂)ₙ or pyrazineNHCO(CH₂)ₙ; and n represents 2, 3, 4, 5, 6.

Another aspect of the invention relates to a compound of formula **I, II, III, IV** or **V** selected from:
*N*1-Hydroxy-*N*8-((1-(phenylsulfonyl)-5-(3-(piperidin-1-yl)propoxy)-1*H*-indol-2-yl)methyl)-*N*8-(prop-2-yn-1-yl)octanediamide (**DDI199**);
1-((3-Bromophenyl)sulfonyl)-5-methoxy-1*H*-indole (**MTP21**);
5-(Benzyloxy)-1-((3-bromophenyl)sulfonyl)-1*H*-indole (**MTP12**);
1-((3-Bromophenyl)sulfonyl)-5-(3-(piperidin-1-yl)propoxy)-1*H*-indole (**MTP30**);
1-((3-Bromophenyl)sulfonyl)-5-(3-(4-(prop-2-yn-1-yl)piperazin-1-yl)propoxy)-1*H-*indole (**MTP43**);
Ethyl (*E*)-3-(3-((5-methoxy-1*H*-indol-1-yl)sulfonyl)phenyl)acrylate (**MTP25**);
Ethyl (*E*)-3-(3-((5-(benzyloxy)-1*H*-indol-1-yl)sulfonyl)phenyl)acrylate (**MTP13**);
Ethyl (*E*)-3-(3-((5-(3-(Piperidin-1-yl)propoxy)-1*H*-indol-1-yl)sulfonyl)phenyl)acrylate **(MTP41);**
Ethyl (*E*)-3-(4-((5-Methoxy-1*H*-indol-1-yl)sulfonyl)phenyl)acrylate (**SMD2**);
Ethyl (*E*)-3-(4-((5-(3-(Piperidin-1-yl)propoxy)-1*H*-indol-1-yl)sulfonyl)phenyl)acrylate **(APP13);**
(*E*)-3-(3-((5-Methoxy-1*H*-indol-1-yl)sulfonyl)phenyl)acrylic acid (**MTP37**);
(*E*)-3-(3-((5-(Benzyloxy)-1*H*-indol-1-yl)sulfonyl)phenyl)acrylic acid (**MTP153**);
(*E*)-3-(3-((5-(3-(Piperidin-1-yl)propoxy)-1*H*-indol-1-yl)sulfonyl)phenyl)acrylic acid (**MTP146**);
(*E*)-3-(4-((5-Methoxy-1*H*-indol-1-yl)sulfonyl)phenyl)acrylic acid (**SMD3**);
(*E*)-3-(4-((5-(3-(Piperidin-1-yl)propoxy)-1*H*-indol-1-yl)sulfonyl)phenyl)acrylic acid (**APP15**);
(*E*)-*N*-Hydroxy-3-(3-((5-methoxy-1*H*-indol-1-yl)sulfonyl)phenyl)acrylamide (**MTP142**);
(*E*)-3-(3-((5-(Benzyloxy)-1*H*-indol-1-yl)sulfonyl)phenyl)-*N-*hydroxyacrylamide (**MTP156**);
(*E*)-*N*-Hydroxy-3-(3-((5-(3-(piperidin-1-yl)propoxy)-1*H*-indol-1-yl)sulfonyl)phenyl)acrylamide (**MTP150**);
(*E*)-*N*-Hydroxy-3-(4-((5-methoxy-1*H*-indol-1-yl)sulfonyl)phenyl)acrylamide (**SMD17**);
(*E*)-*N*-Hydroxy-3-(4-((5-(3-(piperidin-1-yl)propoxy)-1*H*-indol-1-yl)sulfonyl)phenyl)acrylamide (**APP19**);
(*E*)-*N*-(2-Aminophenyl)-3-(3-((5-methoxy-1*H*-indol-1-yl)sulfonyl)phenyl)acrylamide **(MTP143);**
(E)-*N*-(2-Aminophenyl)-3-(3-((5-(benzyloxy)-1*H*-indol-1-yl)sulfonyl)phenyl)acrylamide (**MTP157**);
(*E*)-*N*-(2-Aminophenyl)-3-(3-((5-(3-(piperidin-1-yl)propoxy)-1*H*-indol-1-yl)sulfonyl)phenyl)acrylamide (**MTP167**);
(*E*)-*N*-(2-Aminophenyl)-3-(4-((5-methoxy-1-*H*-indol-1-yl)sulfonyl)phenyl)acrylamide **(SMD10);**
(*E*)-*N*-(2-Aminophenyl)-3-(4-((5-(3-(piperidin-1-yl)propoxy)-1*H*-indol-1-yl)sulfonyl)phenyl)acrylamide (**APP17**);
Methyl 4-((5-methoxy-1*H*-indol-1-yl)sulfonyl)benzoate (**MTP21**);
Methyl 4-((5-(benzyloxy)-1*H*-indol-1-yl)sulfonyl)benzoate (**MTP12**);
Methyl 4-((5-(3-(piperidin-1-yl)propoxy)-1*H*-indol-1-yl)sulfonyl)benzoate (**MTP30**);
Methyl 4-((5-(3-(4-(prop-2-yn-1-yl)piperazin-1-yl)propoxy)-1*H*-indol-1-yl)sulfonyl) benzoate (**MTP43**);
4-((5-Methoxy-1*H*-indol-1-yl)sulfonyl)benzohydrazide (**MTP86**);
4-((5-(Benzyloxy)-1*H*-indol-1-yl)sulfonyl)benzohydrazide (**MTP90**);
4-((5-(3-(Piperidin-1-yl)propoxy)-1*H*-indol-1-yl)sulfonyl)benzohydrazide (**MTP96**);
4-((5-(3-(4-(Prop-2-yn-1-yl)piperazin-1-yl)propoxy)-1*H*-indol-1-yl)sulfonyl)benzohydrazide (**MTP99**);
4-((5-Methoxy-1*H*-indol-1-yl)sulfonyl)-N-propylbenzohydrazide (**FRB24**);
4-((5-(3-(Piperidin-1-yl)propoxy)-1*H*-indol-1-yl)sulfonyl)-*N*-propylbenzohydrazide **(FRB44);**
4-((5-(3-(4-(Prop-2-yn-1-yl)piperazin-1-yl)propoxy)-1H-indol-1-yl)sulfonyl)-*N-*propylbenzohydrazide (**FRB56**);
4-((5-Methoxy-1*H*-indol-1-yl)sulfonyl)-*N',N'*-dipropylbenzohydrazide (**FRB21**);
*N*-Hydroxy-4-((5-methoxy-1*H*-indol-1-yl)sulfonyl)benzamide (**MTP89**);
4-((5-(Benzyloxy)-1*H*-indol-1-yl)sulfonyl)-*N*-hydroxybenzamide (**MTP98**);
*N*-Hydroxy-4-((5-(3-(piperidin-1-yl)propoxy)-1*H*-indol-1-yl)sulfonyl)benzamide **(MTP100);**
*N*-Hydroxy-4-((5-(3-(4-(prop-2-yn-1-yl)piperazin-1-yl)propoxy)-1*H*-indol-1-yl) sulfonyl)benzamide (**MTP109**);
4-((5-Methoxy-1*H*-indol-1-yl)sulfonyl)benzoic acid (**MTP105**);
4-((5-(Benzyloxy)-1*H*-indol-1-yl)sulfonyl)benzoic (**MTP148**);
4-((5-(3-(Piperidin-1-yl)propoxy)-1*H*-indol-1-yl)sulfonyl)benzoic acid (**MTP162**);
4-((5-(3-(4-(Prop-2-yn-1-yl)piperazin-1-yl)propoxy)-1*H*-indol-1-yl)sulfonyl)benzoic acid (**MTP194**);
3-((5-(3-(Piperidin-1-yl)propoxy)-1*H*-indol-1-yl)sulfonyl)benzoic acid (**APP35**);
3-((5-(3-(4-(Prop-2-yn-1-yl)piperazin-1-yl)propoxy)-1*H*-indol-1-yl)sulfonyl)benzoic acid (**APP37**);
*N*-(2-Aminophenyl)-4-((5-(benzyloxy)-1*H*-indol-1-yl)sulfonyl)benzamide (**MTP155**);
*N*-(2-Aminophenyl)-4-((5-(3-(piperidin-1-yl)propoxy)-1*H*-indol-1-yl)sulfonyl)benzamide (**MTP165**);
*N*-(2-Aminophenyl)-4-((5-(3-(4-(prop-2-yn-1-yl)piperazin-1-yl)propoxy)-1*H*-indol-1-yl)sulfonyl)benzamide (**MTP185**);
Ethyl 5-(benzyloxy)-1-(phenylsulfonyl)-1*H*-indole-2-carboxylate (**MTP127**);
Ethyl 1-(phenylsulfonyl)-5-(3-(piperidin-1-yl)propoxy)-1*H-*indole-2-carboxylate (**MTP132);**
5-(Benzyloxy)-1-(phenylsulfonyl)-1*H*-indole-2-carbohydrazide (**MTP128**);
1-(Phenylsulfonyl)-5-(3-(piperidin-1-yl)propoxy)-1*H*-indole-2-carbohydrazide **(MTP133);**
1-(Phenylsulfonyl)-5-(3-(4-(prop-2-yn-1-yl)piperazin-1-yl)propoxy)-1*H-*indole-2-carbohydrazide (**MTP137**);
5-(Benzyloxy)-1-(phenylsulfonyl)-1*H*-indole-2-carboxylic acid (**MTP136**);
1-(Phenylsulfonyl)-5-(3-(piperidin-1-yl)propoxy)-1*H*-indole-2-carboxylic acid (**MTP186);**
1-(Phenylsulfonyl)-5-(3-(4-(prop-2-yn-1-yl)piperazin-1-yl)propoxy)-1*H*-indole-2-carboxylic acid (**MTP191**);
5-(Benzyloxy)-*N*-hydroxy-1-(phenylsulfonyl)-1*H* indole-2-carboxamide (**MTP172**);
*N*-(2-Aminophenyl)-5-(benzyloxy)-1-(phenylsulfonyl)-1*H* indole-2-carboxamide **(MTP140);**
*N*-(2-Aminophenyl)-1-(phenylsulfonyl)-5-(3-(piperidin-1-yl)propoxy)-1*H*-indole-2-carboxamide (**MTP209**);
*N*-(2-Aminophenil)-1-(phenylsulfonyl)-5-(3-(4-(prop-2-yn-1-yl)piperazin-1-yl)propoxy)-1*H*-indole-2-carboxamide (**MTP192**);
*N'*-(Prop-2-yn-1-yl)benzohydrazide (**MTP230**);
5-(Benzyloxy)-*N*'-(prop-2-yn-1-yl)-1*H*-indole-2-carbohydrazide (**MTP229**); and
8-Oxo-*N*-phenyl-8-(2-(prop-2-yn-1-yl)hydrazineyl)octanamide (**MTP234**).

Some compounds of the invention may be in the form of salts. There is no limitation on the type of salt that can be used, provided that these are pharmaceutically acceptable when used for therapeutic purposes. The term pharmaceutically acceptable salt refers to those salts which are, according to medical judgment, suitable for use in contact with the tissues of humans and other mammals without undue toxicity, irritation, allergic response, and the like. Pharmaceutically acceptable salts are well known in the art.

Another aspect of the invention relates to a pharmaceutical composition which comprises a compound of formula **I, II, III, IV, V** or a pharmaceutically acceptable salt thereof and one or more pharmaceutically acceptable excipients.

The salts of a compound of formula **I, II, III, IV** or **V** can be obtained during the final isolation and purification of the compounds of the invention or can be prepared by treating a compound of formula **I, II, III, IV** or **V** with a sufficient amount of the desired acid or base to give the salt in a conventional manner. The salts of the compounds of formula **I, II, III, IV** or **V** can be converted into other salts of the compounds of formula **I, II, III, IV** or **V** by ion exchange using ionic exchange resins.

The compounds of formula **I, II, III, IV** or **V** and their salts may differ in some physical properties, but they are equivalent for the purposes of the present invention. All salts of the compounds of formula **I, II, III, IV** or **V** are included within the scope of the invention.

The compounds of the present invention may form complexes with solvents in which they are reacted or from which they are precipitated or crystallized. These complexes are known as solvates. As used herein, the term solvate refers to a complex of variable stoichiometry formed by a solute (a compound of formula **I, II, III, IV** or **V** or a salt thereof) and a solvent. Examples of solvents include pharmaceutically acceptable solvents such as water, ethanol and the like. A complex with water is known as a hydrate. Solvates of compounds of the invention (or salts thereof), including hydrates, are included within the scope of the invention.

The compounds of formula **I, II, III, IV** or **V** may exist in different physical forms, i.e. amorphous and crystalline forms. Moreover, the compounds of the invention may have the ability to crystallize in more than one form, a characteristic which is known as polymorphism. Polymorphs can be distinguished by various physical properties well known in the art such as X-ray diffraction pattern, melting point or solubility. All physical forms of the compounds of formula I, **II, III, IV** or **V,** including all polymorphic forms ("polymorphs") thereof, are included within the scope of the invention.

Another aspect of the invention relates to a compound of formula **I, II, III, IV, V** or a pharmaceutically acceptable salt thereof in the form of a pharmaceutically acceptable pro-drug, polymorph, salt or hydrate of any of the above compounds of formula **I** are included within the present invention.

Another aspect of the invention relates to a compound of formula **I, II, III, IV** or **V** as defined above or to a pharmaceutical salt thereof, for use in therapy.

Another aspect of the invention relates to the compound of formula **I, II, III, IV** or **V** as defined above or to a pharmaceutical salt thereof, for use in the treatment of cancer, preferably for use in the treatment of a cancer selected from glioblastoma, pancreatic, colorectal, lung, skin, gastric , breast, liver, prostate, head, and neck, and more preferably for the treatment of glioblastoma.

Another aspect of the present invention relates to the use of a compound of formula **I, II, III, IV** or **V** as defined above or a pharmaceutically acceptable salt thereof for the manufacture of a medicament for use as a medicament.

Another aspect of the present invention relates to the use of a compound of formula **I, II, III, IV** or **V** as defined above or a pharmaceutically acceptable salt thereof for the manufacture of a medicament for use in the treatment of cancer, preferably for use in the treatment of a cancer selected from glioblastoma, pancreatic, colorectal, lung, skin, gastric , breast, liver, prostate, head, and neck, and more preferably for the treatment of glioblastoma.

Another aspect of the present invention relates to a method of treating a disease in a subject in need thereof, especially a human being, which comprises administering to said subject an effective amount of a compound of formula **I, II, III, IV** or **V** as defined above or a pharmaceutically acceptable salt thereof.

Another aspect of the present invention relates to a method of treating cancer, preferably for treating glioblastoma, in a subject in need thereof, especially a human being, which comprises administering to said subject an effective amount of a compound of formula **I, II, III, IV** or **V** as defined above or a pharmaceutically acceptable salt thereof.

On the other hand, it is noted that any of the compounds mentioned as examples throughout the present invention can be used separately or in combination, particularly as adjuvant therapy administered simultaneously, alternatively, or successively with respect to a first-line therapy suitable for the treatment of cancer, preferably for the treatment of a cancer selected from glioblastoma, pancreatic, colorectal, lung, skin, gastric , breast, liver, prostate, head, and neck, and more preferably for the treatment of glioblastoma.

Another aspect of the invention relates to the use of a compound of formula **I, II, III, IV** or **V** or a pharmaceutical composition thereof as defined above, for identifying and evaluating in a robotic manner other compounds which may be useful as a medicament, preferably for the treatment of cancer, more preferably for the treatment of a cancer selected from glioblastoma, pancreatic, colorectal, lung, skin, gastric , breast, liver, prostate, head, and neck, and even more preferably for the treatment of glioblastoma.

The composition of the present invention can be administered in the form of any pharmaceutical formulation, the nature of which, as it is well known, will depend upon the nature of the active compound and its route of administration. Any route of administration may be used, for example oral, parenteral, nasal, ocular, rectal and topical administration.

The dosage and frequency of doses will depend upon the nature and severity of the disease to be treated, the age, the general condition and body weight of the patient, as well as the composition administered and the route of administration, among other factors.

The compounds of formula I as defined above, can be administered separately or in combination, particularly as adjuvant therapy administered simultaneously, alternatively or successively with respect to a first-line therapy suitable for the treatment of cancer, and preferably for the treatment of glioblastoma.

Therefore, another aspect of the present invention relates to a composition comprising a compound of formula **I, II, III, IV** or **V** as defined above, for use as adjuvant therapy administered simultaneously, alternatively, or successively with respect to a first-line therapy suitable for the treatment of cancer, preferably for the treatment of glioblastoma.

In the above definitions, the term -C₁₋₄alkyl, C₂₋₄ alkyl, or -C₁₋₆ alkyl, as a group or part of a group, means a straight or branched alkyl chain which contains from 1 to 4, from 2 to 4, or from 1 to 6 carbon atoms respectively, and includes, among others, the groups methyl, ethyl, propyl, isopropyl, butyl, isobutyl, sec-butyl, *tert*-butyl, pentyl and hexyl.

A C₂₋₄alkynyl group means straight or branched alkyl chain which contains from 2 to 4 C atoms, and also contains one or two triple bonds. Examples include the groups ethynyl, 1-propynyl, 2-propynyl, 1-butynyl, 2-butynyl, 3-butynyl and 1,3-butadiynyl.

A halogen group or its abbreviation halo means fluoro, chloro, bromo or iodo.

The compounds of the present invention may form complexes with solvents in which they are reacted or from which they are precipitated or crystallized. These complexes are known as solvates. As used herein, the term solvate refers to a complex of variable stoichiometry formed by a solute (a compound of formula **I, II, III, IV, V** or a pharmaceutically acceptable salt) and a solvent. Examples of solvents include pharmaceutically acceptable solvents such as water, ethanol and the like. A complex with water is known as a hydrate. Solvates of compounds of the invention (or salts thereof), including hydrates, are included within the scope of the invention.

Unless otherwise defined, all technical and scientific terms used herein have the same meaning as commonly understood by one of ordinary skilled in the art to which this invention belongs. Methods and materials similar or equivalent to those described herein can be used in the practice of the present invention. Throughout the description and claims the word "comprise" and its variations are not intended to exclude other technical features, additives, components, or steps. Additional objects, advantages and features of the invention will become apparent to those skilled in the art upon examination of the description or may be learned by practice of the invention. The following examples and drawings are provided by way of illustration and are not intended to be limiting of the present invention.

### BRIEF DESCRIPTION OF THE DRAWINGS

**Fig. 1****.** Shows IC50 values (µM) of representative compounds of formula I, II, III and IV for U87 glioblastoma cell line.
**Fig. 2****.** Shows western blot analysis of the expression of HDAC6 and HDAC1, and their main targets acetylated α-tubulin and acetylated histone H3 in U87 cell line, after 48 h of treatment.
**Fig. 3****.** Shows that inhibitor compounds reduce proliferating capacity and induce apoptosis in U87 cell line in a dose-dependent manner. Proliferating capacity of U87 cells represented as PH3 positive cells after 48 h of treatment with increasing concentrations of the compounds of study.
**Fig. 4****.** Shows that inhibitor compounds reduce proliferating capacity and induce apoptosis in U87 cell line in a dose-dependent manner. Capacity of apoptotic induction of the compounds represented as caspase-3 positive cells after 48 h of treatment of U87 cells with increasing concentrations of the compounds of study.
**Fig. 5****.** Shows DDI199 reduces proliferation and induces apoptosis in a dose-dependent manner both in conventional and patient-derived glioblastoma cell lines. Proliferation capacity of conventional (U87 and U251) and patient-derived (GNS166 and GNS179) glioblastoma cell lines, represented as percentage of PH3 positive cells (n≥3).
**Fig. 6****.** Shows DDI199 reduces proliferation and induces apoptosis in a dose-dependent manner both in conventional and patient-derived glioblastoma cell lines. Capacity of DDI199 to induce apoptosis, represented as the percentage of caspase-3 positive cells (n=3).

### Examples

**Synthesis. General Methods.** Reactions were monitored by TLC using precoated silica gel aluminium plates containing a fluorescent indicator (Merck, 5539). Detection was done by UV (254 nm) followed by charring with sulfuric-acetic acid spray, 1% aqueous potassium permanganate solution or 0.5% phosphomolybdic acid in 95% EtOH. Anhydrous Na₂SO₄ was used to dry organic solutions during work-ups and the removal of solvents was carried out under vacuum with a rotary evaporator. Flash column chromatography was performed using silica gel 60 (230-400 mesh, Merck). Melting points were determined on a Kofler block and are uncorrected. IR spectra were obtained on a Perkin-Elmer Spectrum One spectrophotometer. ¹H NMR spectra were recorded with a Varian VXR-200S spectrometer, using tetramethylsilane as internal standard and ¹³C NMR spectra were recorded with a Bruker WP-200-SY. All the assignments for protons and carbons were in agreement with 2D COSY, HSQC, HMBC, and 1D NOESY spectra. Values with (^{*}) can be interchanged. The purity of compounds was checked by elemental analyses, conducted on a Carlo Erba EA 1108 apparatus, and confirmed to be ≥ 95%.

**General Method for the Synthesis of the *N*-Sulfonamides (A).** To a solution of the corresponding indole (1.0 mmol) in anhydrous DMF (6 mL), cooled at 0 °C, sodium hydride (2.0 mmol) was added and the mixture was stirred for 30 min. Then, benzenesulfonyl chloride (1.1 mmol) was added and he mixture was stirred at room temperature (rt) for 24 h. After completion (tic analysis), distilled water (20 mL) was added and the mixture was extracted with AcOEt (3 x 20 mL). The combined organic extracts were washed with water (3 x 20 mL), dried over anhydrous magnesium sulfate and evaporated. The crude product was purified using column chromatography.

**General Method for the Heck Reaction (B).** A mixtute of the appropriate indole (1.0 mmol), tert-butyl actylate (1.2 mmol), triethylamine (1.7 mmol), triphenylphosphine (0.5 mmol), palladium acetate (0.5 mmol), and sodium bicarbonate (1.0 mmol) was heated at 80 °C in DMF (2 mL) for 24 h. Then, the reaction was quenched with H₂O and extracted with CH₂Cl₂ (DCM) (3 x 30 mL). The organic layer was collected and dried over anhydrous MgSO₄. After the removal of MgSO₄ by filtration, the filtrate was concentrated *in vacuo.* The residue was purified by flash chromatography over silica gel.

**General Method for Acid Synthesis (C).** A mixture of the ester (1.0 equiv), 2N KOH (4.0 equiv), THF (10 mL), and ethanol (10 mL) was stirred at rt for 4 h, then it was quenched on crushed ice and made acidic with 37% HCl. Ethyl acetate (20 mL) was added, and the organic layer was separated, washed with brine (3 x 20 mL) and dried over anhydrous MgSO₄. After the removal of MgSO₄ by filtration, the filtrate was concentrated in vacuo. The residue was purified by flash chromatography over silica gel.

**General Method for Amide Synthesis (D).** *N,N*-Diisopropylethylamine (DIPEA) (2.5-3.5 mmol) and 1-[Bis(dimethylamino)methylene]-1*H*-1,2,3-triazolo[4,5-b]pyridinium 3-oxid hexafluorophosphate (HATU) (1.0 mmol) were sequentially added at room temperature to a solution of the corresponding acid (1.0 mmol) in dry DMF (4 mL). The reaction was stirred for 15 min and then hydroxylamine hydrochloride or 1,2-phenylenediamine was added (1.0 mmol). After completion (tic analysis), the crude product was precipitated on a solution of water/brine (4:1) and the solid was purified using column chromatography.

**General Procedure for Alkylation Reaction (E).** A mixture of substituted indole (1.0 equiv), appropriate amine hydrochloride (1.5 equiv) and potassium carbonate (3 equiv) was disolved in CHCl₃ (2.5 mL) and water (1.0 mL). Then, the reaction mixture was stirred at 80 °C for 3 d. After complete reaction (TLC analysis), the mixture was extracted with DCM (3 x 20 mL). Combined organic extracts were washed with sodium bicarbonate (15 mL), dried over anhydrous magnesium sulfate and evaporated. Product was purified using column chromatography.

**General Procedure for Hydroxamic Acid Synthesis (F).** NH₂OH·HCl (25.0 equiv) suspended in MeOH (3 mL) was mixed with KOH (25.0 equiv) in MeOH (10 mL), and the mixture was cooled to 0 °C in an ice bath. The mixture was then filtered into a solution of the substituted indole (1.0 equiv) in MeOH (34 mL) at 0 °C. Next, KOH (4.0 equiv) dissolved in MeOH (3 mL) was added dropwise for 5 min with stirring, and the reaction mixture was then stirred for an additional 24 h at room temperature (rt). After complete reaction (TLC analysis), the residue was concentrated in vacuo and purified by flash chromatography over silica gel.

**General Procedure for *N*-Propyl-hydrazide Synthesis (G).** The substituted hydrazide (1.0 equiv.) was dissolved in 50 mL of methanol. Then propionaldehyde (1.05-1.6 equiv.) was added followed by MgSO₄ (6.9 equiv.). Four hours later, MgSO₄ was filtered off and the residue was concentrated in vacuum. After, to the solution of crude in methanol was added NaBH₃CN (2 equiv) and HCl/Methanol (1:1) for adjusting the mixture to pH 3-4. The reaction was stirred at room temperature for 12 h, the volatile was removed under vacuum and the crude was purified using column chromatography.

**General Procedure for Hydrazide Synthesis (H).** To a solution of hydrazine monohydrate (5.0 equiv) in dioxane (5 mL), substituted indole (1.0 equiv) was added. The resulting mixture was stirred at 110 °C for overnight. After complete reaction (TLC analysis), the residue was added brine solution and extracted with DCM (3 x 20 mL). Combined organic layer was dried over anhydrous magnesium sulfate and concentrated in vacuum. Product was purified using column chromatography.

**General Method for *N*-Propargylhydrazide Synthesis (I).** *N,N-*Diisopropylethylamine (DIPEA) (3.5 mmol) and 1-[Bis(dimethylamino)methylene]-1*H-*1,2,3-triazolo[4,5-b]pyridinium 3-oxid hexafluorophosphate (HATU) (1.0 mmol) were sequentially added at room temperature to a solution of the corresponding acid (1.0 mmol) in dry DMF (4 mL). The reaction was stirred for 15 min and then N-propargylhydrazine hydrochloride (1.0 mmol) was added. After completion (tic analysis), the crude product was purified using column chromatography.

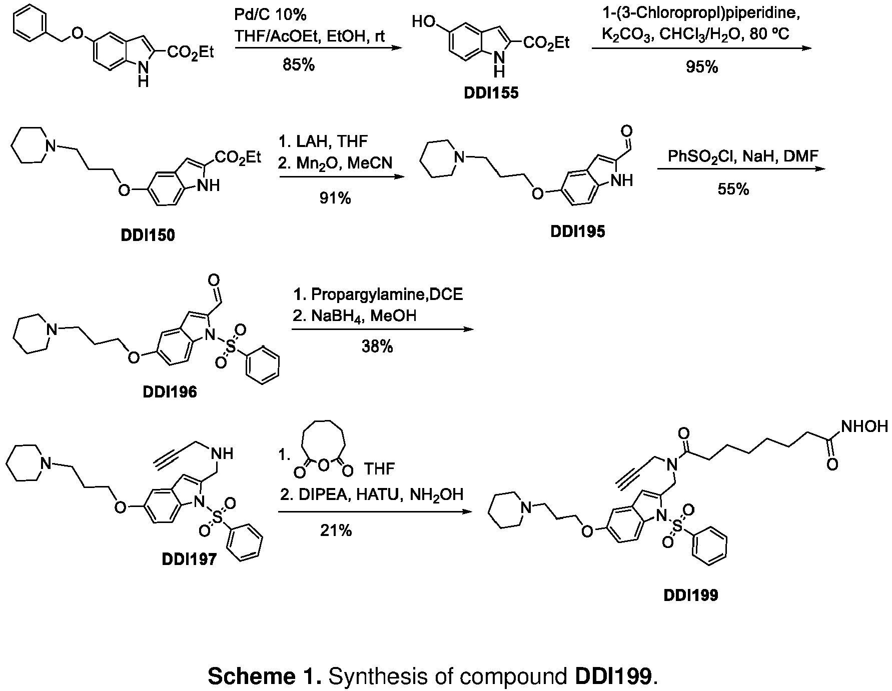

**Ethyl 5-(benzyloxy)-1*H*-indole-2-carboxylate (DDI55).** To a solution of commercial ethyl 5-(benzyloxy)-1H-indole-2-carboxylate (1 g, 3.4 mmol) in a mixture of THF, AcOEt and EtOH (8/8/6) (22 mL) Pd/C 10% (150 mg) was added and the mixture was hydrogenated at rt for 4 h at 45 psi. Then the crude was filtered over celite, and washed with MeOH. Then, the solvent was removed, and the crude was washed with DCM to give product **DDI155** (Buchi, G. et al. J. Am. Chem. Soc. 1985, 107, 5555) (593 mg, 85%): ¹H NMR (300 MHz, DMSO-d₆) δ 11.54 (s, 1H), 8.88 (s, 1H), 7.24 (d, *J* = 8.8 Hz, 1H), 6.91 (dd, J = 12.4, 1.8 Hz, 2H), 6.78 (dd, J = 8.8, 2.3 Hz, 1H), 4.29 (q, *J* = 7.1 Hz, 2H), 1.31 (t, *J* = 7.1 Hz, 3H); MS (ESI) *m*/*z:* 206 [M+1]⁺, 228 [M+Na]⁺.

**Ethyl 5-(3-(piperidin-1-yl)propoxy)-1*H*-indole-2-carboxylate (DDI150).** A mixture of Ethyl 5-(benzyloxy)-1*H*-indole-2-carboxylate (**DDI55**) (102.61 mg, 0.5 mmol), commercial 1-(3-chloropropyl)piperidine chlorhydrate (148,6 mg, 0.75 mmol) and potassium carbonate (414.6 mg, 3 mmol) were dissolved in CHCl₃ (2.5 mL) and water (0.5 ml). Then, the reaction mixture was stirred at 80 ºC for 3 d. After complete reaction, distilled water (20 mL) was added, and the mixture was extracted with chloroform (3 x 20 mL). The combined organic extracts were washed with brine (15 ml), dried over anhydrous magnesium sulfate and evaporated to get product **DDI150** (150,4 mg, 95%): ¹H NMR (300 MHz, CDCl₃) δ 8.89 (s, 1H), 7.30 (dt, *J* = 8.9, 0.8 Hz, 1H), 7.13 (dd, *J* = 2.1, 1.0 Hz, 1H), 7.08 (dt, *J* = 2.4, 0.7 Hz, 1H), 6.91 (dd, *J* = 8.9, 2.4 Hz, 1H), 4.33 (q, *J* = 7.1 Hz, 2H), 3.96 (t, *J* = 6.3 Hz, 2H), 2.55 (t, *J* = 7.8 Hz, 2H), 2.51-2.38 (m, 4H), 2.12 - 1.95 (m, 2H), 1.64 (p, *J*= 5.5 Hz, 4H), 1.51-1.44 (m, 2H), 1.34 (t, *J* = 7.1 Hz, 3H); ¹³C NMR (75 MHz, CDCl₃) δ 160.9, 153.0, 131.2, 126.8, 116.3, 111.6, 107.1, 102.6, 66.0, 59.9, 55.1, 53.6, 25.7, 24.8, 23.3, 13.4; MS (ESI) *m*/*z*: 331 [M+1]⁺, 353 [M+Na]⁺.

**5-(3-(Piperidin-1-yl)propoxy)-1*H*-indole-2-carbaldehyde (DDI195).** To a suspension of LAH (614.1 mg, 16.18 mmol) in dry THF (22 mL), under argon at 0 °C, ethyl 5-(3-(piperidin-1-yl)propoxy)-1*H*-indole-2-carboxylate (**DDI150**) (1.52 g, 4.6 mmol) was slowly added. Then, the mixture reacted at rt. The solid was removed, washing the cake with AcOEt, and extracted with AcOEt (3 x 20 mL); then, the solvent was evaporated, and the crude ((5-(3-(piperidin-1-yl)propoxy)-1*H*-indol-2-yl)methanol) was dissolved in MeCN (20 mL), MnO₂ (4.0 g, 46 mmol) was added, and the mixture was reacted at rt for 1 d. Then, the mixture was filtered and the solvent was removed giving product **DDI195** (1.2 g, 91%): ¹H NMR (300 MHz, CDCl₃) δ, 9.79 (s, 1H), 9.40 (s, 1H) 7.34 (d, *J* = 8.9 Hz, 1H), 7.16 (d, *J=* 1.2 Hz, 1H), 7.09 (d, *J* = 2.4 Hz, 1H), 7.05 (dd, *J* = 8.9, 2.4 Hz, 1H), 4.04 (t, *J* = 6.3 Hz, 2H), 2.54 (t, *J* = 8.2 Hz, 2H), 2.51-2.38 (m, 4H), 2.06-1.99 (m, 2H), 1.67 - 1.59 (m, 4H), 1.46 (d, *J* = 5.7 Hz, 2H).

**1-(Phenylsulfonyl)-5-(3-(piperidin-1-yl)propoxy)-1*H*-indole-2-carbaldehyde (DDI196).** 5-(3-(Piperidin-1-yl)propoxy)-1H-indole-2-carbaldehyde (**DDI195**) (1.2 g, 4.19 mmol) was dissolved in anhydrous DMF (22 mL) under an argon atmosphere, and the solution cooled at 0 ºC. The, sodium hydride (335.2 mg, 8.38 mmol, 2 equiv, 60% in oil) was added, and reaction mixture was stirred for 30 min. Then, benzenesulfonyl chloride (888.05 mg, 5.03 mmol, 1.1 equiv), dissolved in DMF (2 mL), was added and the reaction mixture was stirred at rt for 2 h. After complete reaction, the solvent was evaporated and the residue was dissolved in ethyl acetate, washed with water (30 mL) and extracted with ethyl acetate (3x 30 ml). The combined organic extracts were washed with brine (20 mL), dried over anhydrous magnesium sulfate and evaporated. Crude product was purified using column chromatography using DCM, methanol 5% to give the product **DI196** (989.5 mg, 55%): ¹H NMR (300 MHz, CDCl₃) δ 10.48 (s, 1H), 8.11 (d, *J* = 9.2 Hz, 1H), 7.99 - 7.81 (m, 1H), 7.72 (d, *J* = 7.8 Hz, 1H), 7.60 - 7.48 (m, 1H), 7.38 (m, 3H), 7.09 (dd, *J* = 9.2, 2.6 Hz, 1H), 7.02 - 6.78 (m, 1H), 4.02 (d, *J* = 5.9 Hz, 2H), 3.23-2.74 (m, 6H), 2.38 - 2.19 (m, 2H), 1.98-1.81 (m, 4H), 1.73-1.48 (m, 2H).

***N*-((1-(Phenylsulfonyl)-5-(3-(piperidin-1-yl)propoxy)-1*H*-indol-2-yl)methyl)prop-2-yn-1-amine (DDI197).** 1-(Phenylsulfonyl)-5-(3-(piperidin-1-yl)propoxy)-1H-indole-2-carbaldehyde (**DDI196**) (165.1 mg, 0.39 mmol) was dissolved in dichloroethane (2 mL) and propargylamine (0.029 mL, 0.46 mmol) was added. The mixture was stirred for 1 d at rt. The solvent was evaporated and then, was redissolved in MeOH (1.5 mL) and NaBH₄ (14.8 mg, 0.39 mmol) was added. The reaction was stirred for 1d, the solvent evaporated, and the residue dissolved in ethyl acetate, washed with brine (10 mL), dried over anhydrous magnesium sulfate and evaporated. Crude product was purified by column chromatography using DCM: methanol 6% to give the product **DDI197** (174 mg, 38%): ¹H NMR (300 MHz, CDCl₃) δ 8.02 (d, *J=* 9.6 Hz, 1H), 7.77 (d, *J* = 7.9 Hz, 2H), 7.58-7.46 (m, H), 7.46-7.29 (m, 2H), 6.95-6.82 (m, 2H), 6.53 (s, 1H), 4.18 (s, 2H), 4.00 (t, *J=* 6.4 Hz, 2H), 3.41 (d, *J=* 2.4 Hz, 2H), 2.64-2.38 (m, 6H), 2.23 (t, *J=* 2.4 Hz, 1H), 2.13-1.95 (m, 2H), 1.75-1.56 (m, 4H), 1.55-1.38 (m, 2H).

***N*1-Hydroxy-*N*8-((1-(phenylsulfonyl)-5-(3-(piperidin-1-yl)propoxy)-1*H*-indol-2-yl)methyl)-*N*8-(prop-2-yn-1-yl)octanediamide (DDI199).** Indole **DDI197** (174 mg, 0.37 mmol) was dissolved in anhydrous THF (5 mL) and oxonane-2,9-dione (99.22 mg, 0.64 mmol) was added. The reaction mixture was stirred at rt for 1 d. Then, the solvent was removed and the product was purified using column chromatography using as eluent DCM: methanol 10% to give 8-oxo-8-(((1-(phenylsulfonyl)-5-(3-(piperidin-1-yl)propoxy)-1*H*-indol-2-yl)methyl)(prop-2-yn-1-yl)amino)octanoic acid **(DDI198)** (117.8 mg, 0.19 mmol), that was dissolved in anhidrous DMF (3 mL), and treated with HATU (74.25 mg, 0.19 mmol) and DIPEA (0.083 mL, 0.46 mmol). The mixture was stirred for 15 min, and hydroxilamine hydrochloride (13.2 mg, 0.19 mmol) and DIPEA (24.5 mg 0.19 mmol, 1 equiv) were added. The mixture was stirred at rt for 1 d, and was poured into of a mixture of cooled brine (100 mL), keeping into the fridge for a 1 d. The mixture was filtered, and the crude product was purified by column chromatography using DCM: methanol 9% to give the product that was purified by HPLC to give pure **DDI199** (51 mg, 21%): mp 83-5 ºC; IR (KBr) v 285 (O-H), 3227 (N-H), 1651 (C=O), 1462 (O=S=O), 1161 (C-O-C) cm⁻¹; ¹H NMR (400 MHz, DMSO-d₆+D₂O, 100 ºC) δ (the CONHOH signals were eliminated by D₂O, and the NCH₂C≡C*H* signal is overlapped on the DMSO signal or on the signals in the range 2.39-2.30 ppm) 7.86 (d, *J=* 9.1 Hz, 1H, H7), 7.74 (dm, *J*= 7.3 Hz, 2H, H2', H6'), 7.65 (tm, *J*= 8.5 Hz, 1H, H4'), 7.54 (ddm, *J*= 8.5, 7.3 Hz, 1H, H3', H5'), 7.00 (d, *J*= 2.6 Hz, 1H, H4), 6.89 (dd, *J*= 9.1, 2,6 Hz, 1 H, H6), 6.45 (s, 1H, H3), 4.91 [d, *J*= 1.5 Hz, 2H, C*H₂*N(CH₂C≡CH)CO], 4.20 [d, *J=* 2.1 Hz, 2H, CH₂N(*CH₂*C≡CH)CO], 3.98 (t, *J*= 6.7 Hz, 2 Hz, OC*H₂*CH₂CH₂N), 2.37 (t, *J*= 6.7 Hz, 2H, OC*H₂*CH₂CH₂N), 2.32 {t, *J*= 5.4 Hz, 4H, [CH₂(CH₂)₂(C*H*₂)₂]N]}, 2.02-1.94 (m, 2H, NCOC*H₂*CH₂CH₂CH₂CH₂CH₂CONHOH), 1.82 (p, *J*= 6.7 Hz, 2H, OCH₂C*H*₂CH₂N), 1.56-1.43 {m, 10 H: 4H for [CH₂(C*H*₂)₂(CH₂)₂]N], 4 H for NCOCH₂CH₂CH₂CH₂CH₂CH₂CONHOH, 2 H for NCOCH₂CH₂CH₂CH₂CH₂C*H*₂CONHOH}, 1.36 {pm, *J=* 5.7 Hz, 2H, [C*H₂*(CH₂)₂(CH₂)₂]N}, 1.30-1.17 (m, 4 H, NCOCH₂CH₂CH₂CH₂CH₂CH₂CONHOH, 4H); ¹³C NMR (101 MHz, DMSO-d₆+D₂O, 100 ºC) [the signals for the NCH₂*C*≡*C*H group do not show at the NMR spectrum, but have been located at 75.0 (C), 78.2 (CH), thanks to the HBMC spectrum, due to the cross peak with the signal at 4.19 ppm assigned to the NCH₂; the signals for *C*H₂N(CH₂C≡CH)CO, and CH₂N(*C*H₂C≡CH)CO do not show and have not been located in the NMR spectrum] δ 172.6 (2 C, NCOCH₂, CH₂*C*ONHOH), 155.8 (C5), 137.6 (2 C, C2, C1"), 134.3 (C4"), 131.3 (C7a), 130.4 (C3a), 129.6 (2C, C3', C5'), 125.8 (2C, C2', C6'), 114.8 (C7), 113.7 (C6), 109.8 (C3), 104.9 (C4), 66.6 (O*C*H₂CH₂CH₂N), 54.9 [CH₂(CH₂)₂(CH₂)₂]N*C*H₂], 53.9 [2 C, CH₂(CH₂)₂(*C*H₂)₂]NCH₂], 31.9 [NCO*C*H₂ CH₂CH₂CH₂CH₂CH₂CONHOH], 28.1 [2 C: NCOCH₂CH₂*C*H₂*C*H₂CH₂CH₂CONHOH], 26.3 (OCH₂*C*H₂CH₂N), 25.3 [3C: 2 C, CH₂(*C*H₂)₂(CH₂)₂]NCH₂, 1C: NCOCH₂*C*H₂CH₂CH₂CH₂CH₂CONHOH], 24.5 NCOCH₂CH₂CH₂CH₂CH₂CH₂CONHOH, 24.2 [*C*H₂(CH₂)₂(CH₂)₂]NCH₂], 23.9 [NCOCH₂CH₂CH₂CH₂CH₂CH₂CONHOH]. HRMS. Calcd for ([C₃₄H₄₄N₄O₆S] + H)⁺: 637.3054. Found: 637.3045.

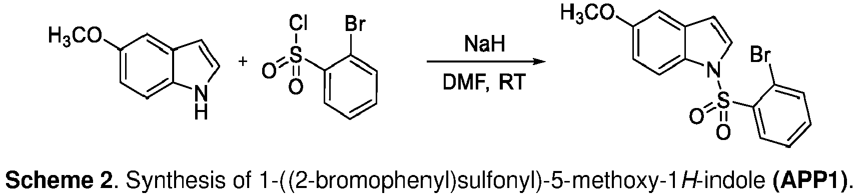

**1-((2-Bromophenyl)sulfonyl)-5-methoxy-1*H*-indole (APP1).** Following the **General Method A**, starting from commercial 5-methoxy-1H indole (300 mg, 2.04 mmol), after flash chromatography of the residue using hexane/AcOEt (10:1) as eluent gave compound **APP1** (538 mg, 72%) was obtained as a white solid: mp 75-7 °C; IR (cm⁻¹): v 1377 (O=S=O), 1145 (C-O-C), 1030 (C-Br); ¹H NMR (400 MHz, CDCl₃) *δ* 8.02 (dd, *J* = 7.8, 1.8 Hz, 1H), 7.74 (d, *J* = 3.7 Hz, 1H, H2), 7.68 (dd, *J* = 7.8, 1.4 Hz, 1H), 7.58 (dd, *J* = 9.0, 0.7 Hz, 1H, H7), 7.46 (td, *J* = 7.8, 1.4 Hz, 1H), 7.39 (td, *J* = 7.8, 1.7 Hz, 1H), 7.04 (d, *J* = 2.5 Hz, 1H, H4), 6.86 (dd, *J* = 9.0, 2.5 Hz, 1H, H6), 6.61 (dd, *J* = 3.7, 0.7 Hz, 1H, H3), 3.82 (s, 3H); ¹³C NMR (101 MHz, CDCl₃) *δ* 156.4, 138.2, 136.0 (CH_{Ar}), 134.6 (CH_{Ar}), 131.5, 131.2 (CH_{Ar}), 129.2, 128.8 (C2), 127.8 (CH_{Ar}), 120.6, 113.8 (C7), 113.5 (C6), 107.5 (C3), 103.8 (C4), 55.6 (OCH₃). HRMS (ESI): Calcd for C₁₅H₁₃BrNO₃S⁺ [*M* + H]⁺: 365.9794. Found: 365.9804.

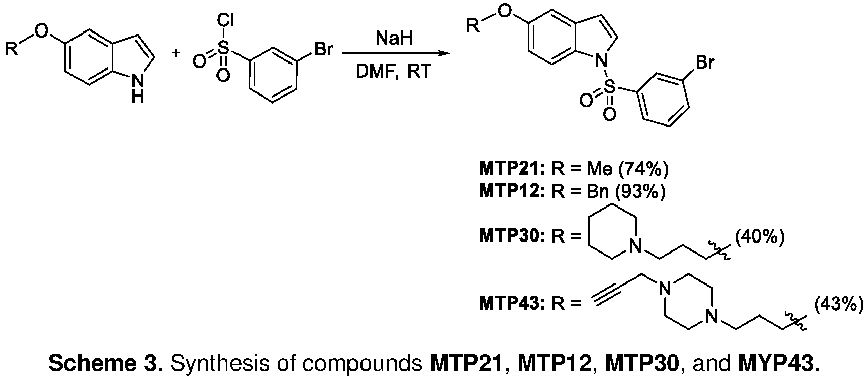

**1-((3-Bromophenyl)sulfonyl)-5-methoxy-1*H*-indole (MTP21).** Following the **General Method A,** starting from commercial 5-methoxy-1H indole (500 mg, 3.40 mmol), after flash chromatography of the residue using hexane/AcOEt (10%) as eluent gave compound **MTP21** (1.1 g, 74%) was obtained as a white solid: mp 75-7 °C; IR (cm⁻¹) v 1373 (O=S=O), 1226 (C-O-C), 1143 (C-Br); ¹H NMR (400 MHz, CDCl₃) δ 7.99 (t, *J* = 1.9 Hz, 1H), 7.87 (dt, *J* = 9.0, 0.7 Hz, 1H, H7), 7.77 (ddd, *J* = 7.9, 1.8, 1.0 Hz, 1H), 7.65 (ddd, *J* = 8.0, 1.9, 1.0 Hz, 1H), 7.50 (d, *J* = 3.6 Hz, 1H, H2), 7.30 (t, *J* = 8.0 Hz, 1H), 6.99 (d, *J* = 2.5 Hz, 1H, H4), 6.96 (dd, *J* = 8.9, 2.5 Hz, 1H, H6), 6.63 (dd, *J* = 3.6, 0.8 Hz, 1H, H3), 3.83 (s, 3H); ¹³C NMR (101 MHz, CDCl₃) δ 156.7, 139.8, 136.8 (CH_{Ar}), 131.8, 130.7 (CH_{Ar}), 129.5 (CH_{Ar}), 129.4, 126.9 (C2), 125.2 (CH_{Ar}), 123.1, 114.3 (C7), 114.0 (C6), 110.0 (C3), 103.8 (C4), 55.6 (CH₂). HRMS (ESI): Calcd for C₁₅H₁₃BrNO₃S⁺ [*M* + H]⁺: 365.9794. Found: 365.9798.

**5-(Benzyloxy)-1-((3-bromophenyl)sulfonyl)-1*H*-indole (MTP12).** Following the **General Method A,** starting from commercial 5-(benzyloxy)-1*H*-indole (200 mg, 0.90 mmol), after flash chromatography of the residue using hexane/AcOEt (10%) as eluent compound **MTP12** (360 mg, 93%) was obtained as a white solid: mp 105-7 °C; IR (cm⁻¹) v 1354 (O=S=O), 1200 (C-O-C), 1180 (C-Br); ¹H NMR (400 MHz, CDCl₃) δ 7.91 (t, *J* = 1.9 Hz, 1H), 7.80 (dd, *J* = 8.8, 0.8 Hz, 1H, H7), 7.68 (ddd, *J* = 7.9, 1.8, 1.0 Hz, 1H), 7.60 - 7.53 (m, 1H), 7.41 (d, *J* = 3.7 Hz, 1H, H2), 7.38 - 7.17 (m, 6H), 7.01 - 6.92 (m, 2H, H4 and H6), 6.53 (dd, *J* = 3.7, 0.8 Hz, 1H, H3), 4.99 (s, 2H); ¹³C NMR (101 MHz, CDCl₃) δ 155.8, 139.8, 136.9, 136.8 (CH_{Ar}), 131.8, 130.7 (CH_{Ar}), 129.6, 129.5 (CH_{Ar}), 128.6 (2CH_{Ar}), 128.0 (CH_{Ar}), 127.5 (2CH_{Ar}), 126.9 (C2), 125.2 (CH_{Ar}), 123.1, 114.7 (C6), 114.4 (C7), 110.0 (C3), 105.2 (C4), 70.5 (CH₂). HRMS (ESI): Calcd for C₂₁H₁₆BrNNaO₃S⁺ [*M* + Na]⁺: 463.9926. Found: 463.9923.

**1-((3-Bromophenyl)sulfonyl)-5-(3-(piperidin-1-yl)propoxy)-1H-indole (MTP30).** Following the **General Method A,** starting from 5-(3-(piperidin-1-yl)propoxy)-1*H-*indole (Nyantakyi, S. A. et al. *J*. *Med. Chem.* **2018,** 61, 5733) (400 mg, 1.55 mmol), after flash chromatography of the residue using DCM/ Methanol (5%) as eluent, compound **MTP30** (300 mg, 40%) was obtained as a brown oil: IR (cm⁻¹) v 1376 (O=S=O), 1265 (C-O-C), 1181 (C-Br); ¹H NMR (400 MHz, CDCl₃) δ 7.89 (t, *J* = 1.9 Hz, 1H), 7.76 (d, *J* = 9.0 Hz, 1H), 7.66 (dt, *J* = 8.1, 1.3 Hz, 1H), 7.54 (dt, *J* = 7.9, 1.3 Hz, 1H), 7.39 (d, *J=* 3.7 Hz, 1H), 7.19 (t, *J=* 8.0 Hz, 1H), 6.96 - 6.80 (m, 2H), 6.52 (d, *J=* 3.7 Hz, 1H), 3.93 (t, *J=* 6.2 Hz, 2H), 2.59 - 2.38 (m, 6H), 2.06 - 1.89 (m, 2H), 1.57 (q, *J* = 5.7 Hz, 4H), 1.39 (d, *J=* 6.1 Hz, 2H); ¹³C NMR (101 MHz, CDCl₃) δ 155.9, 139.7, 136.7 (CH_{Ar}), 131.8, 130.6 (CH_{Ar}), 129.4 (CH_{Ar}), 129.3, 126.8 (C2), 125.1 (CH_{Ar}), 123.0, 114.4 (C7), 114.2 (C6), 110.0 (C3), 104.7 (C4), 66.6 (CH₂), 55.8 (CH₂), 54.4 (2CH₂), 26.3 (CH₂), 25.3 (2CH₂), 23.9 (CH₂). HRMS (ESI): Calcd for C₂₂H₂₆BrN₂O₃S⁺ [*M* + H]⁺: 477.0842. Found: 477.0835.

**1-((3-Bromophenyl)sulfonyl)-5-(3-(4-(prop-2-yn-1-yl)piperazin-1-yl)propoxy)-1*H-*indole (MTP43).** Following the **General Method A,** starting from 5-(3-(4-(prop-2-yn-1-yl)piperazin-1-yl)propoxy)-1*H* indole **(MTP39)** (400 mg, 1.34 mmol), after flash chromatography of the residue using DCM/Methanol (5%) as eluent, gave compound **MTP43** (270 mg, 43%) as a white oil: IR (cm⁻¹) v 1372 (O=S=O), 1224 (C-O-C), 1180 (C-Br); ¹H NMR (400 MHz, CDCl₃) δ 7.98 (t, *J=* 1.9 Hz, 1H), 7.85 (d, *J=* 9.0 Hz, 1H, H7), 7.75 (ddd, *J* = 8.0, 1.9, 1.0 Hz, 1H), 7.64 (ddd, *J* = 8.0, 1.9, 1.0 Hz, 1H), 7.49 (d, *J* = 3.6 Hz, 1H, H2), 7.29 (t, *J* = 8.0 Hz, 1H), 6.99 (d, *J* = 2.5 Hz, 1H, H4), 6.94 (dd, *J* = 9.0, 2.5 Hz, 1H, H6), 6.61 (dd, *J* = 3.6, 0.8 Hz, 1H, H3), 4.03 (t, *J=* 6.2 Hz, 2H), 3.33 (d, *J=* 2.5 Hz, 2H), 2.64 - 2.57 (m, 10H), 2.27 (t, *J* = 2.5 Hz, 1H), 2.02 - 1.99 (m, 2H); ¹³C NMR (101 MHz, CDCl₃) δ 155.9, 139.8, 136.8 (CH_{Ar}), 131.8, 130.7 (CH_{Ar}), 129.5 (CH_{Ar}), 129.4, 126.9 (C2), 125.2 (CH_{Ar}), 123.1, 114.4 (C7), 114.3 (C6), 110.0 (C3), 104.7 (C4), 78.6, 73.4 (CH), 66.5 (CH₂), 55.1 (2CH₂), 52.9 (2CH₂), 51.4 (CH₂), 46.7 (CH₂), 26.4 (CH₂); HRMS (ESI): Calcd for C₂₄H₂₇BrN₃O₃S⁺ [*M*+ H]⁺: 516.0951. Found: 516.0954. Anal. Calcd for C₂₄H₂₆BrN₃O₃S·H₂O: C, 53.93; H, 5.28; N, 7.86; S, 6.00. Found: C, 53.83; H, 5.13; N, 7.82; S, 5.96.

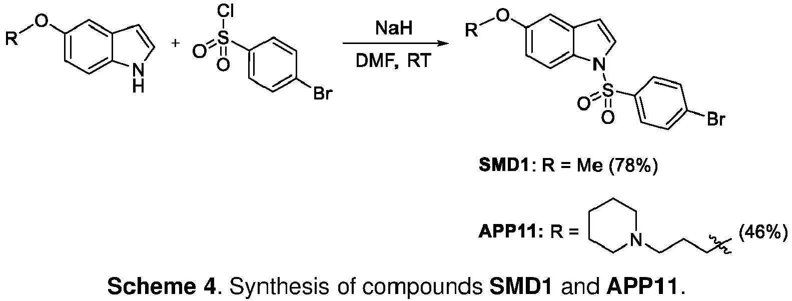

**1-((4-Bromophenyl)sulfonyl)-5-methoxy-1*H*-indole (SMD1).** Following the **General Method A,** starting from commercial 5-methoxy-1*H*-indole (300 mg, 2.04 mmol), after flash chromatography of the residue using hexane/AcOEt (10:1) as eluent gave compound **SMD1** (585 mg, 78%) was obtained as a white solid: mp 135-7 °C; IR (cm⁻¹): v 1371 (O=S=O), 1150 (C-O-C), 1090 (C-Br); ¹H NMR (400 MHz, CDCl₃) *δ* 7.85 (dt, *J* = 8.8, 0.7 Hz, 1H, H7), 7.75 - 7.67 (m, 2H), 7.62 - 7.52 (m, 2H), 7.49 (d, *J* = 3.7 Hz, 1H, H2), 6.98 (d, *J* = 2.5 Hz, 1H, H4), 6.94 (dd, *J* = 8.8, 2.5 Hz, 1H, H6), 6.62 (dd, *J* = 3.7, 0.8 Hz, 1H, H3), 3.82 (s, 3H); ¹³C NMR (101 MHz, CDCl₃) *δ* 156.6, 137.0, 132.5 (2CH_{Ar}), 131.9, 129.4, 129.0, 128.1 (2CH_{Ar}), 126.9 (C2), 114.3 (C7), 113.9 (C6), 110.0 (C3), 103.8 (C4), 55.6 (OMe). HRMS (ESI): Calcd for C₁₅H₁₂BrNNaO₃S⁺ [*M* + Na]⁺: 387.9613. Found: 387.9619.

**1-((4-Bromophenyl)sulfonyl)-5-(3-(piperidin-1-yl)propoxy)-1*H*-indole (APP11).** Following the **General Method A,** starting from 5-(3-(piperidin-1-yl)propoxy)-1H-indole (Nyantakyi, S. A. et al. J. Med. Chem. 2018, 61, 5733) (830 mg, 3.22 mmol), after flash chromatography of the residue using DCM/ Methanol (2%) as eluent, compound **APP11** (706 mg, 46%) was obtained as a white solid: mp 194-6 °C: IR (cm-1): v 1396 (O=S=O), 1185 (C-O-C), 1133 (C-Br); ¹H NMR (300 MHz, CDCl₃) δ 7.85 (dd, *J* = 8.9, 0.7 Hz, 1H, H7), 7.69 (d, *J* = 8.9 Hz, 2H), 7.55 (d, *J* = 8.9 Hz, 2H), 7.47 (d, *J=* 3.7 Hz, 1H, H2), 6.98 (d, *J=* 2.5 Hz, 1H, H4), 6.93 (dd, *J* = 8.9, 2.5 Hz, 1H, H6), 6.60 (dt, *J* = 3.7, 0.7 Hz, 1H, H3), 4.01 (t, *J* = 6.4 Hz, 2H), 2.51 - 2.35 (m, 6H), 2.12 - 1.79 (m, 2H), 1.63 - 1.55 (m, 4H), 1.47 - 1.44 (m, 2H); ¹³C NMR (101 MHz, CDCl₃) δ 156.1, 137.0, 132.5 (2CH_{Ar}), 131.9, 129.4, 129.0, 128.1 (2CH_{Ar}), 126.9 (C2), 114.4 (C6), 114.3 (C7), 110.0 (C3), 104.7 (C4), 67.0 (CH₂), 56.0 (CH₂), 54.7 (2CH₂), 26.9 (CH₂), 26.0 (2CH₂), 24.4 (CH₂). HRMS (ESI): Calcd for C₂₂H₂₆BrN₂O₃S+ [*M* + H]+: 477.0842. Found: 477.0847.

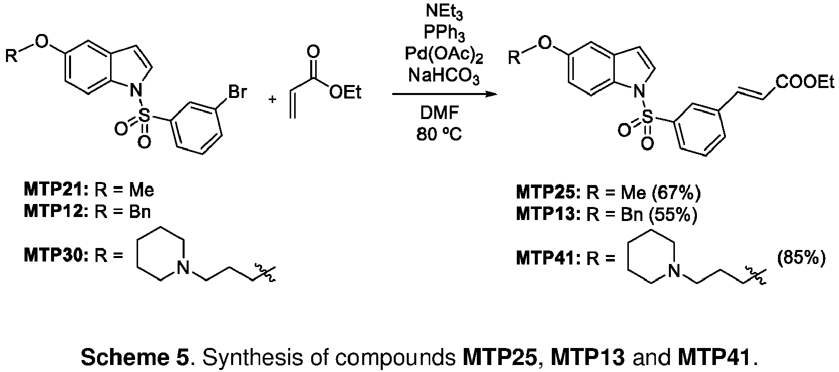

**Ethyl (*E*)-3-(3-((5-methoxy-1*H*-indol-1-yl)sulfonyl)phenyl)acrylate (MTP25).** Following the **General Method B,** starting from **MTP21** (550 mg, 1.50 mmol) after flash chromatography of the residue using hexane/AcOEt (10%) as eluent, compound **MTP25** (578 mg, 67%) was obtained as a white solid: mp 95-7 °C; IR (cm⁻¹) v 1712 (C=O), 1371 (O=S=O), 1169 (C-O-C); ¹H NMR (400 MHz, CDCl₃) δ 7.96 (t, *J* = 1.9 Hz, 1H), 7.89 (d, *J* = 8.9 Hz, 1H, H7), 7.82 (ddd, *J* =7.9, 1.9, 1.1 Hz, 1H), 7.67 - 7.63 (m, 1H), 7.60 (d, *J* = 16.0 Hz, 1H, C*H*=CH), 7.52 (d, *J* = 3.6 Hz, 1H, H2), 7.45 (t, *J =* 7.9 Hz, 1H), 7.01 - 6.93 (m, 2H, H4 and H6), 6.62 (dd, *J* = 3.6, 0.8 Hz, 1H, H3), 6.44 (d, *J* = 16.0 Hz, 1H, CH=C*H*), 4.28 (q, *J* = 7.1 Hz, 2H), 3.82 (s, 3H), 1.35 (t, *J* = 7.1 Hz, 3H); ¹³C NMR (101 MHz, CDCl₃) δ 166.1, 156.6, 141.9 (CH=CH), 139.1, 135.8, 132.6 (CH_{Ar}), 131.8, 129.8 (CH_{Ar}), 129.5, 127.7 (CH_{Ar}), 127.0 (C2), 125.8 (CH_{Ar}), 121.1 (CH=CH), 114.3 (C7), 113.9 (C6), 109.8 (C3), 103.8 (C4), 60.9 (CH₂), 55.6 (OCH₃), 14.2 (CH₃). HRMS (ESI): Calcd for C₂₀H₁₉NNaOₛS⁺ [*M* + Na]⁺: 408.0876. Found: 408.0851.

**Ethyl (*E*)-3-(3-((5-(benzyloxy)-1*H*-indol-1-yl)sulfonyl)phenyl)acrylate (MTP13).** Following the **General Method B,** starting from **MTP12** (180 mg, 0.41 mmol), after flash chromatography of the residue using hexane/AcOEt (3%) as eluent, compound **MTP13** (103 mg, 55%) was obtained as a white solid: mp 102-4 °C; IR (cm⁻¹) v 1643 (C=O), 1451 (O=S=O), 1114 (C-O-C); ¹H NMR (500 MHz, CDCl₃) δ 7.97 (t, *J=* 1.7 Hz, 1H), 7.90 (d, *J* = 8.9 Hz, 1H, H7), 7.83 (ddd, *J* =7.9, 1.7, 1.1 Hz, 1H), 7.65 (d, *J =* 7.8 Hz, 1H), 7.61 (d, *J* = 16.0 Hz, 1H, C*H*=CH), 7.52 (d, *J=* 3.7 Hz, 1H, H2), 7.48 - 7.30 (m, 6H), 7.80 - 7.00 (m, 2H, H4 and H6), 6.61 (dd, *J* = 3.7, 0.6 Hz, 1H), 6.44 (d, *J* = 16.0 Hz, 1H, CH=C*H*), 5.07 (s, 2H), 4.28 (q, *J* = 7.1 Hz, 2H), 1.35 (t, *J* = 7.1 Hz, 3H); ¹³C NMR (126 MHz, CDCl₃) δ 166.1, 155.8, 141.9 (CH=CH), 139.1, 136.9, 135.8, 132.6 (CH_{Ar}), 131.8, 129.9 (CH_{Ar}), 129.6, 128.6 (2CH_{Ar}), 128.0 (CH_{Ar}), 127.7 (CH_{Ar}), 127.4 (2CH_{Ar}), 127.0 (C2), 125.8 (CH_{Ar}), 121.1 (CH=CH), 114.6 (C7), 114.3 (C6), 109.8 (C3), 105.1 (C4), 70.5 (CH₂), 60.9 (CH₂), 14.3 (CH₃). HRMS (ESI): Calcd for C₂₆H₂₃NNaO₅S⁺ [*M* + Na]⁺: 484.1189. Found: 484.1188.

**Ethyl (*E*)-3-(3-((5-(3-(Piperidin-1-yl)propoxy)-1*H*-indol-1-yl)sulfonyl)phenyl)acrylate (MTP41).** Following the **General Method B,** starting from 1-((3-bromophenyl)sulfonyl)-5-(3-(piperidin-1-yl)propoxy)-1H-indole (**MTP30**) (667 mg, 1.40 mmol), after flash chromatography of the residue using DCM/Methanol (2%) as eluent, gave compound **MTP41** (694 mg, 85%) as a brown solid: mp 95-7 °C; IR (cm⁻¹) v 1714 (C=O), 1372 (O=S=O), 1155 (C-O-C); ¹H NMR (300 MHz, CDCl₃) δ 7.95 (t, *J* = 1.8 Hz, 1H), 7.88 (dt, *J* = 9.0, 0.9 Hz, 1H, H7), 7.81 (ddd, *J* = 7.9, 1.9, 1.1 Hz, 1H), 7.65 (ddt, *J* = 7.4, 1.5, 0.9 Hz, 1H), 7.59 (d, *J* = 16.0 Hz, 1H, C*H*=CH), 7.52 (d, *J=* 3.7 Hz, 1H, H2), 7.45 (t, *J=* 7.9 Hz, 1H), 6.96 (d, *J=* 2.1 Hz, 1H, H4), 6.90 (dd, *J* = 9.0, 2.1 Hz, 1H, H6), 6.61 (dd, *J* = 3.7, 0.9 Hz, 1H, H3), 6.43 (d, *J* = 16.0 Hz, 1H, CH=C*H*), 4.28 (q, *J=* 7.1 Hz, 2H), 4.06 (t, *J* = 5.7 Hz, 2H), 3.09 - 3.00 (m, 6H), 2.44 - 2.35 (m, 2H), 2.10 - 1.95 (m, 4H), 1.78 - 1.60 (m, 2H), 1.34 (t, *J* = 7.1 Hz, 3H); ¹³C NMR (101 MHz, CDCl₃) δ 166.1, 155.6, 141.8 (CH=CH), 139.0, 135.8, 132.6 (CH_{Ar}), 131.8, 129.9 (CH_{Ar}), 129.6, 127.6 (CH_{Ar}), 127.0 (C2), 125.8 (CH_{Ar}), 121.1 (CH=CH), 114.3 (C7), 114.3 (C6), 109.8 (C3), 104.6 (C4), 66.2 (CH₂), 60.9 (CH₂), 55.7 (2CH₂), 54.1 (2CH₂), 25.5 (CH₂), 24.4 (CH₂), 23.3 (CH₂), 14.2 (CH₃); HRMS (ESI): Calcd for C₂₇H₃₃N₂O₅S⁺ [*M* + H]⁺: 497.2105. Found: 497.2087. Anal. Calcd for C₂₇H₃₂N₂O₅S·3H₂O: C, 58.89; H, 6.96; N, 5.09; S, 5.82. Found: C, 58.98; H, 7.17; N, 5.13; S, 5.87.

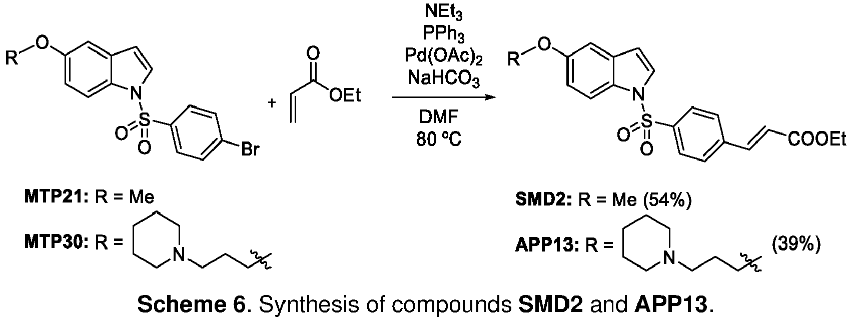

**Ethyl (*E*)-3-(4-((5-Methoxy-1*H*-indol-1-yl)sulfonyl)phenyl)acrylate (SMD2).** Following the **General Method B,** starting from **MTP21** (580 mg, 1.58 mmol) after flash chromatography of the residue using hexane/AcOEt (10:1) as eluent, compound **SMD2** (330 mg, 54%) was obtained as a yellow solid: mp 115-7 °C; IR (cm⁻¹): v 1717 (C=O), 1312 (O=S=O), 1107 (C-O-C); ¹H NMR (300 MHz, CDCl₃) *δ* 7.89 (dt, *J* = 8.9, 0.8 Hz, 1H, H7), 7.89 - 7.80 (m, 2H), 7.64 - 7.40 (m, 4H), 6.99 - 6.98 (m, 1H, H4), 6.94 (dd, *J* = 8.9, 2.5 Hz, 1H, H6), 6.61 (dd, *J* = 3.6, 0.8 Hz, 1H, H3), 6.44 (d, *J=* 16.0 Hz, 1H, CH=C*H*), 4.26 (q, *J=* 7.1 Hz, 2H), 3.82 (s, 3H), 1.33 (t, *J=* 7.1 Hz, 3H); ¹³C NMR (101 MHz, CDCl₃) *δ* 166.0, 156.6, 141.8 (*C*H=CH), 139.6, 138.8, 131.8, 129.5, 128.5 (2CH_{Ar}), 127.2 (2CH_{Ar}), 127.0 (C2), 122.1 (CH=CH), 114.4 (C6), 113.9 (C4), 109.8 (C3), 103.7 (C7), 60.9 (CH₂), 55.6 (OCH₃), 14.2 (CH₃). HRMS (ESI): Calcd for C₂₀H₁₉NNaO₅S⁺ [*M* + Na]⁺: 408.0876. Found: 408.0884.

**Ethyl (*E*-3-(4-((5-(3-(Piperidin-1-yl)propoxy)-1*H*-indol-1-yl)sulfonyl)phenyl)acrylate (APP13).** Following the **General Method B,** starting from **MTP21** (1 g, 2.25 mmol) after flash chromatography of the residue using DCM/MeOH (2%) as eluent, compound **APP13** (439 mg, 39%) was obtained as a brown oil: IR (cm⁻¹): v 1713 (C=O), 1371 (O=S=O), 1154 (C-O-C); ¹H NMR (400 MHz, CDCl₃) *δ* 7.87 (d, *J* = 9.0 Hz, 1H, H7), 7.84 (d, *J* = 8.6 Hz, 2H), 7.58 (d, *J* = 16.0 Hz, 1H, C*H*=CH), 7.53 (d, *J=* 8.6 Hz, 2H), 7.50 (d, *J=* 3.7 Hz, 1H, H2), 6.97 (d, *J=* 2.5 Hz, 1H, H4), 6.92 (dd, *J* = 9.0, 2.5 Hz, 1H, H6), 6.60 (dd, *J* = 3.7, 0.8 Hz, 1H, H3), 6.44 (d, *J* = 16.0 Hz, 1H, CH=C*H*), 4.26 (q, *J* = 7.1 Hz, 2H), 4.01 (t, *J* = 6.2 Hz, 2H), 2.67 - 2.46 (m, 6H), 2.12 - 2.00 (m, 2H), 1.69 (q, *J* = 5.8 Hz, 4H), 1.54 - 1.45 (m, 2H), 1.32 (t, *J=* 7.1 Hz, 3H); ¹³C NMR (101 MHz, CDCl₃) *δ* 166.0, 155.8, 141.8 (CH=CH), 139.6, 138.8, 131.8, 129.5, 128.4 (2CH_{Ar}), 127.2 (2CH_{Ar}), 127.0 (C2), 122.1 (CH=CH), 114.3 (C6), 114.3 (C4), 109.8 (C3), 104.6 (C7), 66.6 (CH₂), 60.9 (CH₂), 55.9 (CH₂), 54.3 (2CH₂), 26.2 (CH₂), 25.5 (2CH₂), 23.9 (CH₂), 14.2 (CH₃). HRMS (ESI): Calcd for C₂₇H₃₃N₂O₅S⁺ [*M* + H]⁺: 497.2105. Found: 497.2111.

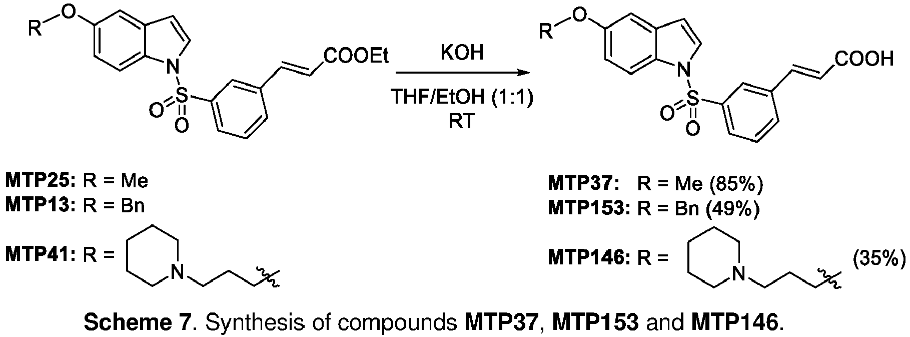

**(*E*)-3-(3-((5-Methoxy-1*H*-indol-1-yl)sulfonyl)phenyl)acrylic acid (MTP37).** Following the **General Method C,** starting from **MTP25** (100 mg, 0.26 mmol) after flash chromatography of the residue using DCM/Methanol (5%) as eluent, compound **MTP37** (75 mg, 85%) was obtained as a brown solid: mp 167-9 °C; IR (cm⁻¹) v 3054 (COO-H), 1692 (C=O), 1422 (O=S=O), 1265 (C-O-C); ¹H NMR (400 MHz, DMSO-d₆) δ 8.25 (s, 1H), 8.00 (d, *J* = 7.8 Hz, 1H), 7.92 (d, *J* = 8.0 Hz, 1H), 7.87 (d, *J* = 9.0 Hz, 1H, H7), 7.81 (d, *J* = 3.7 Hz, 1H, H2), 7.64 - 7.57 (m, 2H), 7.10 (d, *J* = 2.5 Hz, 1H), 6.94 (dd, *J* = 9.0, 2.5 Hz, 1H, H6), 6.77 (d, *J* = 3.7 Hz, 1H, H3), 6.66 (d, *J* = 16.1 Hz, 1H, CH=C*H*), 3.74 (s, 3H) (the COOH signal could not be assigned); ¹³C NMR (101 MHz, DMSO-d₆) δ 167.2, 156.2, 141.5 (CH=CH), 137.8, 136.0, 133.5 (CH_{Ar}), 131.7, 130.4 (CH_{Ar}), 128.7, 127.9 (C2), 127.7 (CH_{Ar}), 126.2 (CH_{Ar}), 122.3 (CH=CH), 114.1 (C7), 113.7 (C6), 110.1 (C3), 104.1 (C4), 55.4 (OCH₃). HRMS (ESI): Calcd for C₁₈H₁₅NNaO₅S⁺ [*M* + Na]⁺: 380.0563. Found: 380.0561.

**(*E*)-3-(3-((5-(Benzyloxy)-1*H*-indol-1-yl)sulfonyl)phenyl)acrylic acid (MTP153).** Following the **General Method C,** starting from **MTP13** (1.1 g, 2.38 mmol) after flash chromatography of the residue using DCM/Methanol (5%) as eluent, compound **MTP153** (503 mg, 49%) was obtained as a brown solid: mp 166-8 °C; IR (cm⁻¹) v 2976 (COO-H), 1694 (C=O), 1369 (O=S=O), 1155 (C-O-C); ¹H NMR (400 MHz, CDCl₃) δ 7.99 (t, *J* = 1.8 Hz, 1H), 7.91 (dt, *J* = 8.8, 1.0 Hz, 1H, H7), 7.87 (ddd, *J* = 8.0, 1.8, 1.0 Hz, 1H), 7.73 - 7.68 (m, 2H), 7.53 (d, *J* = 3.7 Hz, 1H), 7.48 (t, *J* = 8.0 Hz, 1H), 7.46 - 7.30 (m, 5H), 7.07 - 7.02 (m, 2H, H4 and H6), 6.62 (dd, *J* = 3.7, 1.0 Hz, 1H), 6.46 (d, *J=* 16.0 Hz, 1H, CH=C*H*), 5.07 (s, 2H) (the COOH signal could not be assigned); ¹³C NMR (101 MHz, CDCl₃) δ 170.6, 155.8, 144.3 (CH=CH), 139.2, 136.9, 135.4 (CH_{Ar}), 132.8 (CH_{Ar}), 131.8 (CH_{Ar}), 130.0 (CH_{Ar}), 129.6, 128.6 (2CH_{Ar}), 128.2, 128.0 (CH_{Ar}), 127.5 (2CH_{Ar}), 127.0, 126.1 (C2), 120.0 (CH=CH), 114.7 (C6), 114.4 (C7), 109.9 (C3), 105.2 (C4), 70.5 (CH₂). HRMS (ESI): Calcd for C₂₄H₁₉NNaO₅S⁺ [*M* + Na]⁺: 456.0876. Found: 456.0881.

**(*E*)-3-(3-((5-(3-(Piperidin-1-yl)propoxy)-1*H*-indol-1-yl)sulfonyl)phenyl)acrylic acid (MTP146).** Following the **General Method C,** starting from **MTP41** (450 mg, 0.91 mmol) after flash chromatography of the residue using DCM/Methanol/NH₄OH (80:18:2) as eluent, compound **MTP146** (150 mg, 35%) was obtained as a white solid: mp 168-70 °C; IR (cm⁻¹) v 2929 (COO-H), 1641 (C=O), 1366 (O=S=O), 1141 (C-O-C); ¹H NMR (400 MHz, CDCl₃) δ 8.24 (s, 1H), 7.99 (d, *J* = 7.6 Hz, 1H), 7.91 (d, *J* = 7.6 Hz, 1H), 7.87 (d, *J* = 9.0 Hz, 1H, H7), 7.80 (d, *J* = 3.7 Hz, 1H, H2), 7.65 - 7.50 (m, 2H), 7.10 (t, *J=* 2.1 Hz, 1H, H4), 6.93 (dd, *J* = 9.0, 2.1 Hz, 1H, H6), 6.76 (dd, *J* = 3.7, 1.5 Hz, 1H, H3), 6.65 (d, *J=* 16.1 Hz, 1H, CH=C*H*), 3.98 (td, *J* = 6.2, 1.7 Hz, 2H), 2.62 - 2.57 (m, 6H), 1.97 - 1.87 (m, 2H), 1.62 - 1.45 (m, 4H), 1.48 - 1.32 (m, 2H) (the COOH signal could not be assigned); ¹³C NMR (101 MHz, CDCl₃) δ 171.7, 155.3, 138.6, 137.8 (*C*H=CH), 137.3, 132.3 (CH_{Ar}), 131.8, 129.8, 129.6 (CH_{Ar}), 127.2 (C2), 126.5 (CH=CH), 125.3 (CH_{Ar}), 114.4 (C7), 113.8 (C6), 109.6 (C3), 105.2 (C4), 65.8 (CH₂), 54.8 (CH₂), 53.0 (2CH₂), 24.1 (CH₂), 23.0 (2CH₂), 22.5 (CH₂). HRMS (ESI): Calcd for C₂₅H₂₉N₂O₅S⁺ [*M* + H]⁺: 469.1792. Found: 469.1792.

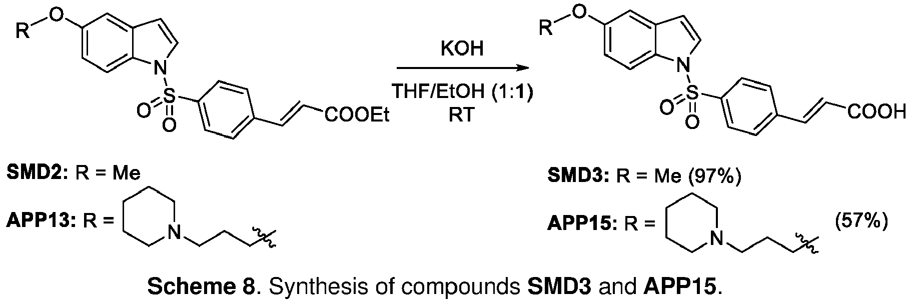

**(*E*)-3-(4-((5-Methoxy-1*H*-indol-1-yl)sulfonyl)phenyl)acrylic acid (SMD3).** Following the **General Method C,** starting from **SMD2** (255 mg, 0.66 mmol) after flash chromatography of the residue using DCM/Methanol (5%) as eluent, compound **SMD3** (230 mg, 97%) was obtained as a brown solid: mp 226-8 °C; IR (cm⁻¹): v 2971 (COO-H), 1694 (C=O), 1372 (O=S=O), 1150 (C-O-C); ¹H NMR (300 MHz, DMSO-d₆) *δ* 12.62 (s, 1H, COOH), 7.97 - 7.79 (m, 5H), 7.77 (d, *J=* 3.7 Hz, 1H, H2), 7.63 - 7.50 (m, 1H), 7.11 - 7.09 (m, 1H, H4), 6.94 (dd, *J* = 8.9, 2.6 Hz, 1H, H6), 6.78 (d, *J* = 3.7 Hz, 1H, H3), 6.63 (d, *J=* 16.1 Hz, 1H, CH=C*H*), 3.74 (s, 3H); ¹³C NMR (101 MHz, DMSO-d₆) *δ* 167.0 (COOH), 156.2, 141.4 (CH=CH), 140.0, 137.4, 131.7, 129.2 (2CH_{Ar}), 128.7, 127.8 (C2), 127.1 (2CH_{Ar}), 123.3 (CH=CH), 114.0 (C7), 113.7 (C6), 110.1 (C3), 104.0 (C4), 55.4 (OCH₃). HRMS (ESI): Calcd for C₁₈H₁₅NNaO₅S⁺ [*M* + Na]⁺: 380.0563. Found: 380.0574.

**(*E*)-3-(4-((5-(3-(Piperidin-1-yl)propoxy)-1*H*-indol-1-yl)sulfonyl)phenyl)acrylic acid (APP15).** Following the **General Method C,** starting from **APP13** (1 g, 2.01 mmol) after flash chromatography of the residue using DCM/Methanol/NH₄OH (98:1.8:0.2) as eluent, compound **APP15** (533 mg, 57%) was obtained as a brown solid: mp 73-5 °C; IR (cm⁻¹): v 3381 (COO-H), 1638 (C=O), 1364 (O=S=O), 1141 (CO-C); ¹H NMR (400 MHz, DMSO-d₆) δ 7.92 (d, *J* = 8.9 Hz, 2H), 7.85 (d, *J* = 8.9 Hz, 2H), 7.82 (d, *J=* 9.0 Hz, 1H, H7), 7.76 (d, *J=* 3.7 Hz, 1H, H2), 7.54 (d, *J* = 16.1 Hz, 1H, C*H*=CH), 7.11 (d, *J=* 2.5 Hz, 1H, H4), 6.94 (dd, *J* = 9.0, 2.5 Hz, 1H, H6), 6.76 (d, *J* = 3.7 Hz, 1H, H3), 6.63 (d, *J=* 16.1 Hz, 1H, CH=C*H*), 3.99 (t, *J=* 6.3 Hz, 2H), 2.66 - 2.53 (m, 6H), 1.95 - 1.92 (m, 2H), 1.57 - 1.53 (m, 4H), 1.43 - 1.40 (m, 2H) (the COOH signal could not be assigned); ¹³C NMR (101 MHz, DMSO-d₆) *δ* 167.6 (COOH), 155.9, 141.5 (CH=CH), 140.6, 137.8, 132.2, 129.6 (2CH_{Ar}), 129.2, 128.2 (C2), 127.5 (2CH_{Ar}), 124.3 (CH=CH), 114.6 (C6), 114.5 (C7), 110.6 (C3), 105.3 (C4), 66.5 (CH₂), 55.0 (CH₂), 53.9 (2CH₂), 25.8 (CH₂), 25.0 (2CH₂), 23.7 (CH₂). HRMS (ESI): Calcd for C₂₅H₂₉N₂O₅S⁺ [*M* + H]⁺: 469.1792. Found: 469.1797.

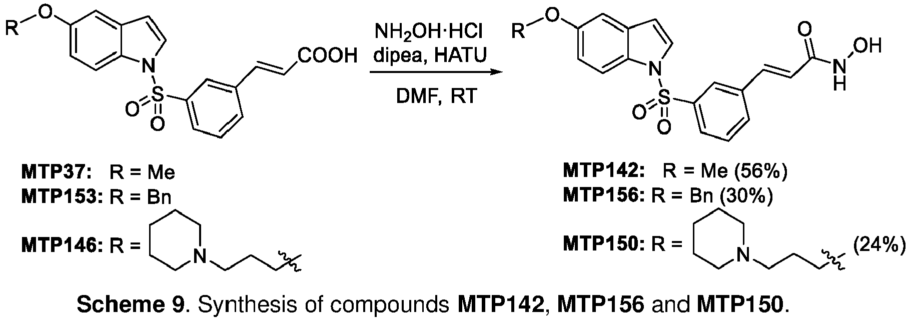

**(*E*)-*N*-MHydroxy-3-(3-((5-methoxy-1*H*-indol-1-yl)sulfonyl)phenyl)acrylamide (MTP142).** Following the **General Method D,** starting from **MTP37** (170 mg, 0.48 mmol) after flash chromatography of the residue using DCM/Methanol/NH₄OH (95:4.5:0.5) as eluent, compound **MTP142** (100 mg, 56%) was obtained as a white solid: mp 95-7 °C; IR (cm⁻¹) v 3403 (O-H), 2953 (N-H), 1611 (C=O), 1245 (O=S=O), 1171 (C-O-C); ¹H NMR (500 MHz, DMSO-d₆) δ 10.82 (s, 1H, N*H* or O*H*), 9.14 (s, 1H, N*H* or O*H*), 8.14 (d, *J=* 1.9 Hz, 1H), 7.92 - 7.81 (m, 3H), 7.77 (d, *J* = 3.7 Hz, 1H, H2), 7.59 (t, *J* = 7.9 Hz, 1H), 7.49 (d, *J* = 15.9 Hz, 1H, C*H*=CH), 7.11 (d, *J* = 2.6 Hz, 1H, H4), 6.95 (dd, *J* = 9.0, 2.6 Hz, 1H, H6), 6.78 (d, *J* = 3.7 Hz, 1H, H3), 6.55 (d, *J=* 15.9 Hz, 1H, CH=C*H*), 3.74 (s, 3H); ¹³C NMR (126 MHz, DMSO-d₆) δ 162.0, 156.2, 137.7, 136.5, 136.1 (*C*H=CH), 132.8 (CH_{Ar}), 131.7, 130.5 (CH_{Ar}), 128.7, 127.7 (C2), 126.9 (CH_{Ar}), 125.3 (CH_{Ar}), 122.0 (CH=*C*H), 114.0 (C7), 113.7 (C6), 110.0 (C3), 104.0 (C4), 55.4 (OCH₃). HRMS (ESI): Calcd for C₁₈H₁₆N₂NaO₅S⁺ [*M* + Na]⁺: 395.0672. Found: 395.0676.

**(*E*)-3-(3-((5-(Benzyloxy)-1*H*-indol-1-yl)sulfonyl)phenyl)-*N*-hydroxyacrylamide (MTP156).** Following the **General Method D,** starting from **MTP153** (180 mg, 0.42 mmol) after flash chromatography of the residue using DCM/Methanol/NH₄OH (96:3.6:0.4) as eluent, compound **MTP156** (54 mg, 30%) was obtained as a white solid: mp 75-7 °C; IR (cm⁻¹) v 3142 (O-H), 2919 (N-H), 1614 (C=O), 1369 (O=S=O), 1139 (C-O-C); ¹H NMR (500 MHz, DMSO-d₆) δ 8.14 (t, *J* = 1.8 Hz, 1H), 7.89 (ddd, *J* = 7.8, 1.9, 0.9 Hz, 1H), 7.87 - 7.83 (m, 2H), 7.78 (d, *J* = 3.7 Hz, 1H, H2), 7.59 (t, *J* = 7.8 Hz, 1H), 7.48 (d, *J* = 15.9 Hz, 1H, CH=CH), 7.46 - 7.40 (m, 2H), 7.41 - 7.34 (m, 2H), 7.35 - 7.28 (m, 1H), 7.20 (d, *J* = 2.5 Hz, 1H, H4), 7.03 (dd, *J* = 9.0, 2.5 Hz, 1H, H6), 6.77 (dd, *J* = 3.7, 0.9 Hz, 1H, H3), 6.56 (d, *J* = 15.9 Hz, 1H, CH=C*H*), 5.08 (s, 2H) (the CON*H*O*H* signal could not be assigned); ¹³C NMR (126 MHz, DMSO-d₆) δ 161.9, 155.2, 137.7, 137.1, 136.5, 136.0 (CH=CH), 132.8 (CH_{Ar}), 131.6, 130.5 (CH_{Ar}), 128.8, 128.4 (2CH_{Ar}), 127.8 (C2), 127.8, 127.7 (2CH_{Ar}), 126.9 (CH_{Ar}), 125.3 (CH_{Ar}), 122.1 (CH=CH), 114.3 (C6), 114.0 (C7), 110.0 (C3), 105.2 (C4), 69.6 (CH₂). HRMS (ESI): Calcd for C₂₄H₂₁N₂O₅S⁺ [*M* + H]⁺: 449.1166. Found: 449.1167.

**(*E*)-*N*-Hydroxy-3-(3-((5-(3-(piperidin-1-yl)propoxy)-1*H*-indol-1-yl)sulfonyl)phenyl)acrylamide (MTP150).** Following the **General Method D,** starting from **MTP146** (287 mg, 0.61 mmol) after flash chromatography of the residue using DCM/Methanol/NH₄OH (90:9:1) as eluent, compound **MTP150** (70 mg, 24%) was obtained as white solid: mp 108-10 °C; IR (cm⁻¹): v 3145 (O-H), 2931 (N-H), 1611 (C=O), 1369 (O=S=O), 1141 (C-O-C); ¹H NMR (500 MHz, DMSO-d₆) *δ* 8.13 (t, *J* = 1.9 Hz, 2H), 7.89 - 7.81 (m, 3H), 7.76 (d, *J* = 3.7 Hz, 1H, H2), 7.59 (t, *J* = 7.9 Hz, 1H), 7.48 (d, *J* = 15.9 Hz, 1H, CH=CH), 7.10 (d, *J* = 2.5 Hz, 1H, H4), 6.94 (dd, *J* = 9.0, 2.5 Hz, 1H, H6), 6.76 (d, *J* = 3.7 Hz, 1H, H3), 6.55 (d, *J* = 15.9 Hz, 1H, CH=C*H*), 3.97 (t, *J* = 6.3 Hz, 2H), 2.47 - 2.29 (m, 6H), 1.89 - 1.83 (m, 2H), 1.51 - 1.47 (m, 4H), 1.38 - 1.36 (m, 2H) (the CONHOH signal could not be assigned); ¹³C NMR (126 MHz, DMSO-d₆) *δ* 161.9, 155.5, 137.7, 136.5, 136.0 (CH=CH), 132.8 (CH_{Ar}), 131.7, 130.5 (CH_{Ar}), 128.6, 127.7 (C2), 126.9 (CH_{Ar}), 125.3 (CH_{Ar}), 122.1 (CH=CH), 114.2 (C6), 114.0 (C7), 110.1 (C3), 104.8 (C4), 66.2 (CH₂), 55.0 (CH₂), 53.9 (2CH₂), 26.1 (CH₂), 25.4 (2CH₂), 23.9 (CH₂). HRMS (ESI): Calcd for C₂₅H₃₀N₃O₅S⁺ [*M* + H]⁺: 484.1901. Found: 484.1902.

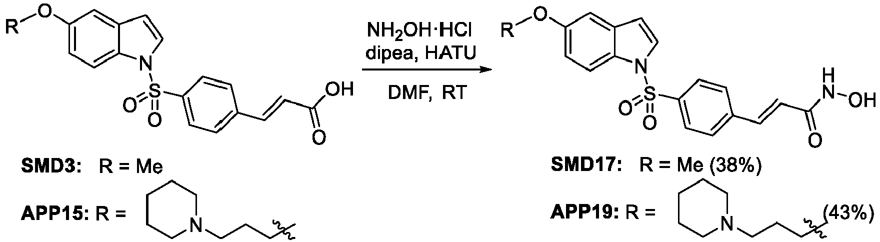

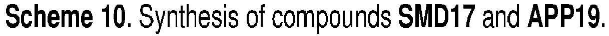

**(*E*)-*N-*Hydroxy-3-(4-((5-methoxy-1*H-*indol-1-yl)sulfonyl)phenyl)acrylamide (SMD17).** Following the **General Method D,** starting from **SMD3** (250 mg, 0.70 mmol) after flash chromatography of the residue using DCM/Methanol/NH₄OH (98:1.8:0.2) as eluent, compound **SMD17** (100 mg, 38%) was obtained as yellow solid: mp 135-7 °C; IR (cm⁻¹): v 3123 (O-H), 2989 (N-H), 1614 (C=O), 1376 (O=S=O), 1141 (C-O-C); ¹H NMR (400 MHz, DMSO-d₆) *δ* 7.93 (d, *J* = 8.6 Hz, 2H), 7.82 (d, *J* = 9.0 Hz, 1H, H7), 7.75 (d, *J* = 3.6 Hz, 1H, H2), 7.72 (d, *J =* 8.6 Hz, 2H), 7.43 (d, *J* = 15.9 Hz, 1H, CH=CH), 7.11 (d, *J* = 2.5 Hz, 1H, H4), 6.94 (dd, *J* = 9.0, 2.6 Hz, 1H, H6), 6.78 (dd, *J* = 3.6, 0.8 Hz, 1H, H3), 6.53 (d, *J=* 15.9 Hz, 1H, CH=C*H*), 3.74 (s, 3H) (the CONHOH signal could not be assigned); ¹³C NMR (101 MHz, DMSO-d₆) *δ* 161.8, 156.2, 140.7 (CH=CH), 136.8, 136.0, 131.7, 128.7, 128.5 (2CH_{Ar}), 127.7(C2), 127.3 (2CH_{Ar}), 123.2 (CH=CH), 114.0 (C7), 113.7 (C6), 110.1 (C3), 104.0 (C4), 55.0 (OCH₃). HRMS (ESI): Calcd for C₁₈H₁₆N₂NaO₅S⁺ [*M* + Na]⁺: 395.0672. Found: 395.0678.

**(*E*)-*N*-Hydroxy-3-(4-((5-(3-(piperidin-1-yl)propoxy)-1*H*-indol-1-yl)sulfonyl)phenyl)acrylamide (APP19).** Following the **General Method D,** starting from **APP13** (200 mg, 0.43 mmol) after flash chromatography of the residue using DCM/Methanol/NH₄OH (98:1.8:0.2) as eluent, compound **APP19** (89 mg, 43%) was obtained as yellow solid: mp 95-7 °C; IR (cm⁻¹): v 3175 (O-H), 2925 (N-H), 1613 (C=O), 1369 (O=S=O), 1143 (C-O-C); ¹H NMR (300 MHz, DMSO-d₆) *δ* 7.93 (d, *J* = 8.4 Hz, 2H), 7.81 (d, *J* = 9.0 Hz, 1H, H7), 7.75 - 7.71 (m, 3H), 7.43 (d, *J* = 15.8 Hz, 1H, CH=CH), 7.10 (d, *J* = 2.3 Hz, 1H, H4), 6.93 (dd, *J* = 9.0, 2.5 Hz, 1H, H6), 6.76 (d, *J* = 3.7 Hz, 1H, H3), 6.53 (d, *J* = 15.8 Hz, 1H, CH=C*H*), 3.97 (t, *J* = 6.3 Hz, 2H), 2.37 - 2.34 (m, 6H), 1.85 - 1.82 (m, 2H), 1.50 - 1.44 (m, 4H), 1.38 - 1.36 (m, 2H) (the CONHOH signal could not be assigned); ¹³C NMR (101 MHz, DMSO-d₆) *δ* 162.2, 156.1, 141.2, 137.3 (CH=CH), 136.5, 132.2, 129.2, 129.0 (2CH_{Ar}), 128.2 (C2), 127.8 (2CH_{Ar}), 123.7 (CH=CH), 114.7 (C6), 114.5 (C7), 110.6 (C3), 105.3 (C4), 66.8 (CH₂), 55.6 (CH₂), 54.5 (2CH₂), 26.7 (CH₂), 26.0 (2CH₂), 24.5 (CH₂). HRMS (ESI): Calcd for C₂₅H₃₀N₃O₅S⁺ [*M* + H]⁺: 484.1901. Found: 484.1900.

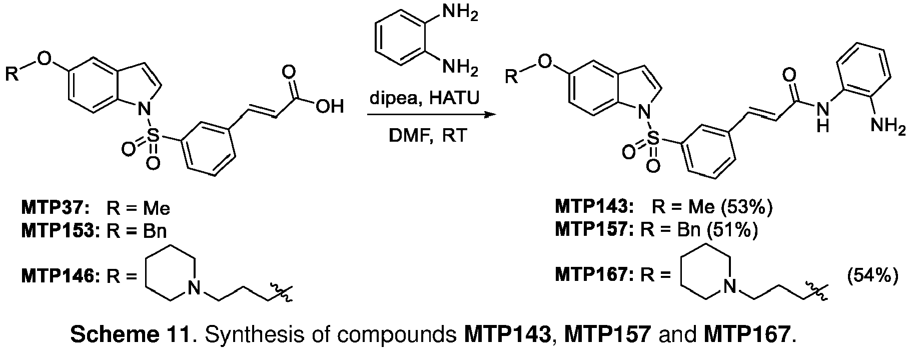

**(*E*)-*N*-(2-Aminophenyl)-3-(3-((5-methoxy-1*H*-indol-1-yl)sulfonyl)phenyl)acrylamide (MTP143).** Following the **General Method D,** starting from **MTP37** (150 mg, 0.42 mmol) after flash chromatography of the residue using DCM/Methanol/NH₄OH (97:2.7:0.3) as eluent, compound **MTP143** (100 mg, 53%) was obtained as yellow solid: mp 164-6 °C; IR (cm⁻¹): v 3227 (N-H), 1660 (C=O), 1361 (O=S=O), 1140 (C-O-C); ¹H NMR (400 MHz, DMSO-d₆) *δ* 9.44 (s, 1H, CON*H*), 8.21 (t, *J* = 1.8 Hz, 1H), 7.92 - 7.89 (m, 2H), 7.86 (d, *J* = 9.0 Hz, 1H, H7), 7.78 (d, *J* = 3.7 Hz, 1H, H2), 7.63 (t, *J* = 7.8 Hz, 1H), 7.58 (d, *J* = 15.8 Hz, 1H, CH=CH), 7.34 (dd, *J* = 7.8, 1.5 Hz, 1H), 7.12 (d, *J* = 2.5 Hz, 1H, H4), 7.00 (d, *J* = 15.8 Hz, 1H, CH=C*H*), 6.98 - 6.89 (m, 2H), 6.80 (dd, *J* = 3.7, 0.7 Hz, 1H, H3), 6.75 (dd, *J* = 8.0, 1.5 Hz, 1H), 6.58 (td, *J* = 7.5, 1.5 Hz, 1H), 4.96 (s, 2H, CONHC₆H₄NH₂), 3.74 (s, 3H,OCH₃); ¹³C NMR (101 MHz, DMSO-d₆) *δ* 162.9, 156.2, 141.6, 137.8, 137.2 (CH=CH), 136.6, 133.1 (CH_{Ar}), 131.7, 130.6 (CH_{Ar}), 128.7, 127.7 (C2), 127.0 (CH_{Ar}), 126.0 (CH_{Ar}), 125.2 (CH=CH), 124.7 (CH_{Ar}), 123.2, 116.3 (CH_{Ar}), 116.0 (CH_{Ar}), 114.0 (C7), 113.8 (C6), 110.1 (C3), 104.1 (C4), 55.4 (OCH₃). HRMS (ESI): Calcd for C₂₄H₂₂N₃O₄S⁺ [M + H]⁺: 448.1326. Found: 448.1323.

**(*E*)-*N*-(2-Aminophenyl)-3-(3-((5-(benzyloxy)-1*H*-indol-1-yl)sulfonyl)phenyl)acrylamide (MTP157).** Following the **General Method D,** starting from **MTP153** (100 mg, 0.28 mmol) after flash chromatography of the residue using DCM/Methanol/NH₄OH (98:1.8:0.2) as eluent, compound **MTP157** (74 mg, 51%) was obtained as yellow solid: mp 148-50 °C; IR (cm⁻¹): v 3216 (N-H), 1613 (C=O), 1356 (O=S=O), 1153 (C-O-C); ¹H NMR (400 MHz, DMSO-d₆) *δ* 9.43 (s, 1H, CON*H*), 8.21 (d, *J* = 1.8 Hz, 1H), 7.95 - 7.87 (m, 2H), 7.87 (d, *J* = 9.0 Hz, 1H, H7), 7.78 (d, *J* = 3.7 Hz, 1H, H2), 7.64 (t, *J* = 7.9 Hz, 1H), 7.59 (d, *J* = 15.9 Hz, 1H, CH=CH), 7.47 - 7.28 (m, 6H), 7.21 (d, *J* = 2.5 Hz, 1H, H4), 7.05 (dd, *J* = 9.0, 2.5 Hz, 1H, H6), 7.00 (d, *J* = 15.9 Hz, 1H, CH=C*H*), 6.93 (td, *J* = 7.7, 7.3, 1.5 Hz, 1H), 6.79 (dd, *J* = 3.7, 0.7 Hz, 1H, H3), 6.75 (dd, *J* = 8.0, 1.5 Hz, 1H), 6.58 (td, *J* = 7.5, 1.5 Hz, 1H), 5.09 (s, 2H), 4.96 (s, 2H, CONHC₆H₄NH₂); ¹³C NMR (101 MHz, DMSO-d₆) *δ* 162.9, 155.3, 141.6, 137.8, 137.2 (CH=CH), 137.1, 136.6, 133.1 (CH_{Ar}), 131.6, 130.6 (CH_{Ar}), 128.8, 128.4 (2CH_{Ar}), 127.8 (C2), 127.7 (CH_{Ar}), 127.7 (2CH_{Ar}), 127.0 (CH_{Ar}), 126.0 (CH_{Ar}), 125.3 (CH=CH), 125.2 (CH_{Ar}), 124.7 (CH_{Ar}), 123.2, 116.3 (CH_{Ar}), 116.0 (CH_{Ar}), 114.4 (C6), 114.0 (C7), 110.0 (C3), 105.3 (C4), 69.6 (CH₂). HRMS (ESI): Calcd for C₃₀H₂₆N₃O₄S⁺ [*M* + H]⁺: 524.1639. Found: 524.1638.

**(*E*)-*N*-(2-Aminophenyl)-3-(3-((5-(3-(piperidin-1-yl)propoxy)-1*H*-indol-1-yl)sulfonyl)phenyl)acrylamide (MTP167).** Following the **General Method D,** starting from **MTP146** (166 mg, 0.35 mmol) after flash chromatography of the residue using DCM/Methanol/NH₄OH (97:2.7:0.3) as eluent, compound **MTP167** (107 mg, 54%) was obtained as yellow solid: mp 89-91 °C; IR (cm⁻¹): v 2936 (N-H), 1620 (C=O), 1371 (O=S=O), 1153 (C-O-C); ¹H NMR (400 MHz, DMSO-d₆) *δ* 9.44 (s, 1H, CON*H*), 8.20 (s, 1H), 7.93 - 7.89 (m, 2H), 7.85 (d, *J* = 8.8 Hz, 1H, H7), 7.77 (d, *J* = 3.6 Hz, 1H, H2), 7.68 - 7.52 (m, 3H), 7.34 (d, *J* = 8.1 Hz 1H), 7.11 - 7.10 (m, 1H, H4), 7.00 (d, *J* = 15.8 Hz, 1H, CH=C*H*), 6.96 - 6.89 (m, 2H), 6.80 - 6.74 (m, 2H), 6.58 (t, *J* = 7.2 Hz, 1H), 4.96 (s, 2H, CONHC₆H₄NH₂), 3.98 (t, *J* = 6.3 Hz, 2H), 3.30 - 3.20 (m, 6H), 1.94 - 1.83 (m, 2H), 1.54 - 1.48 (m, 4H), 1.43 - 1.34 (m, 2H); ¹³C NMR (101 MHz, DMSO-d₆) δ 162.9, 155.5, 141.6, 137.8, 137.2 (CH=CH), 136.6, 133.1 (CH_{Ar}), 131.7, 130.6 (CH_{Ar}), 128.7, 127.7 (C2), 127.0 (CH_{Ar}), 126.0 (CH_{Ar}), 125.3 (CH_{Ar}), 125.2 (CH=CH), 124.7 (CH_{Ar}), 123.2, 116.3 (CH_{Ar}), 116.0 (CH_{Ar}), 114.2 (C6), 114.0 (C7), 110.1 (C3), 104.8 (C4), 66.1 (CH₂), 54.8 (CH₂), 53.7 (2CH₂), 25.7 (CH₂), 25.0 (2CH₂), 23.6 (CH₂). HRMS (ESI): Calcd for C₃₁H₃₅N₄O₄S⁺ [*M* + H]⁺: 559.2374. Found: 559.2372.

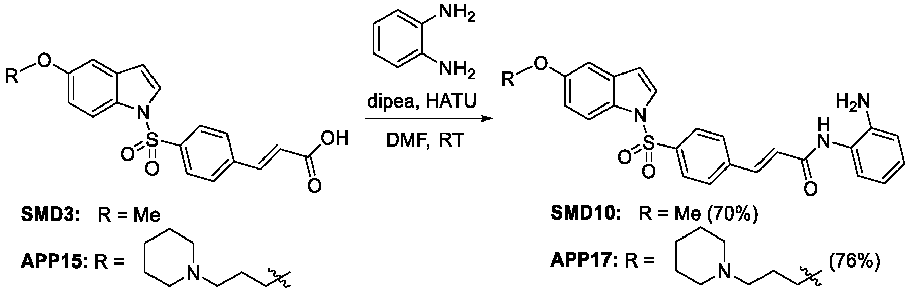

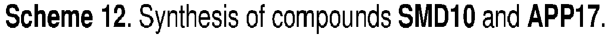

**(*E*)-*N*-(2-Aminophenyl)-3-(4-((5-methoxy-1*H*-indol-1-yl)sulfonyl)phenyl)acrylamide (SMD10).** Following the **General Method D,** starting from **SMD3** (190 mg, 0.53 mmol) after flash chromatography of the residue using DCM/Methanol (2%) as eluent, compound **SMD10** (167 mg, 70%) was obtained as yellow solid: mp 94-6 °C; IR (cm⁻¹): v 3257 (N-H), 1619 (C=O), 1371 (O=S=O), 1173 (C-O-C); ¹H NMR (400 MHz, DMSO-d₆) *δ* 9.48 (s, 1H, CON*H*), 7.99 (d, *J* = 8.3 Hz, 2H), 7.84 (d, *J* = 9.0 Hz, 1H, H7), 7.82 - 7.72 (m, 3H), 7.53 (d, *J* = 15.8 Hz, 1H, CH=CH), 7.32 (d, *J* = 7.1 Hz, 1H), 7.12 (d, *J* = 2.5 Hz, 1H, H4), 7.01 - 6.88 (m, 3H), 6.79 (d, *J* = 3.7 Hz, 1H, H3), 6.74 (dd, *J* = 7.9, 1.4 Hz, 1H), 6.56 (t, *J* = 7.5 Hz, 1H), 4.94 (s, 2H, CONHC₆H₄NH₂), 3.75 (s, 3H); ¹³C NMR (101 MHz, DMSO-d₆) *δ* 162.8, 156.2, 141.6, 140.7, 137.2 (CH=CH), 136.9, 131.7, 128.7, 128.6 (2CH_{Ar}), 127.7 (C2), 127.4 (2CH_{Ar}), 126.4 (CH_{Ar}), 126.0 (CH=CH), 124.8 (CH_{Ar}), 123.2, 116.3 (CH_{Ar}), 116.0 (CH_{Ar}), 114.0 (C7), 113.7 (C6), 110.0 (C3), 104.0 (C4), 55.4 (OCH₃). HRMS (ESI): Calcd for C₂₄H₂₂N₃O₄S⁺ [*M* + H]⁺: 448.1326. Found: 448.1334.

**(*E*)-*N*-(2-Aminophenyl)-3-(4-((5-(3-(piperidin-1-yl)propoxy)-1*H*-indol-1-yl)sulfonyl)phenyl)acrylamide (APP17).** Following the **General Method D,** starting from **APP15** (200 mg, 0.43 mmol) after flash chromatography of the residue using DCM/Methanol (2%) as eluent, compound **APP17** (181 mg, 76%) was obtained as yellow solid: mp 100-2 °C; IR (cm⁻¹): v 2931 (N-H), 1619 (C=O), 1455 (O=S=O), 1152 (C-O-C); ¹H NMR (500 MHz, DMSO-d₆) *δ* 9.50 (s, 1H, CON*H*), 7.98 (d, *J* = 8.6 Hz, 2H), 7.83 (d, *J* = 9.0 Hz, 1H, H7), 7.78 - 7.76 (m, 3H), 7.53 (d, *J* = 15.8 Hz, 1H, CH=CH), 7.32 (dd, *J* = 8.0, 1.5 Hz 1H), 7.12 (d, *J* = 2.5 Hz, 1H, H4), 6.99 (d, *J* = 15.8 Hz, 1H, CH=C*H*), 6.95 - 6.87 (m, 2H), 6.78 (dd, *J* = 3.7, 0.8 Hz, 1H, H3), 6.74 (dd, *J* = 8.0, 1.5 Hz, 1H), 6.56 (td, *J* = 7.6, 1.5 Hz, 1H), 4.95 (s, 2H, CONHC₆H₄NH₂), 3.99 (t, *J* = 6.1 Hz, 2H), 2.36 - 2.35 (m, 6H), 1.90 - 1.89 (m, 2H), 1.54 - 1.50 (m, 4H), 1.38 - 1.34 (m, 2H); ¹³C NMR (126 MHz, DMSO-d₆) *δ* 163.2, 155.9, 142.1, 141.2, 137.6 (CH=CH), 137.4, 132.1, 129.2 (C2), 129.0 (2CH_{Ar}), 128.2, 127.8 (2CH_{Ar}), 126.8 (CH=CH), 126.5 (CH_{Ar}), 125.2 (CH_{Ar}), 123.6, 116.7 (CH_{Ar}), 116.5 (CH_{Ar}), 114.6 (C7), 114.5 (C6), 110.5 (C3), 105.3 (C4), 66.5 (CH₂), 55.2 (CH₂), 54.1 (2CH₂), 26.1 (CH₂), 25.3 (2CH₂), 23.9 (CH₂). HRMS (ESI): Calcd for C₃₁H₃₅N₄O₄S⁺ [*M* + H]⁺: 559.2374. Found: 559.2381.

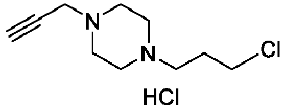

**1-(3-Chloropropyl)-4-(prop-2-yn-1-yl)piperazine hydrochloride (MTP36).** To a solution of piperazine (3 g, 34.88 mmol, 2.0 equiv) in anhydrous DCM (26 mL), cooled at 0 °C, di-*tert*-butyl dicarbonate (4 g, 17.44 mmol, 1.0 equiv) was added and the mixture was stirred at room temperature (rt) overnight. After complete reaction (TLC analysis), the solvent was evaporated in vacuo and water (10 mL) was added to the residue. The solution was filtrated and washed with distilled water (20 mL) was added, and the mixture was extracted with diethyl ether (3 × 20 mL). Combined organic extracts were washed with water (3 × 20 mL), dried over anhydrous magnesium sulfate and evaporated (Roy, D.; Panda, G. Base-Mediated 1,6-Aza-Michael addition of heterocyclic amines and amides to para-quinone methides leading to meclizine-, hydroxyzine- and cetirizine-like architectures. Synthesis 2019, 51, 4434-4442).

To a solution of tert-butyl piperazine-1-carboxylate (2 g, 10.75 mmol, 1.0 equiv) in anhydrous DCM (38 mL), cooled at 0 °C, triethylamine (3mL, 20.97 mmol, 2.6 equiv) and 1-bromo-3-chloropropane (2 mL, 20.97 mmol, 10 mmol) were added. The resulting mixture was stirred at 50 °C for overnight. After complete reaction (TLC analysis), the residue was added brine solution and extracted with DCM (3 × 20 mL). Combined organic layer was dried over anhydrous magnesium sulfate and concentrated in vacuo (Shi-Jun, Z.; Hua-Zhou, Y.; Yan, W.; Ni, Q.; Tao, L.; Bo, Y.; Xiao-Wu, D.; Yong-Zhou, Hu. Design, synthesis and biological evaluation of novel podophyllotoxin derivatives bearing 4β-disulfide/trisulfide bond as cytotoxic agents, RSC Adv., 2015, 5, 103172-103183).

A mixture of *tert*-butyl 4-(3-chloropropyl)piperazine-1 -carboxylate (2 g, 7.63 mmol, 1.0 equiv) and hydrochloric acid in ethanol (36 mL, 28.70 mmol, 4 equiv) was disolved in ethanol (4 mL) Then, the reaction mixture was stirred at room temperature (rt) for 16 h. After complete reaction (TLC analysis), the mixture was extracted with diethyl ether (3 × 20 mL). Combined organic extracts were dried over anhydrous magnesium sulfate and evaporated.

A mixture of 1-(3-chloropropyl)piperazine hydrochloride (2 g, 8.49 mmol, 1.0 equiv), triethylamine (3 mL, 20.40 mmol, 3.0 equiv) and propargyl bromide (1 mL, 7.47 mmol, 1.1equiv) was disolved in DCM (1 mL). Then, the reaction mixture was stirred at room temperature for 24 h. After complete reaction (TLC analysis), distilled water (20 mL) was added to and it was extracted with chloroform (3 × 20 mL). Combined organic extracts were washed with brine (15 mL), dried over anhydrous magnesium sulfate and evaporated. Product was purified by flash chromatography over silica gel, using DCM/methanol (98:2) as eluent to give compound **MTP36** (1 g, 74%) as a white solid: ¹H NMR (300 MHz, CDCl₃) δ 3.60 (t, *J* = 6.6 Hz, 2H), 3.30 (d, *J* = 2.4 Hz, 2H), 2.69 - 4.43 (m, 10H), 2.25 (t, *J* = 2.4 Hz, 1H), 2.00 - 1.91 (m, 2H).

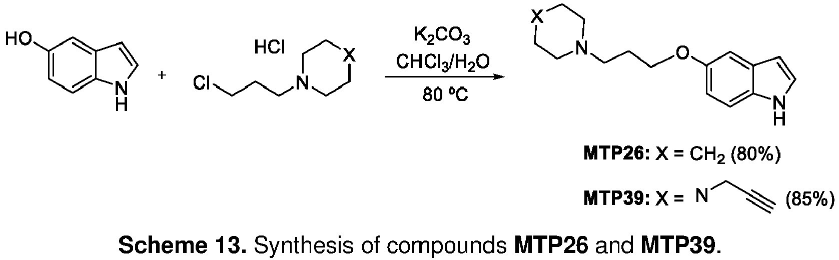

**5-(3-(Piperidin-1-yl)propoxy)-1*H*-indole (MTP26).** (Nyantakyi, S. A.; Li, M.; Gopal, P.; Zimmerman, M.; Dartois, V.; Gengenbacher, M.; Dick, T.; Go, M.-L. Indolyl azaspiroketal mannich bases are potent antimycobacterial agents with selective membrane permeabilizing effects and in vivo activity. J. Med. Chem. 2018, 61, 5733-5750). Following the **General procedure E,** starting from commercial 1*H*-indol-5-ol (1 g, 7.51 mmol), after flash chromatography of the residue using DCM/Methanol (5%) as eluent, gave compound **MTP26** (1550 mg, 80%) as a white solid: ¹H NMR (300 MHz, CDCl₃) δ 8.31 (s, 1H, N*H*), 7.27 (d, *J* = 8.8 Hz, 1H, H7), 7.18 (t, *J* = 2.8 Hz, 1H, H2), 7.11 (d, *J* = 2.4 Hz, 1H, H4), 6.86 (dd, *J* = 8.8, 2.5 Hz, 1H, H6), 6.47 (s, 1H, H3), 4.05 (t, *J* = 6.4 Hz, 2H), 2.58 - 2.44 (m, 6H), 2.09 - 1.97 (m, 2H), 1.67 - 1.60 (m, 4H), 2.50 - 1.42 (m, 2H). ¹³C NMR (101 MHz, CDCl₃) *δ* 153.5, 131.0, 128.3, 124.8 (C2), 112.9 (C6), 111.6 (C7), 103.6 (C3), 102.3 (C4), 67.4 (CH₂), 56.2 (CH₂), 54.6 (2CH₂), 27.0 (CH₂), 26.0 (2CH₂), 24.4 (CH₂).

**5-(3-(4-(Prop-2-yn-1-yl)piperazin-1-yl)propoxy)-1*H*-indole (MTP39).** Following the **General procedure E,** starting from commercial 5-hydroxi-1*H*-indol (310 mg, 2.33 mmol), after flash chromatography of the residue using DCM/Methanol (5%) as eluent, gave compound **MTP39** (590 mg, 85%) as a white solid: mp 144-6 °C; IR (cm⁻¹) v 1265 (C-O-C); ¹H NMR (300 MHz, CDCl₃) δ 8.07 (s, 1H, N*H*), 7.27 (s, 1H), 7.17 (t, *J* = 2.8 Hz, 1H), 7.09 (d, *J* = 2.4 Hz, 1H), 6.82 (dd, *J* = 8.8, 2.4 Hz, 1H), 6.48 - 6.42 (m, 1H), 4.04 (t, *J* = 6.3 Hz, 2H), 3.29 (d, *J* = 2.5 Hz, 2H), 2.64 - 2.57 (m, 10H), 2.23 (t, *J* = 2.4 Hz, 1H), 2.16 - 1.81 (m, 2H); ¹³C NMR (101 MHz, CDCl₃) δ 153.5, 131.0, 128.3, 124.8 (C2), 112.9 (C7), 111.6 (C6), 103.6 (C3), 102.4 (C4), 78.8, 73.2 (CH), 67.0 (CH₂), 55.3 (2CH₂), 53.0 (2CH₂), 51.7 (CH₂), 46.8 (CH₂), 26.8 (CH₂); HRMS (ESI): Calcd for C₁₉H₂₃N₃O⁺ [M + *H*]⁺: 298.1914. Found: 298.1924. Anal. Calcd for C₁₈H₂₃N₃O·1/5H₂O: C, 70.56; H, 7.90; N, 13.71. Found: C, 70.72; H, 7.65; N, 13.58.

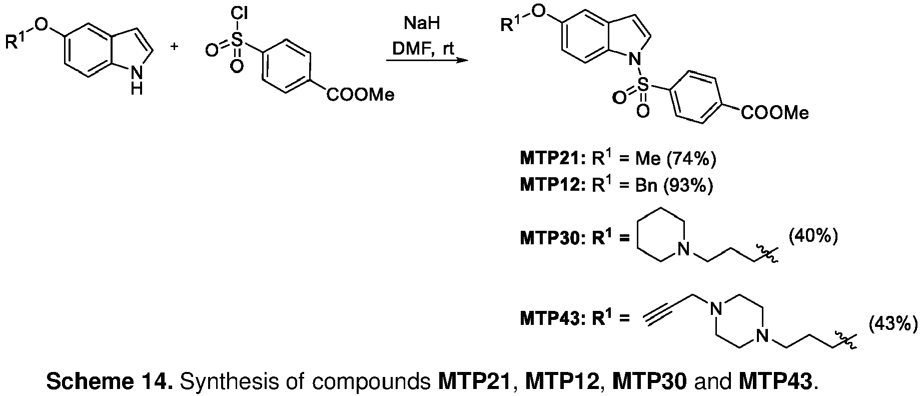

**Methyl 4-((5-methoxy-1*H*-indol-1-yl)sulfonyl)benzoate (MTP21).** Following the **General procedure A,** starting from commercial 5-methoxy-1*H*-indole (400 mg, 2.72 mmol), after flash chromatography of the residue using hexane/AcOEt (12:1) as eluent, gave compound **MTP21** (740 mg, 79%) as a white solid: mp 106-8 °C; IR (cm⁻¹) v 1613 (C=O), 1375 (O=S=O), 1180 (C-O-C),; ¹H NMR (400 MHz, CDCl₃) δ 8.07 (d, *J* = 8.6 Hz, 2H), 7.91 - 7.87 (m, 3H), 7.51 (d, *J* = 3.7 Hz, 1H, H2), 6.97 (d, *J* = 2.5 Hz, 1H, H4), 6.94 (dd, *J* = 9.0, 2.5 Hz, 1H, H6), 6.62 (dd, *J* = 3.7, 0.8 Hz, 1H, H3), 3.91 (s, 3H), 3.81 (s, 3H); ¹³C NMR (101 MHz, CDCl₃) δ 165.2, 156.7, 141.7, 134.7, 131.9, 130.3 (2CH_{Ar}), 129.5, 127.0 (C2), 126.7 (2CH_{Ar}), 114.4(C7), 114.0 (C6), 110.1 (C3), 103.9 (C4), 55.6 (CH₃), 52.7 (CH₃); Anal. Calcd for C₁₇H₁₅NO₅S: C, 59.12; H, 4.38; N, 4.06; S, 9.28. Found: C, 59.44; H, 4.58; N, 4.13; S, 9.00.

**Methyl 4-((5-(benzyloxy)-1H-indol-1-yl)sulfonyl)benzoate (MTP12).** Following the **General procedure A,** starting from commercial 5-(benzyloxy)-1*H*-indole (400 mg, 1.79 mmol), after flash chromatography of the residue using hexane/AcOEt (12:1) as eluent, gave compound **MTP12** (535 mg, 71%) as a white solid: mp 113-5 °C; IR (cm⁻¹) v 1731 (C=O), 1359 (O=S=O), 1162 (C-O-C); ¹H NMR (400 MHz, CDCl₃) *δ* 8.08 (d, *J* = 8.6 Hz, 2H), 7.92 - 7.88 (m, 3H), 7.51 (d, *J* = 3.7 Hz, 1H, H2), 7.45 - 7.33 (m, 5H), 7.05 - 7.01 (m, 2H, H4 and H6), 6.61 (dd, *J* = 3.7, 0.8 Hz, 1H, H3), 5.07 (s, 2H), 3.91 (s, 3H); ¹³C NMR (101 MHz, CDCl₃) δ 165.2, 155.9, 141.7, 136.9, 134.7, 131.8, 130.4 (2CH_{Ar}), 129.6, 128.6 (2CH_{Ar}), 128.0 (CH_{Ar}), 127.4 (2CH_{Ar}), 127.0 (C2), 126.7 (2CH_{Ar}), 114.7 (C7), 114.4 (C6), 110.1 (C3), 105.2 (C4), 70.5 (CH₂), 52.7 (CH₃); Anal. Calcd for C₂₃H₁₉NO₅S: C, 65.55; H, 4.54; N, 3.32; S, 7.61. Found: C, 65.67; H, 4.83; N, 3.33; S, 7.49.

**Methyl 4-((5-(3-(piperidin-1-yl)propoxy)-1H-indol-1-yl)sulfonyl)benzoate (MTP30).** Following the **General procedure A,** starting from 5-(3-(piperidin-1-yl)propoxy)-1*H*-indole (Nyantakyi, S. A. et al. J. Med. Chem. 2018, 61, 5733) (450 mg, 1.74 mmol), after flash chromatography of the residue using DCM/Methanol (2%) as eluent, gave compound **MTP30** (700 mg, 88%) as a white solid: mp 100-2 °C; IR (cm⁻¹) v 1613 (C=O), 1376 (O=S=O), 1156 (C-O-C); ¹H NMR (400 MHz, CDCl₃) δ 8.06 (d, *J* = 8.6 Hz, 2H), 7.89 (d, *J* = 8.6 Hz, 2H), 7.86 (d, *J* = 9.0 Hz, 1H, H7), 7.49 (d, *J* = 3.6 Hz, 1H, H2), 6.96 (d, *J* = 2.5 Hz, 1H, H4), 6.91 (dd, *J* = 9.0, 2.5 Hz, 1H, H6), 6.60 (dd, *J* = 3.6, 0.7 Hz, 1H, H3), 4.01 (t, *J* = 6.2 Hz, 2H), 3.90 (s, 3H), 2.66 - 2.50 (m, 6H), 2.10 - 2.02 (m, 2H), 1.71 - 1.65 (m, 4H), 1.51 - 1.47 (m, 2H); ¹³C NMR (101 MHz, CDCl₃) δ 165.2, 155.9, 141.6, 134.7, 131.8, 130.3 (2CH_{Ar}), 129.4, 126.9 (C2), 126.7 (2CH_{Ar}), 114.4 (C7), 114.3 (C6), 110.1 (C3), 104.7 (C4), 66.6 (CH₂), 55.9 (CH₂), 54.4 (2CH₂), 52.7 (CH₃), 26.2 (CH₂), 25.3 (2CH₂), 23.9 (CH₂); Anal. Calcd for C₂₄H₂₈N₂O₅S·7/2H₂O: C, 55.48; H, 5.99; N, 5.39; S, 6.17. Found: C, 55.81; H, 5.63; N, 5.45; S, 6.54.

**Methyl 4-((5-(3-(4-(prop-2-yn-1-yl)piperazin-1-yl)propoxy)-1*H*-indol-1-yl)sulfonyl) benzoate (MTP43).** Following the **General procedure A,** starting from 5-(3-(4-(prop-2-yn-1-yl)piperazin-1-yl)propoxy)-1*H-*indole (400 mg, 1.34 mmol), after flash chromatography of the residue using DCM/Methanol (4%) as eluent, gave compound **MTP43** (390 mg, 59%) as a white solid: mp 93-5 °C; IR (cm⁻¹) v 1612 (C=O), 1375 (O=S=O), 1155 (C-O-C); ¹H NMR (400 MHz, CDCl₃) δ 8.07 (d, *J* = 8.6 Hz, 2H), 7.90 (d, *J* = 8.6 Hz, 2H), 7.86 (d, *J* = 9.0 Hz, 1H, H7), 7.50 (d, *J* = 3.7 Hz, 1H, H2), 6.97 (d, *J* = 2.5 Hz, 1H, H4), 6.93 (dd, *J* = 9.0, 2.5 Hz, 1H, H6), 6.60 (dd, *J* = 3.7, 0.8 Hz, 1H, H3), 4.01 (t, *J* = 6.3 Hz, 2H), 3.90 (s, 3H), 3.31 (d, *J* = 2.5 Hz, 2H), 2.69 - 2.46 (m, 10H), 2.26 (t, *J* = 2.5 Hz, 1H), 2.01 - 1.97 (m, 2H); ¹³C NMR (101 MHz, CDCl₃) δ 165.2, 156.0, 141.6, 134.7, 131.8, 130.3 (2CH_{Ar}), 129.4, 126.9 (C2), 126.7 (2CH_{Ar}), 114.4 (C7), 114.3 (C6), 110.1 (C3), 104.7 (C4), 78.7, 73.2 (CH), 66.6 (CH₂), 55.1 (2CH₂), 53.0 (CH₃), 52.7 (2CH₂), 51.7 (CH₂), 46.8 (CH₂), 26.7 (CH₂); Anal. Calcd for C₂₆H₂₉N₃O₅S: C, 63.01; H, 5.90; N, 8.48; S, 6.47. Found: C, 63.05; H, 5.99; N, 8.57; S, 6.00.

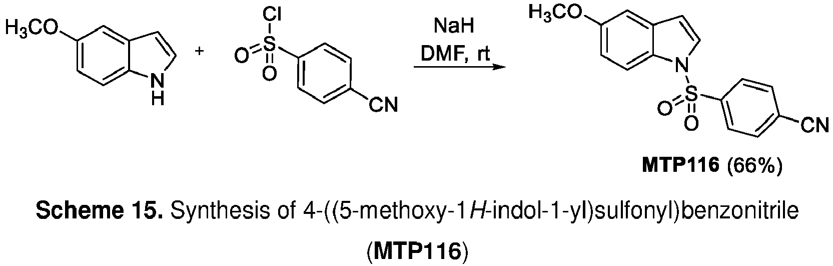

**4-((5-Methoxy-1*H*-indol-1-yl)sulfonyl)benzonitrile (MTP116).** Following the **General procedure A,** starting from commercial 5-methoxyindole (200 mg, 1.36 mmol), after flash chromatography of the residue using hexane/AcOEt (6:1) as eluent, gave compound **MTP116** (280 mg, 66%) as a white solid: mp 139-41 °C; IR (cm⁻¹) v 1615 (C=O), 1376 (O=S=O), 1151 (C-O-C); ¹H NMR (400 MHz, CDCl₃) δ 7.94 (d, *J* = 8.8 Hz, 2H), 7.86 (d, *J* = 9.0 Hz, 1H, H7), 7.72 (d, *J* = 8.8 Hz, 2H), 7.47 (d, *J* = 3.7 Hz, 1H, H2 ), 7.02 - 6.96 (m , 1H, H4), 6.95 (dd, *J* = 9.0, 2.6 Hz, 1H, H6), 6.65 (dd, *J* = 3.7, 0.8 Hz, 1H, H3), 3.82 (s, 3H); ¹³C NMR (101 MHz, CDCl₃) δ 156.9, 141.8, 133.0 (2CH_{Ar}), 132.0, 129.3, 127.2 (2CH_{Ar}), 126.8 (C2), 117.4, 116.9, 114.3 (C7), 114.2 (C6), 110.7 (C3), 104.0 (C4), 55.6 (CH₃); HRMS (ESI): Calcd for C₁₆H₁₃N₂O₃S⁺ [M + *H*]⁺: 313.0641. Found: 313.0629.

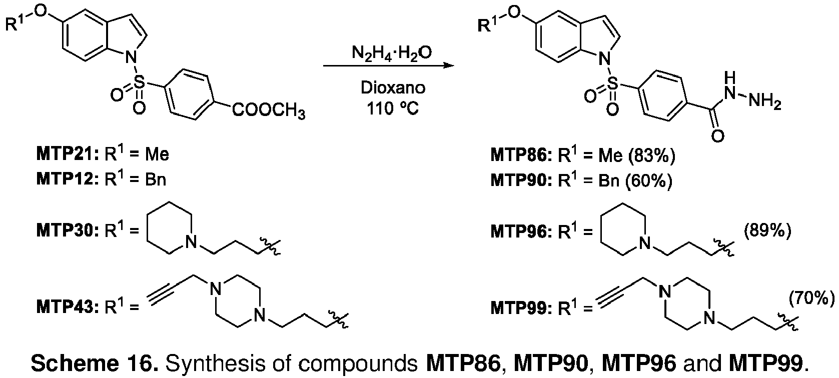

**4-((5-Methoxy-1*H*-indol-1-yl)sulfonyl)benzohydrazide (MTP86).** Following the **General procedure F,** starting from methyl 4-((5-methoxy-1*H*-indol-1-yl)sulfonyl)benzoate (100 mg, 0.29 mmol), after flash chromatography of the residue using DCM/Methanol (3%) as eluent, gave compound MTP86 (83 mg, 83%) as a brown solid: mp 74-6 °C; IR (cm⁻¹) v 3435 (N-H), 1643 (C=O), 1376 (O=S=O), 1152 (C-O-C); ¹H NMR (400 MHz, CD₃OD) δ 7.87 (d, *J* = 8.2 Hz, 2H), 7.80 (d, *J* = 9.0 Hz, 1H, H7), 7.74 (d, *J* = 8.2 Hz, 2H), 7.52 (d, *J* = 3.7 Hz, 1H, H2), 6.95 (d, *J* = 2.5 Hz, 1H, H4), 6.85 (dd, *J* = 9.0, 2.5 Hz, 1H, H6), 6.60 (d, *J* = 3.7 Hz, 1H, H3), 3.69 (s, 3H) (signal for "N*H*" and "N*H₂*" were not detected); ¹³C NMR (101 MHz, CD₃OD) δ 167.6, 158.2, 141.4, 139.4, 133.5, 130.7, 129.1 (2CH_{Ar}), 128.4 (C2), 128.0 (2CH_{Ar}), 115.4 (C7), 114.8 (C6), 111.2 (C3), 104.9 (C4), 56.0 (CH₃); HRMS (ESI): Calcd for C₁₆H₁₆N₃O₄S⁺ [M + *H*]⁺: 346.0856. Found: 346.0843. Anal. Calcd for C₁₆H₁₅N₃O₄S·1/2H₂O: C, 54.23; H, 5.00; N, 11.86; S, 8.99. Found: C, 54.85; H, 5.62; N, 11.52; S, 8.95.

**4-((5-(Benzyloxy)-1*H-*indol-1-yl)sulfonyl)benzohydrazide (MTP90).** Following the **General procedure F,** starting from methyl 4-((5-(benzyloxy)-1*H*-indol-1-yl)sulfonyl)benzoate (100 mg, 0.24 mmol), after flash chromatography of the residue using DCM/Methanol (3%) as eluent, gave compound **MTP90** (60 mg, 60%) as a white solid: mp 220-2 °C; IR (cm⁻¹) v 3419 (N-H), 1645 (C=O), 1365 (O=S=O), 1156 (C-O-C); ¹H NMR (400 MHz, DMSO-d₆) δ 9.95 (s, 1H, N*H*), 8.02 (d, *J* = 8.6 Hz, 2H), 7.91 (d, *J* = 8.6 Hz, 2H), 7.84 (d, *J* = 9.0 Hz, 1H, H7), 7.78 (d, *J* = 3.7 Hz, 1H, H2), 7.45 - 7.41 (m, 2H), 7.40 - 7.36 (m, 2H), 7.33 - 7.29 (m, 1H), 7.20 (d, *J* = 2.5 Hz, 1H, H4), 7.03 (dd, *J* = 9.0, 2.5 Hz, 1H, H6), 6.78 (dd, *J* = 3.7, 0.8 Hz, 1H, H3), 5.08 (s, 2H), 4.56 (s, 2H, N*H*₂); ¹³C NMR (101 MHz, DMSO-d₆) δ 164.1, 155.3, 138.8, 138.7, 137.1, 131.7, 128.8 (CH_{Ar}), 128.5 (3CH_{Ar}), 128.3 (CH_{Ar}), 127.8 (CH_{Ar}), 127.8, 127.7 (3CH_{Ar}), 126.9 (CH_{Ar}), 114.4 (C6), 114.0 (C7), 110.2 (C3), 105.3 (C4), 69.6 (CH₂); HRMS (ESI): Calcd for C₂₂H₁₉N₃NaO₄S⁺ [M + *Na*]⁺: 444.0988. Found: 444.0996. Anal. Calcd for C₂₂H₁₉N₃O₄S·H₂O: C, 60.13; H, 4.82; N, 9.56; S, 7.29. Found: C, 60.15; H, 4.54; N, 9.33; S, 7.12.

**4-((5-(3-(Piperidin-1-yl)propoxy)-1*H*-indol-1-yl)sulfonyl)benzohydrazide (MTP96).** Following the **General procedure F,** starting from methyl 4-((5-(3-(piperidin-1-yl)propoxy)-1*H*-indol-1-yl)sulfonyl)benzoate (90 mg, 0.20 mmol), after flash chromatography of the residue using DCM/Methanol (6%) as eluent, gave compound **MTP96** (80 mg, 89%) as a brown solid: mp 133-5 °C; IR (cm⁻¹) v 3429 (N-H), 1641 (C=O), 1418 (O=S=O), 1265 (C-O-C); ¹H NMR (400 MHz, CDCl₃) δ 7.89 (d, *J* = 8.5 Hz, 2H), 7.85 (d, *J* = 9.0 Hz, 1H, H7), 7.74 (d, *J* = 8.5 Hz, 2H), 7.48 (d, *J* = 3.6 Hz, 1H, H2), 6.96 (d, *J* = 2.5 Hz, 1H, H4), 6.92 (dd, *J* = 9.0, 2.4 Hz, 1H, H6), 6.59 (dd, *J* = 3.6, 0.8 Hz, 1H, H3), 4.00 (t, *J* = 6.3 Hz, 2H), 2.54 - 2.33 (m, 6H), 2.04 - 1.91 (m, 2H), 1.62 - 1.57 (m, 4H), 1.50 - 1.41 (m, 2H) (signal for "N*H*" and "N*H₂*" were not detected); ¹³C NMR (101 MHz, CDCl₃) δ 166.7, 156.1, 140.8, 137.5, 131.9, 129.4, 127.8 (2CH_{Ar}), 127.1 (2CH_{Ar}), 126.9 (C2), 114.5 (C7), 114.3 (C6), 110.2 (C3), 104.7 (C4), 66.9 (CH₂), 55.9 (CH₂), 54.5 (2CH₂), 26.7 (CH₂), 25.8 (2CH₂), 24.3 (CH₂); HRMS (ESI): Calcd for C₂₃H₂₈N₄O₄S [M + *H*]⁺: 457.1904. Found: 457.1904. Anal. Calcd for C₂₃H₂₈N₄O₄S·H₂O: C, 58.21; H, 6.37; N, 10.99; S, 6.76. Found: C, 58.17; H, 6.33; N, 10.62; S, 6.42.

**4-((5-(3-(4-(Prop-2-yn-1-yl)piperazin-1-yl)propoxy)-1*H*-indol-1-yl)sulfonyl)benzohydrazide (MTP99).** Following the **General procedure F,** starting from methyl 4-((5-(3-(4-(prop-2-yn-1-yl)piperazin-1-yl)propoxy)-1*H*-indol-1-yl)sulfonyl)benzoate (100 mg, 0.20 mmol), after flash chromatography of the residue using DCM/Methanol (5%) as eluent, gave compound **MTP99** (69 mg, 70%) as a orange solid: mp 150-2 °C; IR (cm⁻¹) v 3303 (N-H), 1672 (C=O), 1376 (O=S=O), 1155 (C-O-C); ¹H NMR (400 MHz, CDCl₃) δ 8.05 (s, 1H, N*H*), 7.85 - 7.82 (m, 3H), 7.72 (d, *J* = 8.7 Hz, 2H), 7.46 (d, *J* = 3.7 Hz, 1H, H2), 6.95 (d, *J* = 2.5 Hz, 1H, H4), 6.90 (dd, *J* = 9.0, 2.5 Hz, 1H, H6), 6.58 (d, *J* = 3.7 Hz, 1H, H3), 3.98 (t, *J* = 6.3 Hz, 2H), 3.27 (d, *J* = 2.5 Hz, 2H), 2.66 - 2.44 (m, 10H), 2.25 (t, *J* = 2.5 Hz, 1H), 2.08 - 1.79 (m, 2H); (signal for "N*H₂*" were not detected) ¹³C NMR (101 MHz, CDCl₃) δ 166.6, 155.9, 140.5, 137.4, 131.8, 129.3, 127.8 (2CH_{Ar}), 126.9 (2CH_{Ar}), 126.9 (CH_{Ar}), 114.4 (C7), 114.3 (C6), 110.1 (C3), 104.7 (C4), 78.6, 73.3 (CH), 66.6 (CH₂), 55.0 (2CH₂), 52.9 (2CH₂), 51.6 (CH₂), 46.7 (CH₂), 26.5 (CH₂); HRMS (ESI): Calcd for C₂₅H₂₉N₅O₄S [M + *H*]⁺: 496.2013. Found: 496.2012. Anal. Calcd for C₂₅H₂₉N₅O₄S·2H₂O: C, 56.48; H, 5.99; N, 12.99; S, 5.90. Found: C, 56.41; H, 5.95; N, 12.72; S, 5.80.

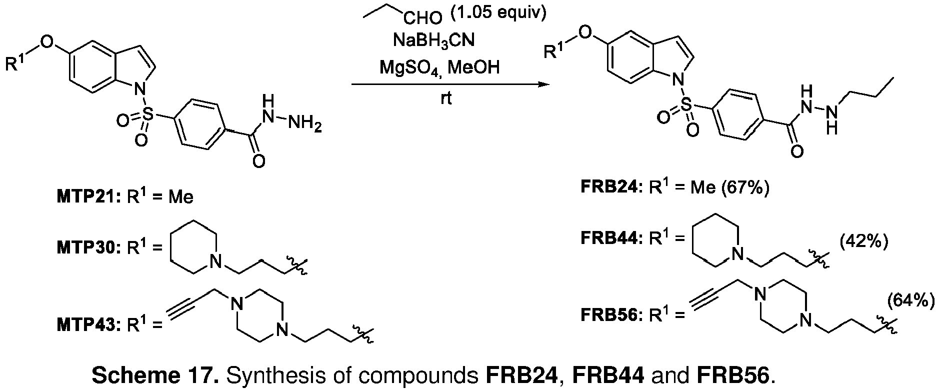

**4-((5-Methoxy-1*H*-indol-1-yl)sulfonyl)-*N'*-propylbenzohydrazide (FRB24).** Following the **General procedure G,** starting from 4-((5-methoxy-1*H*-indol-1-yl)sulfonyl)benzohydrazide (100 mg, 0.29 mmol), after flash chromatography of the residue using DCM/MeOH (0.5%) as eluent, gave compound **FRB24** (76 mg, 68%) as a 46hite solid: mp 103-5 ºC; IR (cm⁻¹) v 3318 (N-H), 1651 (C=O), 1373 (O=S=O), 1139 C-O-C); ¹H NMR (400 MHz, CDCl₃) δ 7.88 (t, *J* = 8.6 Hz, 3H), 7.75 (d, *J* = 8.6 Hz, 1H), 7.49 (d, *J* = 3.6 Hz, 1H), 6.97 (d, *J* = 2.5 Hz, 1H), 6.93 (dd, *J* = 9.0, 2.5 Hz, 1H), 6.62 (d, *J* = 3.7, 1H), 3.81 (s, 3H), 2.86 (t, *J* = 7.3 Hz, 2H), 1.57 - 1.48 (m, 2H), 0.94 (t, *J* = 7.4 Hz, 3H); ¹³C NMR (101 MHz, CDCl₃) δ 166.3, 156.7, 140.5, 137.5, 131.9, 129.4, 127.8 (2CH_{Ar}), 127.0 (2CH_{Ar}), 126.9 (CH_{Ar}), 114.3 (C7), 114.0 (C6), 110.2 (C3), 103.8 (C4), 55.6 (CH₃), 53.9 (CH₂), 21.0 (CH₂), 11.4 (CH₃). HRMS (ESI): Calcd for C₁₉H₂₂N₃O₄S⁺ [M + *H*]⁺: 388.1326. Found: 388.1322.

**4-((5-(3-(Piperidin-1-yl)propoxy)-1*H*-indol-1-yl)sulfonyl)-*N*'-propylbenzohydrazide (FRB44).** Following the **General procedure G,** starting from 4-((5-(3-(piperidin-1-yl)propoxy)-1*H*-indol-1-yl)sulfonyl)benzohydrazide (132 mg, 0.29 mmol), after flash chromatography of the residue using DCM/MeOH/NH₄OH (97:1.7:0.3) as eluent, gave compound **FRB44** (61 mg, 42%)) as a 47hite solid: mp 105-7 °C; IR (cm⁻¹) v 3277 (N-H), 1655 (C=O), 1376 (O=S=O), 1181 (C-O-C); ¹H NMR (400 MHz, CDCl₃) δ 7.91 - 7.80 (m, 3H), 7.73 (d, *J* = 8.0 Hz, 1H), 7.47 (d, *J* = 3.0 Hz, 1H, H2), 6.95 (d, *J* = 2.5 Hz, 1H, H4), 6.92 (dd, *J* = 9.0, 2.4 Hz, 1H, H6), 6.58 (d, *J* = 4.0 Hz, 1H, H3), 3.98 (t, *J* = 6.3 Hz, 2H), 2.83 (t, *J* = 7.3 Hz, 2H) 2.49 - 2.36 (m, 6H), 2.01 - 1.90 (m, 2H), 1.59 - 1.54 (m, 4H), 1.53 - 1.45 (m, 2H), 1.45 - 1.40 (m, 2H), 0.93 (t, *J* = 7.4 Hz, 3H); ¹³C NMR (101 MHz, CDCl₃) δ 165.5, 156.2, 140.8, 137.9, 132.0, 129.5, 127.9 (2CH_{Ar}), 127.2 (2CH_{Ar}), 127.1 (C2), 114.6 (C7), 114.5 (C6), 110.3 (C3), 104.9 (C4), 67.1 (CH₂), 56.1 (CH₂), 54.8 (2CH₂), 54.1 (CH2), 26.9 (CH₂), 26.1 (2CH₂), 24.5 (CH₂), 21.3 (CH₂), 11.6 (CH₃). HRMS (ESI): Calcd for C₂₆H₃₅N₄O₄S⁺ [M + *H*]⁺: 499.2374. Found: 499.2369.

**4-((5-(3-(4-(Prop-2-yn-1-yl)piperazin-1-yl)propoxy)-1*H*-indol-1-yl)sulfonyl)-*N*'-propylbenzohydrazide (FRB56).** Following the **General procedure G**, starting from 4-((5-(3-(4-(prop-2-yn-1-yl)piperazin-1-yl)propoxy)-1*H*-indol-1-yl)sulfonyl)47hite hydrazide (126 mg, 0.25 mmol), after flash chromatography of the residue using DCM/Methanol (4%) as eluent, gave compound **FRB56** (70 mg, 52%) as a 47hite solid: mp 107-9 °C: IR (cm⁻¹) v 3288 (N-H), 1651 (C=O), 1373 (O=S=O), 1153 (C-O-C); ¹H NMR (400 MHz, CDCl₃) δ 7.88 (t, *J* = 8.5 Hz, 2H), 7.85 (s, 1H, H7), 7.75 (d, *J* = 8.5 Hz, 2H), 7.49 (d, *J* = 3.7 Hz, 1H, H2), 6.97 (d, *J* = 2.5 Hz, 1H, H4), 6.93 (dd, *J* = 9.0, 2.5 Hz, 1H, H6), 6.60 (d, *J* = 3.7 Hz, 1H, H3), 4.01 (t, *J* = 6.3 Hz, 2H), 3.30 (d, *J* = 2.5 Hz, 2H), 2.86 (t, *J* = 7.3 Hz, 2H), 2.66 - 2.50 (m, 10H), 2.25 (t, *J* = 2.5 Hz, 1H), 2.01 - 1.93 (m, 2H), 1.58 - 1.47 (m, 2H), 0.95 (t, *J* = 7.3 Hz, 3H); ¹³C NMR (101 MHz, CDCl₃) δ 166.3, 156.0, 140.7, 137.7, 131.9, 129.4, 127.7 (2CH_{Ar}), 127.0 (2CH_{Ar}), 126.9 (CH_{Ar}), 114.4 (C7), 114.3 (C6), 110.2 (C3), 104.7 (C4), 78.8, 73.2 (CH), 66.7 (CH₂), 55.0 (2CH₂), 53.98 (CH₂), 53.0 (2CH₂), 51.2 (CH₂), 46.8 (CH₂), 26.7 (CH₂), 21.2 (CH₂), 11.5 (CH₃). HRMS (ESI): Calcd for C₂₈H₃₆N₅O₄S⁺ [M + *H*]⁺: 538.2483. Found: 538.2479.

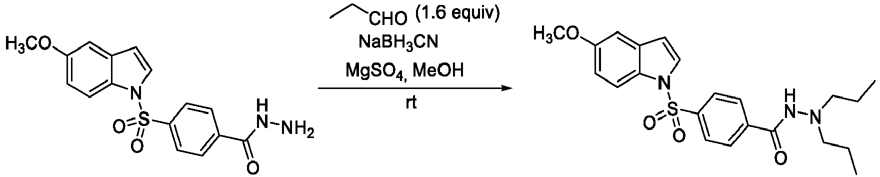

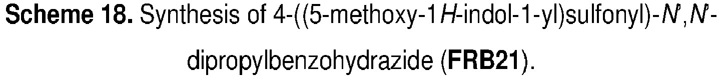

**4-((5-Methoxy-1*H*-indol-1-yl)sulfonyl)-*N*',*N*'-dipropylbenzohydrazide (FRB21).** Following the **General procedure G,** starting from 4-((5-methoxy-1H-indol-1-yl)sulfonyl)benzohydrazide (150 mg, 0.43 mmol), after flash chromatography of the residue using DCM/Methanol (3%) as eluent, gave compound **FRB21** (126 mg, 67%) as a white solid: mp 142-4 ºC; IR (cm⁻¹) v 3228 (N-H), 1650 (C=O), 1367 (O=S=O), 1137 (C-O-C); ¹H NMR (400 MHz, CDCl₃) δ 7.85 (t, *J* = 8.6 Hz, 3H), 7.72 (d, *J* = 8.6 Hz, 1H), 7.48 (d, *J* = 3.6 Hz, 1H), 6.96 (d, *J* = 2.5 Hz, 1H), 6.92 (dd, J = 9.0, 2.5 Hz, 1H), 6.61 (d, *J* = 3.7, 1H), 3.80 (s, 3H), 2.73 (t, *J* = 7.3 Hz, 4H), 1.54 - 1.49 (m, 4H), 0.90 (t, *J* = 7.4 Hz, 6H); ¹³C NMR (101 MHz, CDCl₃) δ 165.0, 156.8, 140.5, 139.1, 132.1, 129.9, 129.7, 128.0 (2CH_{Ar}), 127.2 (2CH_{Ar}), 114.6(C7), 114.2 (C6), 110.4(C3), 104.0 (C4), 60.3 (CH₃), 56.8 (2CH₂), 20.4 (2CH₂), 11.8 (2CH₃). HRMS (ESI): Calcd for C₂₂H₂₈N₃O₄S⁺ [M + *H*]⁺: 430.1795. Found: 430.1791.

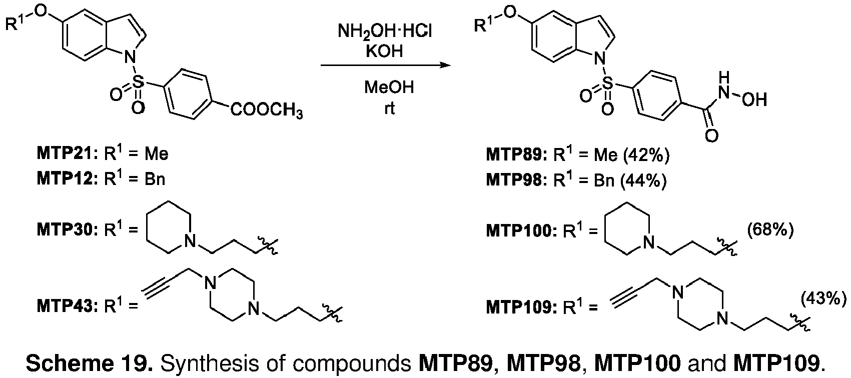

***N*-Hydroxy-4-((5-methoxy-1*H*-indol-1-yl)sulfonyl)benzamide (MTP89).** Following the **General procedure F,** starting from methyl 4-((5-methoxy-1*H*-indol-1-yl)sulfonyl)benzoate (80 mg, 0.23 mmol), after flash chromatography of the residue using DCM/Methanol/NH₄OH (88:10.8:1.2) as eluent, gave compound MTP89 (34 mg, 42%) as a white solid: mp 195-7 °C; IR (cm⁻¹) v 3585 (N-H), 3055 (O-H), 1651 (C=O), 1423 (O=S=O), 1155 (C-O-C); ¹H NMR (500 MHz, DMSO-d₆) δ 8.01 (d, *J* = 8.6 Hz, 2H), 7.85 (d, *J* = 8.6 Hz, 2H), 7.83 (dd, *J* = 9.0, 0.7 Hz, 1H), 7.76 (d, *J* = 3.6 Hz, 1H), 7.11 (d, *J* = 2.6 Hz, 1H), 6.95 (dd, *J* = 9.0, 2.6 Hz, 1H), 6.79 (dd, *J* = 3.7, 0.7 Hz, 1H), 3.74 (s, 3H) (signal for "N*H*" and "O*H*" were not detected); ¹³C NMR (126 MHz, DMSO-d₆) δ 162.4, 156.2, 138.8, 138.2, 131.7, 128.7, 128.2 (C2), 127.7 (2CH_{Ar}), 126.9 (2CH_{Ar}), 114.0 (C7), 113.8 (C6), 110.2 (C3), 104.1 (C4), 55.4 (CH₃); HRMS (ESI): Calcd for C₁₆H₁₅N₂O₅S⁺ [M + *H*]⁺: 347.0696. Found: 347.0688.

**4-((5-(Benzyloxy)-1*H*-indol-1-yl)sulfonyl)-*N*-hydroxybenzamide (MTP98).** Following the **General procedure F,** starting from methyl 4-((5-(benzyloxy)-1*H*-indol-1-yl)sulfonyl)benzoate (150 mg, 0.36 mmol), after flash chromatography of the residue using DCM/Methanol/NH₄OH (88:10.8:1.2) as eluent, gave compound **MTP98** (66 mg, 44%) as a white solid: mp 174-6 °C; IR (cm⁻¹) v 3419 (N-H), 3055 (O-H), 1635 (C=O), 1372 (O=S=O), 1155 (C-O-C); ¹H NMR (400 MHz, DMSO-d₆) δ 11.36 (s, 1H, N*H* or O*H*), 9.24 (s, 1H, N*H* or O*H*), 8.02 (d, *J* = 8.2 Hz, 2H), 7.87 - 7.83 (m, 3H), 7.77 (d, *J=* 3.7 Hz, 1H, H2), 7.46 - 7.41 (m, 2H), 7.41 - 7.35 (m, 2H), 7.35 - 7.29 (m, 1H), 7.20 (d, *J* = 2.5 Hz, 1H, H4), 7.03 (dd, *J* = 9.1, 2.5 Hz, 1H, H6), 6.78 (d, *J* = 3.7 Hz, 1H, H3), 5.08 (s, 2H); ¹³C NMR (101 MHz, DMSO-d₆) δ 162.5, 155.3, 138.8, 138.2, 137.1, 131.6, 128.8, 128.4 (3CH_{Ar}), 128.3 (CH_{Ar}), 127.8 (CH_{Ar}), 127.7 (3CH_{Ar}), 126.9 (2CH_{Ar}), 114.4 (C6), 114.0 (C7), 110.2 (C3), 105.3 (C4), 69.6 (CH₂); HRMS (ESI): Calcd for C₂₂H₁₉N₂O₅S⁺ [M + *H*]⁺: 423.1009. Found: 423.1011.

***N*-Hydroxy-4-((5-(3-(piperidin-1-yl)propoxy)-1*H*-indol-1-yl)sulfonyl)benzamide (MTP100).** Following the **General procedure F,** starting from methyl 4-((5-(3-(piperidin-1-yl)propoxy)-1*H*-indol-1-yl)sulfonyl)benzoate (150 mg, 0.33 mmol), after flash chromatography of the residue using DCM/Methanol/NH₄OH (90:10:1) as eluent, gave compound **MTP100** (103 mg, 68%) as a white solid: mp 135-7 °C; IR (cm⁻¹) v 3505 (N-H), 3059 (O-H), 1713 (C=O), 1363 (O=S=O), 1223 (C-O-C); ¹H NMR (400 MHz, DMSO-d₆) δ 7.99 (d, *J* = 8.5 Hz, 2H), 7.89 (d, *J* = 8.5 Hz, 2H), 7.81 (d, *J* = 9.0 Hz, 1H, H7), 7.75 (d, *J* = 3.6 Hz, 1H, H2), 7.10 (d, *J* = 2.6 Hz, 1H, H4), 6.93 (dd, *J* = 9.0, 2.6 Hz, 1H, H6), 6.77 (d, *J* = 3.6 Hz, 1H, H3), 3.97 (t, *J* = 6.3 Hz, 2H), 2.61 - 2.47 (m, 6H), 1.94 - 1.91 (m, 2H), 1.56 - 1.52 (m, 4H), 1.41 - 1.39 (m, 2H) (signal for "N*H*" and "OH' were not detected); ¹³C NMR (101 MHz, DMSO-d₆) δ 162.2, 155.5, 138.7, 138.3, 131.7, 129.9, 128.7 (CH_{Ar}), 128.3 (CH_{Ar}), 127.7 (C2), 126.8 (2CH_{Ar}), 114.2 (C6), 114.0 (C7), 110.3 (C3), 104.9 (C4), 66.1 (CH₂), 54.7 (CH₂), 53.5 (2CH₂), 25.5 (CH₂), 24.7 (2CH₂), 23.4 (CH₂); HRMS (ESI): Calcd for C₂₃H₂₈N₃O₅S⁺ [M + *H*]⁺: 458.1750. Found: 458.1744.

***N*-Hydroxy-4-((5-(3-(4-(prop-2-yn-1-yl)piperazin-1-yl)propoxy)-1*H*-indol-1-yl) sulfonyl)benzamide (MTP109).** Following the **General procedure F,** starting from methyl 4-((5-(3-(4-(prop-2-yn-1-yl)piperazin-1-yl)propoxy)-1*H*-indol-1-yl)sulfonyl)benzoate (100 mg, 0.20 mmol), after flash chromatography of the residue using DCM/Methanol/NH₄OH (90:10:1) as eluent, gave compound **MTP109** (43 mg, 43%) as a white solid: mp 138-40 °C; IR (cm⁻¹) v 3303 (N-H), 3055 (O-H), 1615 (C=O), 1373 (O=S=O), 1155 (C-O-C); ¹H NMR (300 MHz, DMSO-d₆) δ 8.00 (d, *J* = 8.2 Hz, 2H), 7.85 (d, *J* = 8.2 Hz, 2H), 7.83 (d, *J* = 9.0 Hz, 1H, H7), 7.75 (d, *J* = 3.6 Hz, 1H, H2), 7.11 (d, *J* = 2.5 Hz, 1H, H4), 6.94 (dd, *J* = 9.0, 2.5 Hz, 1H, H6), 6.77 (d, *J* = 3.6 Hz, 1H, H3), 3.97 (t, *J* = 6.3 Hz, 2H), 3.23 (d, *J* = 2.5 Hz, 2H ), 3.12 (d, *J* = 3.0 Hz, 1H), 2.47 - 2.36 (m, 10H), 1.86 - 1.81 (m, 2H) (signal for "N*H*" and "OH' were not detected); ¹³C NMR (101 MHz, DMSO-d₆) δ 132.3, 155.6, 138.8, 138.2, 131.7, 128.6, 128.2 (2CH_{Ar}), 127.6 (C2), 126.8 (2CH_{Ar}), 114.2 (C6), 114.0 (C7), 110.2 (C3), 104.8 (C4), 79.5, 75.6 (CH), 66.2 (CH₂), 54.4 (2CH₂), 52.6 (2CH₂), 51.2 (CH₂), 46.0 (CH₂), 26.3 (CH₂); HRMS (ESI): Calcd for C₂₅H₂₉N₄O₅S⁺ [M + *H*]⁺: 497.1853. Found: 497.1850.

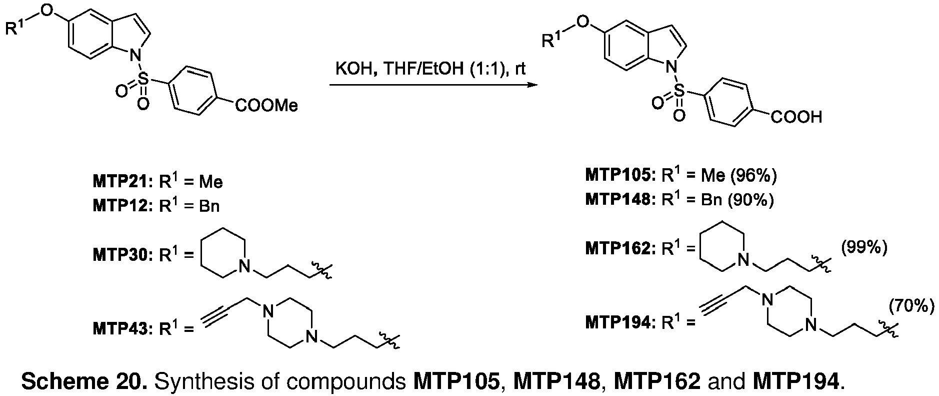

**4-((5-Methoxy-1*H*-indol-1-yl)sulfonyl)benzoic acid (MTP105).** Following the **General procedure C,** starting from methyl 4-((5-methoxy-1*H*-indol-1-yl)sulfonyl)benzoate (500 mg, 1.45 mmol), after flash chromatography of the residue using DCM/Methanol (5%) as eluent, gave compound **MTP105** (460 mg, 96%) as a brown solid: mp >200 °C; IR (cm⁻¹) v 3393 (COO-H), 1698 (C=O), 1374 (O=S=O), 1140 (C-O-C); ¹H NMR (400 MHz, DMSO-d₆) δ 8.05 - 8.04 (m, 4H), 7.81 (d, *J* = 9.0 Hz, 1H, H7), 7.76 (d, *J* = 3.7 Hz, 1H, H2), 7.12 (d, *J =* 2.6, 1H, H4), 6.95 (dd, *J* = 9.0, 2.6 Hz, 1H, H6), 6.80 (d, *J* = 3.7 Hz, 1H, H3), 3.74 (s, 3H) (signal for "CO₂*H*" was not detected); ¹³C NMR (101 MHz, DMSO-d₆) δ 165.7, 156.3, 140.3, 136.0, 131.7, 130.5 (2CH_{Ar}), 128.7, 127.7 (C2), 127.0 (2CH_{Ar}), 114.0 (C7), 113.8 (C6), 110.4 (C3), 104.1 (C4), 55.4 (CH₃); HRMS (ESI): Calcd for C₁₆H₁₄NO₅S [M + *H*]⁺: 332.0587. Found: 332.0588.

**4-((5-(Benzyloxy)-1H-indol-1-yl)sulfonyl)benzoic (MTP148).** Following the **General procedure C,** starting from methyl 4-((5-(benzyloxy)-1*H*-indol-1-yl)sulfonyl)benzoate (770 mg, 1.83 mmol), after flash chromatography of the residue using DCM/Methanol/NH₄OH (95:4.5:0.5) as eluent, gave compound **MTP148** (670 mg, 90%) as a brown solid: mp >200 °C; IR (cm⁻¹) v 3393 (COO-H), 1688 (C=O), 1373 (O=S=O), 1143 (C-O-C); ¹H NMR (400 MHz, DMSO-d₆) δ 8.07 - 7.97 (m, 4H), 7.82 (d, *J* = 9.0 Hz, 1H, H7), 7.76 (d, *J* = 3.7 Hz, 1H, H2), 7.46 - 7.28 (m, 5H), 7.20 (d, *J* = 2.5, 1H, H4), 7.03 (dd, *J* = 9.0, 2.5 Hz, 1H, H6), 6.79 (dd, *J* = 3.7, 0.8 Hz, 1H, H3), 5.08 (s, 2H) (signal for "CO₂*H*" was not detected); ¹³C NMR (101 MHz, DMSO-d₆) δ 166.0, 155.3, 139.7, 137.1, 131.6, 130.4 (2CH_{Ar}), 128.8, 128.4 (2CH_{Ar}), 127.8 (C2), 127.7 (CH_{Ar}), 127.7 (2CH_{Ar}), 126.8 (2CH_{Ar}), 114.4(C6), 114.0 (C7), 110.2 (C3), 105.3 (C4), 69.6 (CH₂); HRMS (ESI): Calcd for C₂₂H₁₇NNaO₅S⁺ [M + *H*]⁺: 430.0720. Found: 430.0719.

**4-((5-(3-(Piperidin-1-yl)propoxy)-1*H*-indol-1-yl)sulfonyl)benzoic acid (MTP162).** Following the **General procedure C,** starting from methyl 4-((5-(3-(piperidin-1-yl)propoxy)-1H-indol-1-yl)sulfonyl)benzoate (250 mg, 0.50 mmol), after flash chromatography of the residue using DCM/Methanol/NH₄OH (80:18:2) as eluent, gave compound **MTP162** (220 mg, 99%) as a white solid: mp 185-90 ºC; IR (cm⁻¹) v 3080 (COO-H), 1608 (C=O), 1365 (O=S=O), 1179 (C-O-C); ¹H NMR (400 MHz, DMSO-d₆) δ 7.99 (d, *J* = 8.6 Hz, 2H), 7.93 (d, *J* = 8.6 Hz, 2H), 7.79 (d, *J* = 9.0 Hz, 1H, H7), 7.74 (d, *J* = 3.7 Hz, 1H, H2), 7.08 (d, *J* = 2.5, 1H, H4), 6.92 (dd, *J* = 9.0, 2.5 Hz, 1H, H6), 6.74 (d, *J* = 3.7 Hz, 1H, H3), 3.97 (t, *J* = 6.3 Hz, 2H), 2.77 - 2.62 (m, 6H), 2.00 - 1.93 (m, 2H), 1.62 - 1.43 (m, 4H), 1.43 (s, 2H) (signal for "CO₂*H*" was not detected); ¹³C NMR (101 MHz, DMSO-d₆) δ 166.4, 155.4, 131.6, 130.0(2CH_{Ar}), 129.0, 128.7, 127.7 (C2), 126.4 (2CH_{Ar}), 125.7, 114.1 (C6), 114.0 (C7), 110.0 (C3), 104.8 (C4), 66.0 (CH₂), 54.5 (CH₂), 53.4 (2CH₂), 25.3 (CH₂), 24.5 (2CH₂), 23.2 (CH₂); HRMS (ESI): Calcd for C₂₃H₂₇N₂O₅S⁺ [M + *H*]⁺: 443.1635. Found: 443.1634.

**4-((5-(3-(4-(Prop-2-yn-1-yl)piperazin-1-yl)propoxy)-1*H*-indol-1-yl)sulfonyl)benzoic acid (MTP194).** Following the **General procedure C,** starting from methyl 4-((5-(3-(4-(prop-2-yn-1-yl)piperazin-1-yl)propoxy)-1*H*-indol-1-yl)sulfonyl)benzoate (480 mg, 0.97 mmol), after flash chromatography of the residue using DCM/Methanol/NH₄OH (80:18:2) as eluent, gave compound **MTP194** (328 mg, 70%) as a white solid: mp 181-3 °C; IR (cm⁻¹) v 3294 (COO-H), 1611 (C=O), 1367 (O=S=O), 1155 (C-O-C); ¹H NMR (400 MHz, DMSO-d₆) δ 8.03 (d, *J* = 8.6 Hz, 2H), 7.99 (d, *J* = 8.6 Hz, 2H), 7.80 (d, *J* = 9.0 Hz, 1H, H7), 7.75 (d, *J* = 3.6 Hz, 1H, H2), 7.10 (d, *J* = 2.5, 1H, H4), 6.93 (dd, *J* = 9.0, 2.5 Hz, 1H, H6), 6.77 (d, *J* = 3.6 Hz, 1H, H3), 3.97 (t, *J* = 6.3 Hz, 2H), 3.24 (d, *J* = 2.5 Hz, 2H), 3.14 (t, *J* = 2.5 Hz, ¹H ), 2.52 - 2.41 (m, 10H), 1.90 - 1.84 (m, 2H) (signal for "CO₂*H*" was not detected); ¹³C NMR (101 MHz, DMSO-d₆) δ 166.0, 155.6, 139.6, 137.8, 131.7, 130.3 (2CH_{Ar}), 128.7, 127.7 (C2), 126.8 (2CH_{Ar}), 114.2 (C6), 114.0 (C7), 110.3 (C3), 104.8 (C4), 79.3, 75.8 (CH), 66.1 (CH₂), 54.2 (CH₂), 52.4 (2CH₂), 50.7 (2CH₂), 45.9 (CH₂), 25.9 (CH₂); HRMS (ESI): Calcd for C₂₅H₂₈N₃O₅S⁺ [M + *H*]⁺: 482.1744. Found: 482.1742.

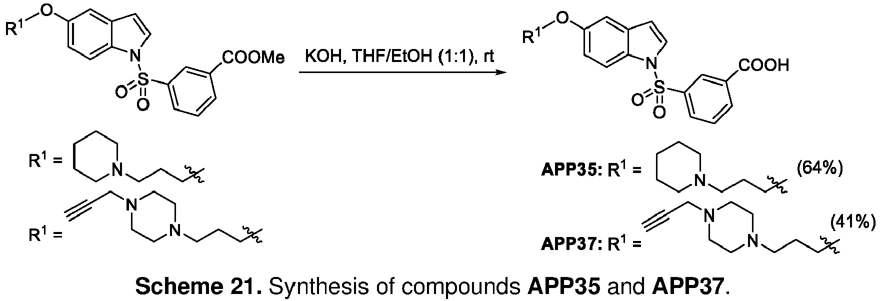

**3-((5-(3-(Piperidin-1-yl)propoxy)-1*H*-indol-1-yl)sulfonyl)benzoic acid (APP35).** Following the **General procedure C,** starting from methyl methyl 3-((5-(3-(piperidin-1-yl)propoxy)-1*H*-indol-1-yl)sulfonyl)benzoate (717 mg, 1.57 mmol), after flash chromatography of the residue using DCM/Methanol/NH₄OH (80:18:2) as eluent, gave compound **APP35** (441 mg, 64%) as a brown solid: mp 105-7 °C; IR (cm⁻¹) v 2952 (COO-H), 1610 (C=O), 1371 (O=S=O), 1139 (C-O-C); ¹H NMR (400 MHz, DMSO-d₆) δ 8.23 (d, *J* = 1.9 Hz, 1H), 8.07 (d, *J* = 7.6 Hz, 1H), 7.95 (dd, *J* = 7.8, 1.6 Hz, 1H), 7.79 (d, *J* = 9.1 Hz, 1H, H7), 7.70 (d, *J* = 3.7 Hz, 1H, H2), 7.53 (t, *J* = 7.8 Hz, ¹H ), 7.06 (d, *J*= 2.5, 1H, H4), 6.94 (dd, *J* = 9.0, 2.5 Hz, 1H, H6), 6.68 (d, *J* = 3.7 Hz, 1H, H3), 3.98 (t, *J=* 6.2 Hz, 2H), 2.81 - 2.69 (m, 6H), 2.00 - 1.92 (m, 2H), 1.65 - 1.59 (m, 4H), 1.46 - 1.42 (m, 2H) (signal for "CO₂*H*" was not detected); ¹³C NMR (101 MHz, DMSO-d₆) δ 166.5, 155.2, 139.1, 136.7, 134.7 (CH_{Ar}), 131.7, 129.4 (CH_{Ar}), 129.0, 127.9 (C2), 127.8 (CH_{Ar}), 126.6 (CH_{Ar}), 114.4 (C6), 114.0 (C7), 110.2 (C3), 105.2 (C4), 65.9 (CH₂), 54.1 (CH₂), 52.9 (2CH₂), 24.6 (CH₂), 23.9 (2CH₂), 22.7 (CH₂); HRMS (ESI): Calcd for C₂₃H₂₇N₂O₅S⁺ [M + *H*]⁺: 443.1635. Found: 443.1612.

**3-((5-(3-(4-(Prop-2-yn-1-yl)piperazin-1-yl)propoxy)-1*H*-indol-1-yl)sulfonyl)benzoic acid (APP37).** Following the **General procedure C,** starting from methyl 3-((5-(3-(4-(prop-2-yn-1-yl)piperazin-1-yl)propoxy)-1*H*-indol-1-yl)sulfonyl)benzoate (667 mg, 1.35 mmol), after flash chromatography of the residue using DCM/Methanol/NH₄OH (80:18:2) as eluent, gave compound **APP37** (268 mg, 41%) as a brown solid: mp 95-7 °C; IR (cm⁻¹) v 3286 (COO-H), 1608 (C=O), 1366 (O=S=O), 1135 (C-O-C); ¹H NMR (500 MHz, DMSO-d₆) δ 8.29 (d, *J* = 1.8 Hz, 1H), 8.15 (d, *J* = 7.7 Hz, 1H), 8.10 (d, *J* = 8.0 Hz, 1H), 7.80 (d, *J* = 9.0 Hz, 1H), 7.76 (d, *J* = 3.7 Hz, 1H, H7), 7.66 (t, *J* = 8.1 Hz, 1H, H2), 7.11 (d, *J* = 2.5 Hz, 1H, H4), 6.95 (dd, *J* = 9.0, 2.5 Hz, 1H, H6), 6.77 (d, *J* = 3.6 Hz, 1H, H3), 3.97 (t, *J =* 6.3 Hz, 2H), 3.24 (d, *J* = 2.5 Hz, 2H), 3.13 (t, *J* = 2.4 Hz, 1H), 2.51 - 2.41 (m, 10H), 1.89 - 1.84 (m, 2H). (signal for "CO₂*H*" was not detected); ¹³C NMR (126 MHz, DMSO-d₆) δ 165.7, 155.6, 137.3, 134.9 (CH_{Ar}), 131.8 (CH_{Ar}), 130.3, 129.7, 129.7, 128.8 (CH_{Ar}), 127.8 (C2), 126.7 (CH_{Ar}), 114.4 (C6), 114.0 (C7), 110.4 (C3), 105.0 (C4), 79.4, 75.8 (CH), 66.1 (CH₂), 54.2 (CH₂), 52.4 (2CH₂), 50.8 (2CH₂), 45.9 (CH₂), 25.9 (CH₂); HRMS (ESI): Calcd for C₂₅H₂₈N₃O₅S⁺ [M + *H*]⁺: 482.1744. Found: 482.1742.

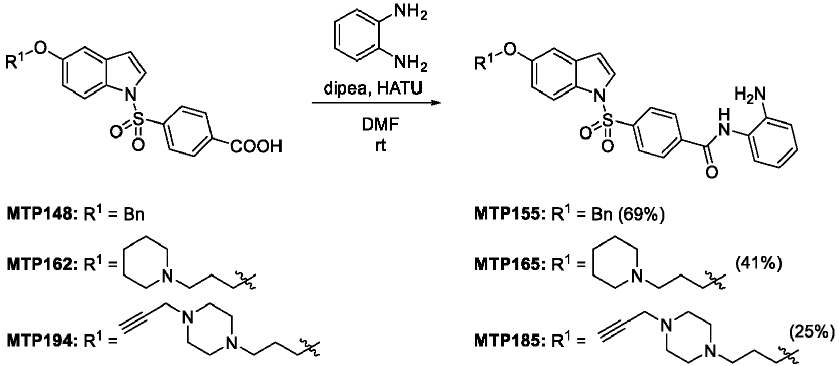

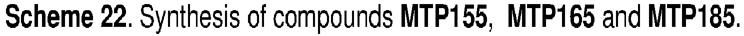

***N*-(2-Aminophenyl)-4-((5-(benzyloxy)-1*H*-indol-1-yl)sulfonyl)benzamide (MTP155).** Following the **General procedure D,** starting from 4-((5-(benzyloxy)-1*H-*indol-1-yl)sulfonyl)benzoic (100 mg, 0.25 mmol), after flash chromatography of the residue using DCM/Methanol (0.5%) as eluent, gave compound **MTP155** (84 mg, 69%) as a yellow solid: mp 85-7 °C; IR (cm⁻¹) v 3364 (N-H), 1607 (C=O), 1371 (O=S=O), 1142 (C-O-C); ¹H NMR (400 MHz, DMSO-d₆) *δ* 9.76 (s, 1H, N*H*), 8.08 - 8.06 (m, 3H), 7.96 (d, *J* = 8.3 Hz, 1H), 7.90 - 7.86 (m, 1H, H7), 7.77 (d, *J* = 3.7 Hz, 1H, H2), 7.44 - 7.42 (m, 2H), 7.39 - 7.36 (m, 2H), 7.35 - 7.28 (m, 1H), 7.20 (dd, *J* = 6.1, 2.5 Hz, 1H, H4), 7.09 (dd, *J* = 7.9, 1.5 Hz, 1H), 7.04 (dd, *J* = 9.0, 2.5 Hz, 1H, H6), 6.95 (td, *J* = 7.6, 1.6 Hz, 1H), 6.79 (d, *J* = 3.7 Hz, 1H, H3), 6.73 (dd, *J* = 8.0, 1.5 Hz, 1H), 6.55 (td, *J* = 7.5, 1.5 Hz, 1H), 5.09 (s, 2H), 5.08 (s, 2H, N*H*₂).; ¹³C NMR (101 MHz, DMSO-d₆) δ 163.9, 155.3, 143.3, 140.1, 138.8, 137.1, 131.7, 129.9 (CH_{Ar}), 129.1 (CH_{Ar}), 128.8, 128.4 (3CH_{Ar}), 127.8 (C2), 127.7 (3CH_{Ar}), 126.8 (CH_{Ar}), 126.6 (CH_{Ar}), 126.2 (CH_{Ar}), 122.4, 115.9 (CH_{Ar}), 115.8 (CH_{Ar}), 114.4 (C6), 114.1 (C7), 110.2 (C3), 105.3 (C4), 69.6 (CH₂); HRMS (ESI): Calcd for C₂₈H₂₄N₃O₄S⁺ [M + *H*]⁺: 498.1482. Found: 498.1485.

***N*-(2-Aminophenyl)-4-((5-(3-(piperidin-1-yl)propoxy)-1*H*-indol-1-yl)sulfonyl)benzamide (MTP165).** Following the **General procedure D,** starting from 4-((5-(3-(piperidin-1-yl)propoxy)-1*H*-indol-1-yl)sulfonyl)benzoic acid (200 mg, 0.45 mmol), after flash chromatography of the residue using DCM/Methanol (4%) as eluent, gave compound **MTP165** (98 mg, 41%) as a yellow solid: mp 80-2 °C; IR (cm⁻¹) v 3233 (N-H), 1622 (C=O), 1370 (O=S=O), 1141 (C-O-C); ¹H NMR (400 MHz, CDCl₃) 8.11 (s, 1H, N*H*), 7.94 - 7.75 (m, 5H), 7.50 (d, *J* = 3.6 Hz, 1H, H2), 7.29 - 7.28 (m, 1H), 7.07 (td, *J* = 7.6, 1.5 Hz, 1H), 6.97 (d, *J* = 2.4 Hz, 1H, H4), 6.93 (dd, *J* = 9.0, 2.4 Hz, 1H, H6), 6.80 - 6.79 (m, 2H), 6.61 (dd, *J* = 3.7, 0.8 Hz, 1H, H3), 4.01 (t, *J* = 6.2 Hz, 2H), 3.76 (s, 2H, N*H*₂), 2.66 - 2.45 (m, 6H), 2.10 - 1.97 (m, 2H), 1.69 - 1.65 (m, 4H), 1.51 - 1.44 (m, 2H).; ¹³C NMR (126MHz, CDCl₃) δ 163.9, 155.9, 140.6, 140.4, 139.0, 131.9, 129.5, 128.2 (2CH_{Ar}), 127.5 (CH_{Ar}), 127.0 (2CH_{Ar}), 127.0 (C2), 125.1, 124.1, 119.9 (CH_{Ar}), 118.5 (CH_{Ar}), 114.4 (C6), 114.4 (C7), 110.2 (C3), 104.8 (C4), 66.6 (CH₂), 55.8 (CH₂), 54.4 (2CH₂), 26.1 (CH₂), 25.2 (2CH₂), 23.9 (CH₂); HRMS (ESI): Calcd for C₂₉H₃₃N₄O₄S⁺ [M + *H*]⁺: 533.2217. Found: 533.2210. ***N*-(2-Aminophenyl)-4-((5-(3-(4-(prop-2-yn-1-yl)piperazin-1-yl)propoxy)-1H-indol-1-yl)sulfonyl)benzamide (MTP185).** Following the **General procedure D,** starting from 4-((5-(3-(4-(prop-2-yn-1-yl)piperazin-1-yl)propoxy)-1*H*-indol-1-yl)sulfonyl) benzoic acid (280 mg, 0.58 mmol), after flash chromatography of the residue using DCM/Methanol (2%) as eluent, gave compound **MTP185** (65 mg, 25%) as a yellow solid: mp 77-9 °C; IR (cm⁻¹) v 3287 (N-H), 1621 (C=O), 1371 (O=S=O), 1140 (C-O-C); ¹H NMR (500 MHz, CDCl₃) δ 7.97 - 7.84 (m, 5H), 7.51 (d, *J* = 3.7 Hz, 1H, H2), 7.31 (d, *J* = 7.8 Hz, 1H), 7.09 (td, *J* = 7.6, 1.5 Hz, 1H), 6.98 (d, *J* = 2.4 Hz, 1H, H4), 6.94 (dd, *J* = 9.0, 2.5 Hz, 1H, H6), 6.87 - 6.76 (m, 2H), 6.62 (dd, *J* = 3.7, 0.8 Hz, 1H, H3), 4.02 (t, *J=* 6.3 Hz, 2H), 3.30 (d, *J* = 2.4 Hz, 2H), 2.70 - 2.40 (m, 10H), 2.25 (t, *J* = 2.4 Hz, 1H), 2.03 - 1.92 (m, 2H);. ¹³C NMR (126 MHz, CDCl₃) δ 163.8, 156.1, 140.7, 140.2, 139.0, 131.9, 129.4 (CH_{Ar}), 128.1 (2CH_{Ar}), 127.5, 127.1 (2CH_{Ar}), 127.0 (C2), 124.9 (CH_{Ar}), 124.2, 120.1 (CH_{Ar}), 118.7 (CH_{Ar}), 114.5 (C6), 114.4 (C7), 110.2 (C3), 104.8 (C4), 78.8, 73.2 (CH), 66.7 (CH₂), 55.1 (CH₂), 53.0 (2CH₂), 51.8 (2CH₂), 46.8 (CH₂), 26.7 (CH₂); HRMS (ESI): Calcd for C₃₁H₃₄NₛO₄S⁺ [M + *H*]⁺: 572.2326. Found: 572.2320.

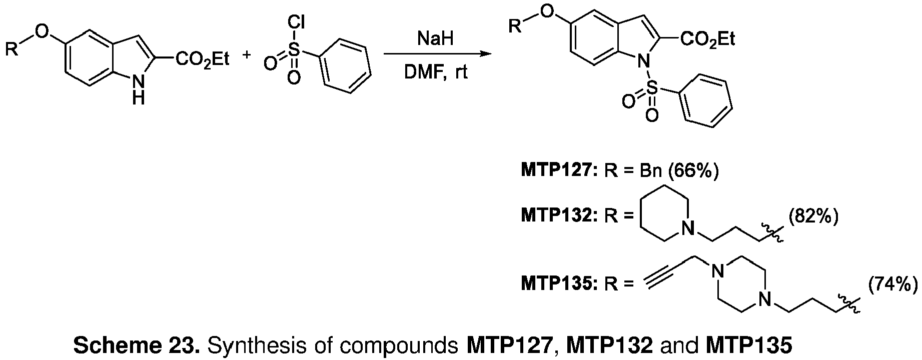

**Ethyl 5-(benzyloxy)-1-(phenylsulfonyl)-1*H*-indole-2-carboxylate (MTP127).** Following the **General Method A,** starting from ethyl 5-(benzyloxy)-1*H*-indole-2-carboxylate (700 mg, 2.37 mmol), after flash chromatography of the residue using hexane/AcOEt (10%) as eluent gave compound **MTP127** (680 mg, 66%) was obtained as a yellow solid: mp 101-3 °C; IR (cm⁻¹) v 1726 (C=O), 1368 (O=S=O), 1205 (C-O-C); ¹H NMR (400 MHz, CDCl₃) δ 8.02 (d, *J* = 9.2 Hz, 1H, H7), 8.00 - 7.96 (m, 1H), 7.61 - 7.29 (m, 9H), 7.13 (dd, *J* = 9.2, 2.5 Hz, 1H, H6), 7.09 (d, *J* = 0.8 Hz, 1H, H3), 7.04 (d, *J* = 2.5 Hz, 1H, H4), 5.08 (s, 2H), 4.40 (q, *J* = 7.2 Hz, 2H), 1.39 (t, *J* = 7.2 Hz, 3H); ¹³C NMR (101 MHz, CDCl₃) δ 161.2, 155.9, 138.5, 136.7, 133.7 (CH_{Ar}), 133.1, 132.5, 129.2, 128.9 (2CH_{Ar}), 128.6 (2CH_{Ar}), 128.1 (CH_{Ar}), 127.5 (2CH_{Ar}), 127.2 (2CH_{Ar}), 117.2 (C6), 117.0 (C3), 116.4 (C7), 105.4 (C4), 70.5 (CH₂), 61.9 (CH₂), 14.1 (CH₃). HRMS (ESI): Calcd for C₂₄H₂₂NO₅S⁺ [*M* + *H*]⁺: 436.1213. Found: 436.1208.

**Ethyl 1-(phenylsulfonyl)-5-(3-(piperidin-1-yl)propoxy)-1*H*-indole-2-carboxylate (MTP132).** Following the **General Method A,** starting from ethyl 5-(3-(piperidin-1-yl)propoxy)-1*H*-indole-2-carboxylate (380 mg, 1.15 mmol), after flash chromatography of the residue using DCM/Methanol (2%) as eluent gave compound **MTP132** (450 mg, 82%) was obtained as a yellow oil: IR (cm⁻¹) v 1726 (C=O), 1368 (O=S=O), 1209 (CO-C); ¹H NMR (400 MHz, CDCl₃) δ 8.00 (d, *J* = 9.2 Hz, 1H, H7), 7.98 - 7.94 (m, 2H), 7.60 - 7.52 (m, 1H), 7.49 - 7.44 (m, 2H), 7.09 (d, *J* = 0.8 Hz, 1H, H3), 7.02 (dd, *J* = 9.2, 2.5 Hz, 1H, H6), 6.95 (d, *J* = 2.5 Hz, 1H, H4), 4.40 (q, *J* = 7.2 Hz, 2H), 4.03 (t, *J* = 6.1 Hz, 2H), 2.79 - 2.70 (m, 6H), 2.19 - 2.15 (m, 2H), 1.82 - 1.76 (m, 4H), 1.56 - 1.54 (m, 2H), 1.39 (t, *J* = 7.2 Hz, 3H); ¹³C NMR (101 MHz, CDCl₃) δ 161.2, 155.9, 138.3, 133.7 (CH_{Ar}), 133.0, 132.5, 129.2, 128.9 (2CH_{Ar}), 127.2 (2CH_{Ar}), 117.0 (C3), 116.9 (C6), 116.4 (C7), 104.8 (C4), 66.3 (CH₂), 61.9 (CH₂), 55.7 (CH₂), 54.2 (2CH₂), 25.6 (CH₂), 24.6 (2CH₂), 23.5 (CH₂), 14.1 (CH₃). HRMS (ESI): Calcd for C₂₅H₃₁N₂O₅S⁺ [*M* + H]⁺: 471.1948. Found: 471.1949.

**Ethyl 1-(phenylsulfonyl)-5-(3-(4-(prop-2-yn-1-yl)piperazin-1-yl)propoxy)-1*H-*indole-2-carboxylate (MTP135).** Following the **General Method A,** starting from ethyl 5-(3-(4-(prop-2-yn-1-yl)piperazin-1-yl)propoxy)-1*H*-indole-2-carboxylate (580 mg, 1.57 mmol), after flash chromatography of the residue using DCM/Methanol (2%) as eluent gave compound **MTP135** (590 mg, 74%) was obtained as a yellow solid: mp 71-3 ºC; IR (cm⁻¹) v 1726 (C=O), 1368 (O=S=O), 1209 (C-O-C); ¹H NMR (400 MHz, CDCl₃) δ 8.00 (d, *J* = 9.2 Hz, 1H, H7), 7.98 - 7.93 (m, 2H), 7.59 - 7.52 (m, 1H), 7.50 - 7.40 (m, 2H), 7.08 (s, 1H, H3), 7.04 (dd, *J* = 9.2, 2.5 Hz, 1H, H6), 6.97 (d, *J* = 2.5 Hz, 1H, H4), 4.40 (q, *J* = 7.2 Hz, 2H), 4.02 (t, *J* = 6.3 Hz, 2H), 3.31 (d, *J* = 2.5 Hz, 2H), 2.65 - 2.56 (m, 10H), 2.26 (t, *J* = 2.5 Hz, 1H), 2.01 (p, *J* = 6.7 Hz, 2H), 1.39 (t, *J* = 7.2 Hz, 3H); ¹³C NMR (101 MHz, CDCl₃) δ 161.2, 156.2, 138.4, 133.7 (CH_{Ar}), 132.9, 132.5, 129.2, 128.9 (2CH_{Ar}), 127.2 (2CH_{Ar}), 117.0 (C6), 117.0 (C3), 116.4 (C7), 104.9 (C4), 78.7, 73.3 (CH), 66.6 (CH₂), 61.9 (CH₂), 55.0 (2CH₂), 53.0 (2CH₂), 51.7 (CH₂), 46.8 (CH₂), 26.6 (CH₂), 14.1 (CH₃). HRMS (ESI): Calcd for C₂₇H₃₂N₃O₅S⁺ [*M* + *H*]⁺: 510.2057. Found: 510.2057.

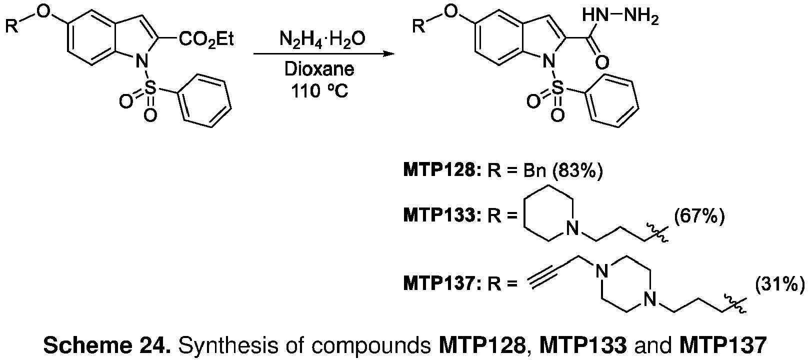

**5-(Benzyloxy)-1-(phenylsulfonyl)-1*H*-indole-2-carbohydrazide (MTP128).** Following the **General Method H,** starting from ethyl 5-(benzyloxy)-1-(phenylsulfonyl)-1*H-*indole-2-carboxylate (100 mg, 0.23 mmol), after flash chromatography of the residue using DCM/Methanol (1%) as eluent gave compound **MTP128** (80 mg, 83%) was obtained as a brown solid: mp 101-3 °C; IR (cm⁻¹) v 3352 (N-H), 1679 (C=O), 1379 (O=S=O), 1169 (C-O-C); ¹H NMR (400 MHz, CDCl₃) δ 7.99 - 7.95 (m, 2H), 7.60 - 7.30 (m, 9H), 7.10 (dd, *J* = 9.1, 2.5 Hz, 1H, H6), 6.99 (d, *J* = 2.5 Hz, 1H, H4), 6.89 (s, 1H, H3), 5.04 (s, 2H) (signal for "NH' and "N*H₂*" were not detected); ¹³C NMR (101 MHz, CDCl₃) δ 162.9, 156.2, 137.0, 136.6, 134.1 (CH_{Ar}), 133.9, 132.1, 129.7, 129.0 (2CH_{Ar}), 128.6 (2CH_{Ar}), 128.1 (CH_{Ar}), 127.5 (2CH_{Ar}), 127.4 (2CH_{Ar}), 116.8 (C6), 116.4 (C7), 115.3 (C3), 105.3 (C4), 70.5 (CH₂). HRMS (ESI): Calcd for C₂₂H₂₀N₃O₄S⁺ [*M* + *H*]⁺: 422.1169. Found: 422.1165.

**1-(Phenylsulfonyl)-5-(3-(piperidin-1-yl)propoxy)-1*H*-indole-2-carbohydrazide (MTP133).** Following the **General Method H,** starting from ethyl 1-(phenylsulfonyl)-5-(3-(piperidin-1-yl)propoxy)-1H-indole-2-carboxylate (100 mg, 0.21 mmol), after flash chromatography of the residue using DCM/Methanol (2%) as eluent gave compound **MTP133** (65 mg, 67%) was obtained as a white solid: mp 114-6 ºC; IR (cm⁻¹) v 3211 (N-H), 1667 (C=O), 1376 (O=S=O), 1169 (C-O-C); ¹H NMR (400 MHz, CDCl₃) δ 7.98 - 7.96 (m, 2H), 7.61 (s, 1H, N*H*), 7.57 - 7.51 (m, 1H), 7.48 - 7.40 (m, 2H), 7.37 - 7.36 (m, 1H), 6.98 (dd, *J* = 9.1, 2.5 Hz, 1H, H6), 6.90 (d, *J* = 2.5 Hz, 1H, H4), 6.89 (d, *J* = 0.8 Hz, 1H, H3), 4.25 - 3.55 (m, 2H, N*H*₂), 4.00 (t, *J* = 6.0 Hz, 2H), 2.93 - 2.65 (m, 6H), 2.19 - 2.08 (m, 2H), 1.81 (s, 4H), 1.60 - 1.50 (m, 2H); ¹³C NMR (101 MHz, CDCl₃) δ 162.8, 156.0, 137.1, 134.1 (CH_{Ar}), 132.0, 129.7, 129.0 (2CH_{Ar}), 127.4 (2CH_{Ar}), 125.9, 116.3 (C7), 116.3 (C6), 115.1 (C3), 104.8 (C4), 66.2 (CH₂), 55.6 (CH₂), 54.1 (2CH₂), 25.4 (CH₂), 24.3 (2CH₂), 23.3 (CH₂). HRMS (ESI): Calcd for C₂₃H₂₉N₄O₄S⁺ [*M* + *H*]⁺: 457.1904. Found: 457.1899.

**1**-**(Phenylsulfonyl)-5-(3-(4-(prop-2-yn-1-yl)piperazin-1-yl)propoxy)-1*H*-indole-2-carbohydrazide (MTP137**). Following the **General Method H,** starting from ethyl 1-(phenylsulfonyl)-5-(3-(4-(prop-2-yn-1-yl)piperazin-1-yl)propoxy)-1*H*-indole-2-carboxylate (150 mg, 0.29 mmol), after flash chromatography of the residue using DCM/Methanol (3%) as eluent gave compound **MTP137** (39 mg, 31%) was obtained as a white solid: mp 122-4 ºC; IR (cm⁻¹) v 3286 (N-H), 1664 (C=O), 1368 (O=S=O), 1169 (C-O-C); ¹H NMR (400 MHz, CDCl₃) δ 7.98 - 7.95 (m, 2H), 7.66 (s, 1H, N*H*), 7.58 - 7.50 (m, 1H), 7.48 - 7.39 (m, 2H), 6.99 (dd, *J* = 9.2, 2.5 Hz, 1H, H6), 6.91 (d, *J* = 2.5 Hz, 1H, H4), 6.89 (d, *J* = 0.8 Hz, 1H, H3), 4.46 - 3.55 (m, 2H, N*H*₂), 4.00 (t, *J* = 6.1 Hz, 2H), 3.32 (d, *J* = 2.5 Hz, 2H), 2.83 - 2.50 (m, 10H), 2.28 (t, *J* = 2.5 Hz, 1H), 2.13 - 1.99 (m, 2H); ¹³C NMR (101 MHz, CDCl₃) δ 162.8, 156.2, 137.1, 134.1 (CH_{Ar}), 131.9, 129.7, 129.0, 129.0 (2CH_{Ar}), 127.4 (2CH_{Ar}), 116.4 (C7), 116.3 (C6), 115.1 (C3), 104.8 (C4), 78.4, 73.6 (CH), 66.3 (CH₂), 55.0 (2CH₂), 52.8 (2CH₂), 50.9 (CH₂), 46.6 (CH₂), 29.7 (CH₂). HRMS (ESI): Calcd for C₂₅H₃₀N₅O₄S⁺ [*M* + *H*]⁺: 496.2013. Found: 496.2016.

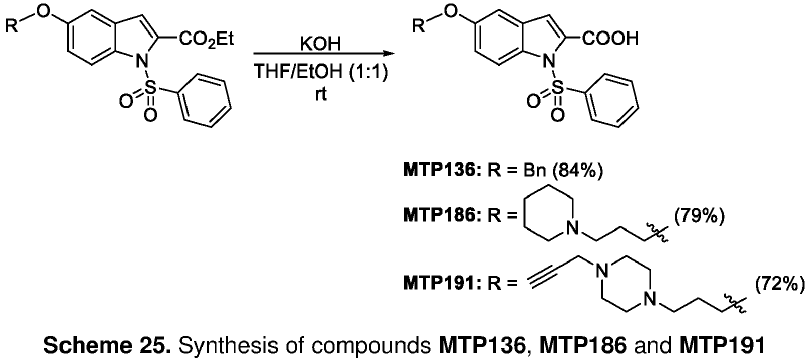

**5-(Benzyloxy)-1-(phenylsulfonyl)-1*H*-indole-2-carboxylic acid (MTP136).** Following the **General Method C**, starting from ethyl 5-(benzyloxy)-1-(phenylsulfonyl)-1*H*-indole-2-carboxylate (200 mg, 0.46 mmol), after flash chromatography of the residue using DCM/Methanol/NH₄OH (90:9:1) as eluent gave compound **MTP136** (157 mg, 84%) was obtained as a white solid: mp 132-4 °C; IR (cm⁻¹) v 2925 (COO-H), 1688 (C=O), 1371 (O=S=O), 1212 (C-O-C); ¹H NMR (300 MHz, DMSO-d₆) δ 8.25 - 8.22 (m, 2H), 7.78 (d, *J* = 9.1 Hz, 1H, H7), 7.62 (d, *J* = 7.3 Hz, 1H), 7.56 - 7.50 (m, 2H), 7.46 - 7.28 (m, 5H), 7.07 (d, *J* = 2.6 Hz, 1H, H4), 6.91 (dd, *J* = 9.1, 2.6 Hz, 1H, H6), 6.45 (s, 1H, H3), 5.06 (s, 2H) (the signal for "COOH' were not detected); ¹³C NMR (101 MHz, DMSO-d₆) δ 163.7, 154.9, 138.7, 137.2, 133.6 (CH_{Ar}), 130.6, 129.7, 128.9 (2CH_{Ar}), 128.4 (2CH_{Ar}), 128.4, 127.8 (CH_{Ar}), 127.7 (2CH_{Ar}), 127.5 (2CH_{Ar}), 115.1 (C7), 113.1 (C6), 106.9 (C3), 104.7 (C4), 69.5 (CH₂). HRMS (ESI): Calcd for C₂₂H₁₈NO₅S⁺ [*M* + *H*]⁺: 408.0900. Found: 408.0904.

**1-(Phenylsulfonyl)-5-(3-(piperidin-1-yl)propoxy)-1H-indole-2-carboxylic acid (MTP186).** Following the **General Method C,** starting from ethyl 1-(phenylsulfonyl)-5-(3-(piperidin-1-yl)propoxy)-1H indole-2-carboxylate (510 mg, 1.08 mmol), after flash chromatography of the residue using DCM/Methanol/NH₄OH (90:9:1) as eluent gave compound **MTP186** (380 mg, 79%) was obtained as a white solid: mp 125-7 °C; IR (cm⁻¹) v 2947 (COO-H), 1600 (C=O), 1361 (O=S=O), 1213 (C-O-C); ¹H NMR (400 MHz, DMSO-d₆) δ 8.04 - 7.86 (m, 3H), 7.76 - 7.65 (m, 1H), 7.65 - 7.51 (m, 2H), 7.31 (s, 1H, COO*H*), 7.25 (s, 1H, H3), 7.17 (d, *J* = 2.6 Hz, 1H, H4), 7.07 (dd, *J* = 9.2, 2.6 Hz, 1H, H6), 4.06 (t, *J* = 5.9 Hz, 2H), 3.50 - 3.47 (m, 2H), 3.30 - 3.18 (m, 2H), 2.99 - 2.81 (m, 2H), 2.13 (dq, *J* = 11.4, 5.9 Hz, 2H), 1.84 - 1.81 (m, 2H), 1.74 - 1.33 (m, 4H); ¹³C NMR (101 MHz, DMSO-d₆) δ 161.9, 155.5, 137.3, 134.6 (CH_{Ar}), 133.7, 131.9, 129.5 (2CH_{Ar}), 129.4, 127.0 (2CH_{Ar}), 116.6 (C6), 116.2 (C3), 116.1 (C7), 105.4 (C4), 65.3 (CH₂), 53.4 (CH₂), 52.5 (2CH₂), 23.5 (CH₂), 22.7 (2CH₂), 21.3 (CH₂) (signal for 158.4 (q, *J* = 38.2 Hz) and 115.2 (q, *J* = 288.7 Hz) correspond to "*C*F₃*C*OOH" added doing to preparation of the sample). HRMS (ESI): Calcd for C₂₃H₂₆N₂O₅S⁺ [*M* + *H*]⁺: 443.1635. Found: 443.1635.

**1-(Phenylsulfonyl)-5-(3-(4-(prop-2-yn-1-yl)piperazin-1-yl)propoxy)-1H-indole-2-carboxylic acid (MTP191**). Following the **General Method C,** starting from ethyl 1-(phenylsulfonyl)-5-(3-(4-(prop-2-yn-1-yl)piperazin-1-yl)propoxy)-1*H*-indole-2-carboxylate (610 mg, 1.30 mmol), after flash chromatography of the residue using DCM/Methanol/NH₄OH (85:13.5:1.5) as eluent gave compound **MTP191** (447 mg, 72%) was obtained as a white solid: mp >230 ºC; IR (cm⁻¹) v 3285 (COO-H), 1609 (C=O), 1354 (O=S=O), 1223 (C-O-C); ¹H NMR (400 MHz, DMSO-d₆) δ 8.00 - 7.88 (m, 3H), 7.73 - 7.65 (m, 1H), 7.62 - 7.58 (m, 2H), 7.25 (s, 1H, H3), 7.17 (d, *J* = 2.5 Hz, 1H, H4), 7.07 (dd, *J* = 9.2, 2.5 Hz, 1H, H6), 4.06 (t, *J* = 5.9 Hz, 2H), 3.72 (d, *J* = 2.5 Hz, 2H), 3.64 - 2.97 (m, 10H), 3.16 (s, 1H), 2.16 - 2.09 (m, 2H) (signal for "COOH' was not detected); ¹³C NMR (101 MHz, DMSO-d₆) δ 161.9, 155.4, 137.3, 134.5 (CH_{Ar}), 133.7, 131.8, 129.5 (2CH_{Ar}), 129.3, 127.0 (2CH_{Ar}), 116.5 (C6), 116.2 (C3), 116.1 (C7), 105.4 (C4), 78.6 (CH), 76.2, 65.1 (CH₂), 53.1 (CH₂), 50.0 (2CH₂), 47.7 (2CH₂), 44.9 (CH₂), 23.5 (CH₂) (signal for 158.4 (q, *J* = 37.6 Hz) and 115.4 (q, *J* = 289.8 Hz) correspond to "*C*F₃*C*OOH" added doing to preparation of the sample). HRMS (ESI): Calcd for C₂₅H₂₈N₃O₅S⁺ [*M* + *H*]⁺: 482.1744. Found: 482.1736

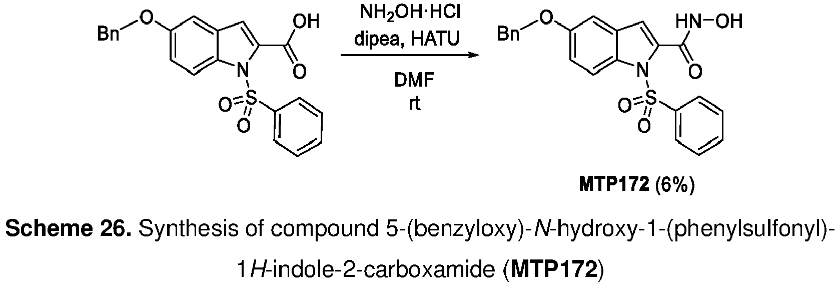

**5-(Benzyloxy)-*N--*hydroxy-1-(phenylsulfonyl)-1*H*-indole-2-carboxamide (MTP172).** Following the **General Method D**, starting from 5-(benzyloxy)-1-(phenylsulfonyl)-1*H*-indole-2-carboxylic acid (150 mg, 0.37 mmol), after flash chromatography of the residue using DCM/Methanol/NH₄OH (90:9:1) as eluent gave compound **MTP172** (10 mg, 6%) was obtained as a white solid: mp 108-10 °C; IR (cm⁻¹) v 3302 (N-H), 3031 (O-H), 1651 (C=O), 1377 (O=S=O), 1161 (C-O-C); ¹H NMR (500 MHz, DMSO-d₆) δ 8.13 - 8.09 (m, 2H), 7.87 (d, *J* = 9.1 Hz, 1H, H7), 7.73 - 7.66 (m, 1H), 7.63 - 7.55 (m, 2H), 7.47 - 7.35 (m, 4H), 7.34 - 7.27 (m, 1H), 7.22 (d, *J* = 2.5 Hz, 1H, H4), 7.08 (dd, *J* = 9.1, 2.5 Hz, 1H, H6), 6.90 (d, *J* = 0.8 Hz, 1H, H3), 5.09 (s, 2H) (signal for "N*H*' and "O*H*' were not detected); ¹³C NMR (126 MHz, DMSO-d₆) δ 158.2, 155.3, 137.3, 137.0, 134.5 (CH_{Ar}), 134.1, 130.3, 129.4 (2CH_{Ar}), 129.3, 128.4 (2CH_{Ar}), 127.9 (CH_{Ar}), 127.7 (2CH_{Ar}), 127.3 (2CH_{Ar}), 115.7 (C6), 115.2 (C7), 112.5 (C3), 105.4 (C4), 69.6 (CH₂). HRMS (ESI): Calcd for C₂₂H₁₉N₂O₅S⁺ [M + *H*]⁺: 423.1009. Found: 423.1016

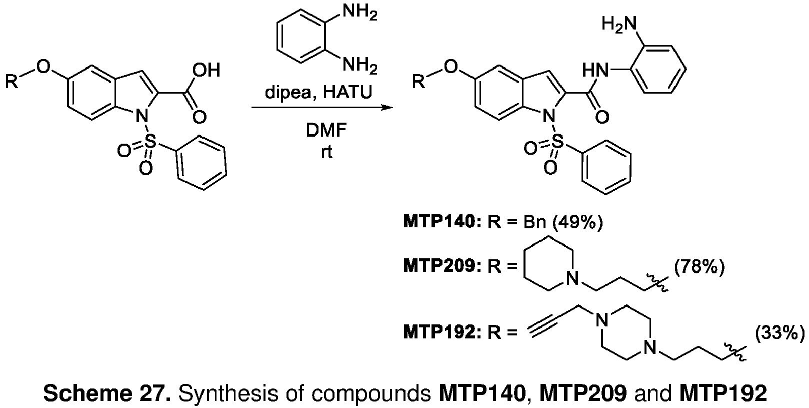

***N-*(2-Aminophenyl)-5-(benzyloxy)-1-(phenylsulfonyl)-1H-indole-2-carboxamide (MTP140).** Following the **General Method D,** starting from 5-(benzyloxy)-1-(phenylsulfonyl)-1H-indole-2-carboxylic acid (170 mg, 0.42 mmol), after flash chromatography of the residue using DCM/Methanol (1%) as eluent gave compound **MTP140** (101 mg, 49%) was obtained as a brown solid: mp 129-31 °C; IR (cm⁻¹) v 3356 (N-H), 1663 (C=O), 1366 (O=S=O), 1151 (C-O-C); ¹H NMR (400 MHz, DMSO-d₆) δ 10.10 (s, 1H, N*H*), 8.10 - 8.08 (m, 2H), 7.90 (d, *J* = 9.1 Hz, 1H, H7), 7.72 - 7.68 (m, 1H), 7.61 - 7.57 (m, 2H), 7.45 - 7.32 (m, 5H), 7.29 - 7.21 (m, 2H), 7.17 (s, 1H, H3), 7.12 - 7.10 (m, 1H, H6), 6.99 (d, *J* = 7.6 Hz, 1H), 6.78 (d, *J* = 8.1 Hz, 1H), 6.61 (t, *J* = 7.6 Hz, 1H), 5.12 (s, 2H), 5.03 (s, 2H, NH₂); ¹³C NMR (101 MHz, DMSO-d₆) δ 159.7, 155.5, 143.1, 137.1, 137.0, 136.5, 134.6 (CH_{Ar}), 130.3, 129.7 (CH_{Ar}), 129.5 (2CH_{Ar}), 128.4 (2CH_{Ar}), 127.9 (CH_{Ar}), 127.7 (2CH_{Ar}), 127.2 (2CH_{Ar}), 126.9, 126.4 (CH_{Ar}), 122.1, 116.0 (CH_{Ar}), 115.8 (CH_{Ar}), 115.7 (C6), 115.5 (C7), 112.6 (C3), 105.6 (C4), 69.6 (CH₂). HRMS (ESI): Calcd for C₂₈H₂₄N₃O₄S⁺ [*M* + *H*]⁺: 498.1482. Found: 498.1482.

***N*-(2-Aminophenyl)-1-(phenylsulfonyl)-5-(3-(piperidin-1-yl)propoxy)-1*H*-indole-2-carboxamide (MTP209).** Following the **General Method D,** starting from 1-(phenylsulfonyl)-5-(3-(piperidin-1-yl)propoxy)-1*H-*indole-2-carboxylic acid (200 mg, 0.45 mmol), after flash chromatography of the residue using DCM/Methanol (2%) as eluent gave compound **MTP209** (189 mg, 78%) was obtained as a brown solid: mp 123-5; IR (cm⁻¹) v 3335 (N-H), 1662 (C=O), 1377 (O=S=O), 1158 (C-O-C); ¹H NMR (500 MHz, CDCl₃) δ 8.03 (d, *J* = 9.1 Hz, 1H, H7), 7.92 - 7.86 (m, 2H), 7.80 (s, 1H, N*H*), 7.55 - 7.48 (m, 1H), 7.46 (dd, *J* = 8.3, 1.5 Hz, 1H), 7.42 - 7.34 (m, 2H), 7.16 - 7.09 (m, 2H), 7.03 (dd, *J* = 9.1, 2.5 Hz, 1H, H6), 6.93 (d, *J* = 2.5 Hz, 1H, H4), 6.89 - 6.82 (m, 2H), 4.17 (s, 2H, N*H*₂), 4.02 (t, *J* = 6.2 Hz, 2H), 2.73 - 2.46 (m, 6H), 2.18 - 2.02 (m, 3H), 1.77 - 1.68 (m, 4H), 1.54 - 1.49 (m, 2H); ¹³C NMR (126 MHz, CDCl₃) δ 160.0, 156.6, 141.5, 136.3, 136.0, 134.2 (CH_{Ar}), 132.3, 130.4, 128.9 (2CH_{Ar}), 127.7 (CH_{Ar}), 127.5 (2CH_{Ar}), 126.1 (CH_{Ar}), 122.9, 119.1 (CH_{Ar}), 117.5 (CH_{Ar}), 117.4 (C3), 117.0 (C7), 116.5 (C6), 105.0 (C4), 66.6 (CH₂), 55.8 (CH₂), 54.4 (2CH₂), 26.0 (CH₂), 25.1 (2CH₂), 23.8 (CH₂). HRMS (ESI): Calcd for C₂₃H₃₃N₄O₄S⁺ [*M* + *H*]⁺: 533.2217. Found: 533.2212.

***N*-(2-Aminophenil)-1-(phenylsulfonyl)-5-(3-(4-(prop-2-yn-1-yl)piperazin-1-yl)propoxy)-1*H*-indole-2-carboxamide (MTP192).** Following the **General Method D,** starting from 1-(phenylsulfonyl)-5-(3-(4-(prop-2-yn-1-yl)piperazin-1-yl)propoxy)-1*H-*indole-2-carboxylic acid (180 mg, 0.37 mmol), after flash chromatography of the residue using DCM/Methanol (4%) as eluent gave compound **MTP192** (70 mg, 33%) was obtained as a yellow solid: mp 70-2 ºC; IR (cm⁻¹) v 3287 (N-H), 1664 (C=O), 1367 (O=S=O), 1153 (C-O-C); ¹H NMR (500 MHz, CDCl₃) δ 8.03 (d, *J* = 9.1 Hz, 1H, H7), 7.93 - 7.83 (m, 2H), 7.76 (s, 1H, N*H*), 7.53 - 7.48 (m, 1H), 7.46 (dd, *J* = 8.5, 1.5 Hz, 1H), 7.38 (dd, *J* = 8.5, 7.6 Hz, 2H), 7.16 - 7.08 (m, 2H), 7.04 (dd, *J* = 9.1, 2.5 Hz, 1H, H6), 6.94 (d, *J* = 2.5 Hz, 1H, H4), 6.88 - 6.78 (m, 2H), 4.02 (t, *J* = 6.3 Hz, 2H), 3.31 (d, *J* = 2.5 Hz, 2H), 2.79 - 2.46 (m, 9H), 2.26 (t, *J* = 2.5 Hz, 1H), 2.06 - 1.93 (m, 2H) (signal for "N*H*₂" was not detected); ¹³C NMR (126 MHz, CDCl₃) δ 160.1, 156.8, 141.5, 136.3, 135.9, 134.2 (CH_{Ar}), 132.3, 130.4, 128.9 (2CH_{Ar}), 127.7 (CH_{Ar}), 127.4 (2CH_{Ar}), 126.1 (CH_{Ar}), 122.9, 119.1 (CH_{Ar}), 117.7 (C3), 117.4 (CH_{Ar}), 117.1 (C7), 116.6 (C6), 105.0 (C4), 78.7, 73.3 (CH), 66.7 (CH₂), 55.1 (CH₂), 53.1 (2CH₂), 51.8 (2CH₂), 46.8 (CH₂), 26.7 (CH₂). HRMS (ESI): Calcd for C₃₁H₃₄N₅O₄S⁺ [*M* + *H*]⁺: 572.2326. Found: 572.2324.

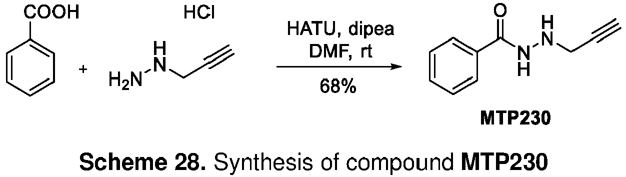

***N*'-(Prop-2-yn-1-yl)benzohydrazide (MTP230).** Following the **General Method for Hydrazides Synthesis,** starting from commercial benzoic acid (300 mg, 2.46 mmol), after flash chromatography of the residue using hexane/AcOEt (6:1) as eluent gave compound **MTP230** (290 mg, 68%) was obtained as a white solid: mp 76-8 °C; IR (cm⁻¹): v 3286 (N-H), 3226 (N-H), 1626 (C=O); ¹H NMR (400 MHz, CDCl₃) δ 8.23 (s, 1H, N*H*), 7.83 - 7.75 (m, 2H), 7.57 - 7.48 (m, 1H), 7.44 (dd, *J* = 8.2, 6.8 Hz, 2H), 5.14 (s, 1H, N*H*), 3.74 (d, *J* = 2.4 Hz, 2H), 2.27 (t, *J* = 2.5 Hz, 1H); ¹³C NMR (101 MHz, CDCl₃) *δ* 167.4, 132.4, 132.0 (CH_{Ar}), 128.6 (2CH_{Ar}), 127.0 (2CH_{Ar}), 79.8, 72.6 (CH), 41.2 (CH₂); HRMS (ESI): Calcd for C₁₀H₁₁N₂O⁺ [*M*+ H]⁺: 175.0866. Found: 175.0860.

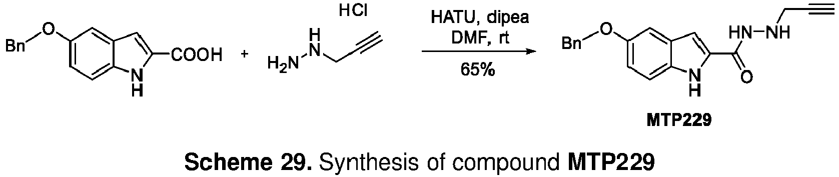

**5-(Benzyloxy)-*N*'-(prop-2-yn-1-yl)-1*H*-indole-2-carbohydrazide (MTP229).** Following the **General Method for Hydrazides Synthesis,** starting from 5-(benzyloxy)-1*H-*indole-2-carboxylic acid (140 mg, 0.52 mmol), after flash chromatography of the residue using hexane/AcOEt (3:1) as eluent gave compound **MTP229** (109 mg, 65%) was obtained as a yellow solid: mp 150-2 °C; IR (cm⁻¹): v 3329 (N-H), 3264 (N-H), 1620 (C=O), 1171 (C-O-C); ¹H NMR (500 MHz, DMSO-d₆) δ 11.48 (d, *J* = 2.2 Hz, 1H, N*H*), 10.05 (d, *J* = 6.0 Hz, 1H, N*H*), 7.50 - 7.44 (m, 2H), 7.42 - 7.35 (m, 2H), 7.35 - 7.28 (m, 2H), 7.16 (d, *J* = 2.4 Hz, 1H, H4), 7.03 (dd, *J* = 2.2, 0.9 Hz, 1H, H3), 6.91 (dd, *J* = 8.9, 2.4 Hz, 1H, H6), 5.44 (q, *J* = 5.4 Hz, 1H, N*H*), 5.09 (s, 2H), 3.64 (dd, *J* = 5.2, 2.5 Hz, 2H), 3.12 (t, *J* = 2.5 Hz, 1H); ¹³C NMR (126 MHz, DMSO-d₆) δ 160.8, 152.8, 137.6, 131.9, 130.4, 128.4 (2CH_{Ar}), 127.7 (3CH_{Ar}), 127.3, 115.1 (C6), 113.1 (C7), 103.5 (C4), 102.3 (C3), 81.3, 74.5 (CH), 69.6 (CH₂), 39.5 (CH₂); HRMS (ESI): Calcd for C₁₉H₁₈N₃O₂⁺ [*M* + H]⁺: 320.1394. Found: 320.1390.

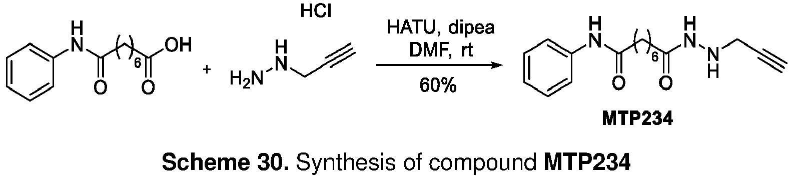

**8-Oxo-*N*-phenyl-8-(2-(prop-2-yn-1-yl)hydrazineyl)octanamide (MTP234).** Following the **General Method for Hydrazides Synthesis,** starting from 8-oxo-8-(phenylamino)octanoic acid (300 mg, 1.20 mmol), after flash chromatography of the residue using hexane/AcOEt (1:1) as eluent gave compound **MTP234** (219 mg, 60%) was obtained as a white solid: mp 130-2 °C; IR (cm⁻¹): v 3326 (N-H), 3295 (N-H), 3282 (N-H), 1642 (C=O); ¹H NMR (500 MHz, DMSO-d₆) δ 9.83 (s, 1H, N*H*), 9.33 (d, *J* = 6.3 Hz, 1H, N*H*), 7.60 - 7.53 (m, 2H), 7.32 - 7.24 (m, 2H), 7.01 (tt, *J* = 7.4, 1.2 Hz, 1H), 5.14 - 5.04 (m, 1H, N*H*), 3.50 - 3.41 (m, 2H), 3.07 (t, *J* = 2.5 Hz, 1H), 2.28 (t, *J* = *7.5* Hz, 2H), 2.02 (t, *J* = 7.4 Hz, 2H), 1.63 - 1.43 (m, 4H), 1.34 - 1.22 (m, 4H); ¹³C NMR (126 MHz, DMSO-d₆) δ 171.4, 171.2, 139.3, 128.6 (2CH_{Ar}), 122.9 (CH_{Ar}), 119.0 (2CH_{Ar}), 81.4, 74.2 (CH), 39.5 (CH₂), 36.4 (CH₂), 33.4 (CH₂), 28.4 (CH₂), 28.4 (CH₂), 25.1 (CH₂), 25.0 (CH₂); HRMS (ESI): Calcd for C₁₇H₂₄N₃O₂⁺ [*M* + H]⁺: 302.1863. Found: 302.1850.

### Biological Results

The biological relevance of the compounds was tested by in vitro experiments in glioblastoma. For this purpose, a total of 16 compounds derived from the different formulas were selected.

As a first screening, dose-response assays were done in U87 glioblastoma cell line in order to obtain the IC50 values of the different compounds. Cells were seeded at a density of 1.500 cells/well in 96-well plates, incubated overnight and treated with increasing concentrations of the corresponding compounds. After 72 h of treatment, cell viability was determined by MTT assay. For this, MTT was added to the wells and cells were incubated for 3,5 h at 37ºC and 5% CO₂. Then, media was removed and DMSO was added to resuspend the formed crystals. Absorbance at 570 nm was measured using a spectrophotometer, and GraphPad Prism software was used to analyze the results and calculate the IC50 values.

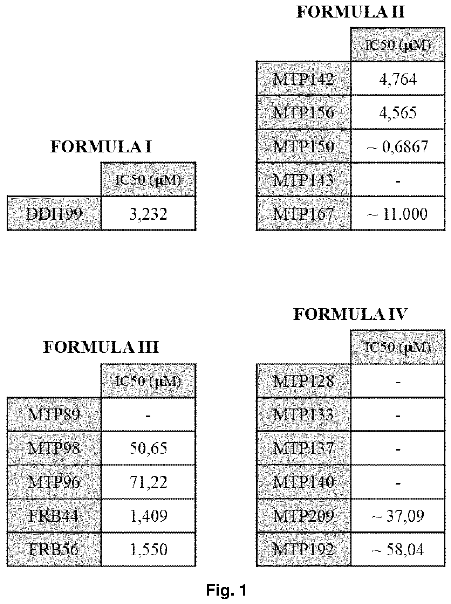

Basing on the IC50 values, DDI199, MTP142, MTP156, MTP150, FRB44 and FRB56 were selected for further studies, as they seem to be the most promising compounds (see figure 1). Thus, the capacity of these compounds as HDAC inhibitors was tested. To do so, U87 cells were treated with different doses of the compounds depending on the IC50 values for 48 h and the levels of HDAC6 and HDAC1, as well as the acetylation of their main targets (α-tubulin and histone H3, respectively), were checked by western blot. For this, specific antibodies against HDAC6 and HDAC1, and acetylated α-tubulin and acetylated histone H3 were used, and β-actin was included as control. Secondary antibodies were horseradish peroxidase (HRP)-linked anti-rabbit and anti-mouse and detection was performed by chemiluminiscence with NOVEX ECL Chemi Substrate.

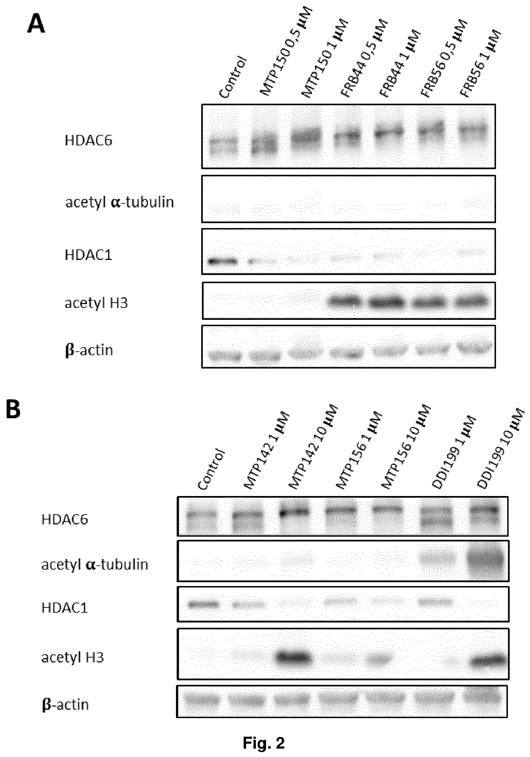

From this analysis, we found that DDI199 shows high HDAC6 inhibitory capacity, being the effect higher with increasing concentrations of the compound. MTP142 also inhibits HDAC6, although this effect is much slighter than that for DDI199. In addition, all the compounds inhibit HDAC1, being the effect more prominent at higher concentrations of the compounds (see Figure 2A and Figure 2B).

Once the inhibitory capacity of the compounds was proved, immunofluorescence assays were performed in order to study their effect in cell proliferation and apoptosis. For this, U87 cells were incubated for 48 h with increasing concentrations of the compounds. After that, cells were incubated with phosphohistone H3 or caspase-3 antibodies for the study of proliferation or apoptosis, respectively. Alexa Fluor 555 IgG anti-mouse and anti-rabbit secondary antibodies were used, and nuclear DNA was stained with DAPI. Pictures were taken with an Eclipse 80i microscope.

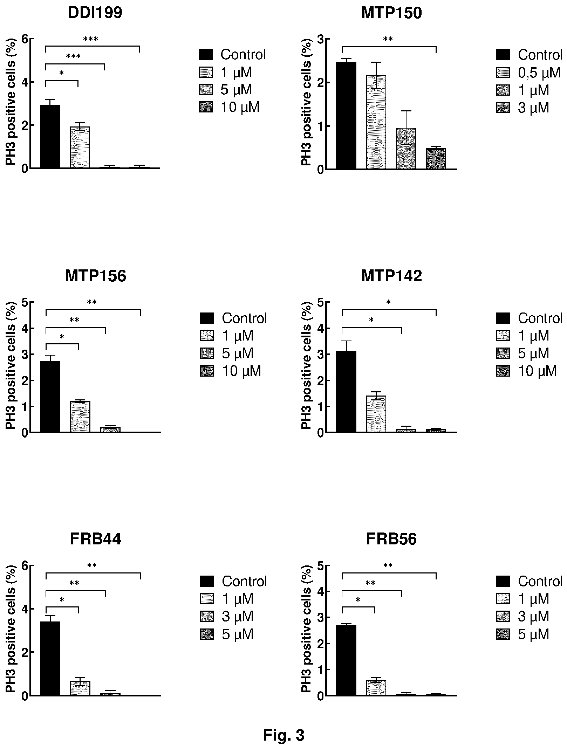

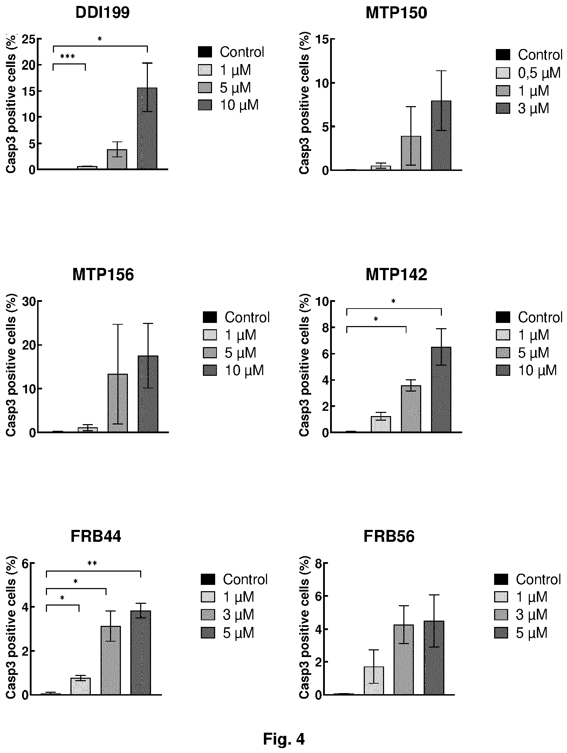

Our results demonstrated that all the tested compounds reduced the proliferating capacity of U87 cells, which is reflected by a reduction of PH3 positive cells. In addition, this effect is dose dependent, as increasing concentrations of the compounds are linked to a smaller proliferation capacity (Figure 3). Moreover, the number of caspase-3 positive cells is increased when the cells are treated with the compounds, indicating the capacity of the compounds to induce apoptosis. This effect is also observed to be dose-dependent (Figure 4).

After the screening of this subset of compounds, and basing on the promising results obtained both by western blot and immunofluorescence assays, we decided to go deeper in the analysis of DDI199.

First, we tested if the effect of the compound in cell viability was exclusive to U87 cell line or if it was repeated in other glioblastoma cell lines. With this aim, additional MTT assays were carried out following the same protocol in other glioblastoma conventional (U251) and patient-derived (GNS179) cell lines. Moreover, in order to compare the potential of our compound with other pan-inhibitors, MTT assays were also carried out for SAHA. Results showed that DDI199 has a higher cytotoxic effect than SAHA (Table 1).

**Table 1. Comparison of IC50 values of DDI199 and pan-inhibitor SAHA in conventional (U87 and U251) and patient-derived (GNS179) cell lines.**

| IC50 (µM) | DDI199 | SAHA |
|---|---|---|
| U87 | 3,232 | 6,761 |
| U251 | 5,454 | 16,03 |
| GNS179 | 4,328 | 5,019 |

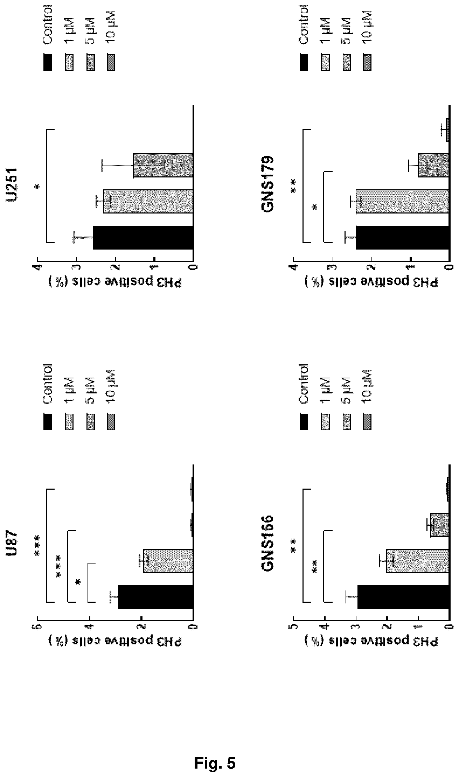

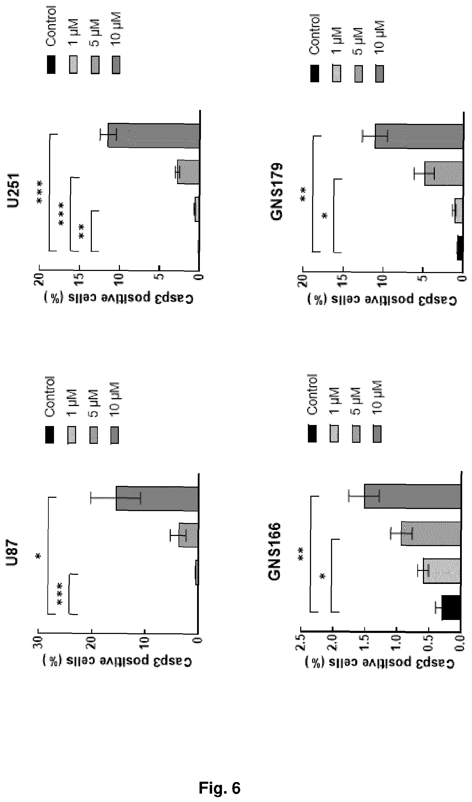

Furthermore, immunofluorescence assays for DDI199 were completed in additional conventional (U251) and patient-derived (GNS166 and GNS179) cell lines. Results demonstrated that DDI199 significantly reduces cell proliferation (Figure 5) and induces apoptosis (Figure 6) in all the tested cell lines, and that in all cases the effect is dose-dependent.

## Claims

1. A compound of formula **I:** 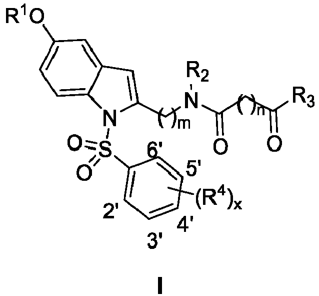 or a pharmaceutical salt thereof, wherein:
R¹ represents H, -C₁₋₄ alkyl, phenyl, benzyl, or Cy₁(C₂₋₄ alkyl);
Cy₁ represents a 5- to 8-membered saturated ring, which contains from 1 to 3 heteroatoms selected from N, O, Se and S; wherein Cy₁ is attached to rest of the molecule through any C or N atom available, and wherein Cy₁ is optionally substituted by one R⁵ group;
R² represents H, Me, CH₂C≡CH, CH₂CH=C=CH₂ or CH₂CH=CH₂;
R³ represents OH, -O C₁₋₄ alkyl, NHOH, NHNR⁶R⁷ or *orto*-NH₂(C₆H₄)NH;
R⁴ represents H, C₁-C₅ alkyl, -O-C₁-C₅ alkyl, -OH, -NO₂ halogen, CN, CO₂Et or
SO₂Ph, wherein R⁴ is located in any available position C2', C3', C4', C5', C6';
R⁵ represents H or -C₁₋₆ alkyl;
R⁶ represents H, -OH or -C₁₋₆ alkyl, preferably H or -C₁₋₄ alkynyl;
R⁷ represents H, -OH or -C₁₋₆ alkyl;
x represents 1, 2, 3 and/or 4;
n represents 2, 3, 4, 5 or 6; and
m represents 0, 1 or 2.

2. A compound of formula **II:** 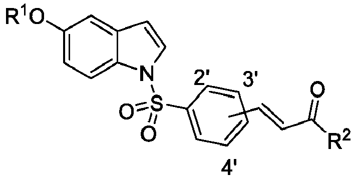 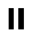 or a pharmaceutical salt thereof, wherein:
R¹ represents -C₁₋₄ alkyl, phenyl, benzyl, or Cy₁(C₂₋₄ alkyl);
Cy₁ represents a 5- to 8-membered saturated ring, which contains from 1 to 3 heteroatoms selected from N, O, Se and S; wherein Cy₁ is attached to rest of the molecule through any C or N atom available, and wherein Cy₁ is optionally substituted by one R³ group;
R² represents -OH, -OC₁₋₄ alkyl, -NHOH, -NHNR⁴R⁵ or *orto*-NH₂(C₆H₄)NH group installed in the α,β-unsaturated group located in any available position C2', C3' or C4' of the molecule;
R³ represents H or -C₁₋₄ alkynyl;
R⁴ represents H, -OH or -C₁₋₆ alkyl; and
R⁵ independently represents H or -C₁₋₆ alkyl.

3. A compound of formula **III:** 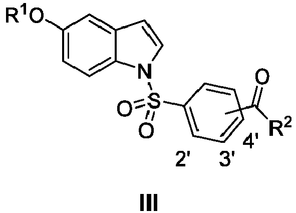 or a pharmaceutical salt thereof, wherein:
R¹ represents -C₁₋₄ alkyl, phenyl, benzyl, or Cy₁(C₂₋₄ alkyl);
Cy₁ represents a 5 to 8-membered saturated ring, which contains from 1 to 3 heteroatoms selected from N, O, Se and S; wherein Cy₁ is attached to rest of the molecule through any C or N atom available, and wherein Cy₁ is optionally substituted by one R³ group;
R² represents -OH, -O C₁₋₄ alkyl, NHOH, NHNR⁴R⁵ or *orto*-NH₂(C₆H₄)NH group installed in the carboxylate group located in any available position C2', C3' or C4' of the molecule;
R³ represents H or -C₁₋₄ alkynyl;
R⁴ represents H, -OH or -C₁₋₆ alkyl; and
R⁵ represents H or -C₁₋₆ alkyl.

4. A compound of formula **IV:** 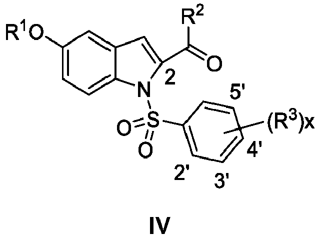 or a pharmaceutical salt thereof, wherein:
R¹ represents -C₁₋₄ alkyl, phenyl, benzyl, or Cy₁(C₂₋₄ alkyl);
Cy₁ represents a 5 to 8-membered saturated ring, which contains from 1 to 3 heteroatoms selected from N, O, Se and S; Cy₁ wherein Cy₁ is attached to rest of the molecule through any C or N atom available, and wherein Cy₁ is optionally substituted by one R⁴ group;
R² represents OH, -OC₁₋₄ alkyl, NHOH, NHNR⁵R⁶ or *orto*-NH₂(C₆H₄)NH group installed in the carboxylic group located in position C2' of the molecule;
each R³ independently represents H; halogen, Me, -C₃H₇, -OMe or -CF₃; or
two R³groups form a fused benzene ring resulting in a 1-naphthyl or 2-naphthyl group;
x represents 1, 2, 3 and/or 4;
R⁴ represents H or -C₁₋₄ alkynyl;
R⁵ represents H or -C₁₋₄ alkynyl; and
R⁶ represents H, -OH or -C₁₋₆ alkyl, preferably R⁶ independently represents H or -C₁₋₆ alkyl.

5. A compound of formula **V:** 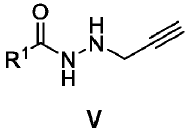 or a pharmaceutical salt thereof, wherein:
R¹ represents 5-hydroxy-1*H*-indol-2-yl, 5-methoxy-1*H*-indol-2-yl, 5-benzyloxy-1*H-*indol-2-yl, 5-(3-(piperidin-1-yl)propoxy)-1*H*-indol-2-yl, 5-(3-(4-(prop-2-yn-1-yl)piperazin-1-yl)propoxy)-1*H*-indol2-yl; C₆H₅, 2'-BrC₆H₄, 3'-BrC₆H₄, 4'-BrC₆H₄; 2'-ClC₆H₄, 3'-ClC₆H₄, 4'-ClC₆H₄; 2'-FC₆H₄, 3'- FC₆H₄, 4'-FC₆H₄; 2'-IC₆H₄, 3'-IC₆H₄, 4'-IC₆H₄; 2'-MeC₆H₄, 3'-MeC₆H₄, 4'-MeC₆H₄; 2'-C₂H₅C₆H₄, 3'-C₂H₅C₆H₄, 4'-C₂H₅C₆H₄; 2'-C₃H₇C₆H₄, 3'-C₃H₇C₆H₄, 4'-C₃H₇C₆H₄; 2'-OMeC₆H₄, 3'-OMeC₆H₄, 4'-OMeC₆H₄; 2'-OHC₆H₄, 3'-OHC₆H₄, 4'-OHC₆H₄; 2',5'-di-FC₆H₃, 3',5'-di-FC₆H₃; 2'-CF₃C₆H₄, 3'-CF₃C₆H₄, 4'-CF₃ C₆H₄; 1-naphthyl, 2-naphthyl; furan, pyrrole, tiophene; pyridine, pyrimidine, pyrazine, C₆H₅NHCO(CH₂)ₙ, 2'-BrC₆H₄NHCO(CH₂)ₙ, 3'-BrC₆H₄NHCO(CH₂)n, 4'-BrC₆H₄ NHCO(CH₂)ₙ; 2'-ClC₆H₄NHCO(CH₂)ₙ, 3'-ClC₆H₄NHCO(CH₂)ₙ, 4'-ClC₆H₄NHCO(CH₂)ₙ; 2'-FC₆H₄NHCO(CH₂)ₙ, 3'-FC₆H₄NHCO(CH₂)ₙ, 4'-FC₆H₄NHCO(CH₂)ₙ; 2'-IC₆H₄NHCO(CH₂)ₙ, 3'-IC₆H₄NHCO(CH₂)ₙ, 4'-IC₆H₄NHCO(CH₂)ₙ; 2'-MeC₆H₄NHCO(CH₂)ₙ, 3'-MeC₆H₄NHCO(CH₂)ₙ, 4'-MeC₆H₄NHCO(CH₂)ₙ; 2'-C₂H₅C₆H₄NHCO(CH₂)ₙ, 3'-C₂H₅C₆H₄NHCO(CH₂)ₙ, 4'-C₂H₅C₆H₄NHCO(CH₂)ₙ; 2'-C₃H₇C₆H₄NHCO(CH₂)ₙ, 3'-C₃H₇C₆H₄NHCO(CH₂)ₙ, 4'-C₃H₇C₆H₄NHCO(CH₂)ₙ; 2'-OMeC₆H₄NHCO(CH₂)ₙ, 3'-OMeC₆H₄NHCO(CH₂)ₙ, 4'-OMeC₆H₄NHCO(CH₂)ₙ; 2'-OHC₆H₄NHCO(CH₂)ₙ, 3'-OHC₆H₄NHCO(CH₂)ₙ, 4'-OHC₆H₄NHCO(CH₂)ₙ; 2',5'-di-FC₆H₃NHCO(CH₂)ₙ, 3',5'-di-FC₆H₃NHCO(CH₂)ₙ; 2'-CF₃C₆H₄NHCO(CH₂)ₙ, 3'-CF₃C₆H₄NHCO(CH₂)ₙ, 4'-CF₃C₆H₄ NHCO(CH₂)ₙ; 1-naphthylNHCO(CH₂)ₙ, 2-naphthyl NHCO(CH₂)ₙ; furanNHCO(CH₂)ₙ, pyrroleNHCO(CH₂)ₙ, tiophenNHCO(CH₂)ₙ; pyridineNHCO(CH₂)ₙ, pyrimidine NHCO(CH₂)ₙ or pyrazineNHCO(CH₂)ₙ; and
n represents 2, 3, 4, 5, 6.

6. a compound of formula **I, II, III, IV** or V selected from:
*N*1-Hydroxy-*N*8-((1-(phenylsulfonyl)-5-(3-(piperidin-1-yl)propoxy)-1*H*-indol-2-yl)methyl)-*N*8-(prop-2-yn-1-yl)octanediamide (**DDI199**);
1-((3-Bromophenyl)sulfonyl)-5-methoxy-1*H*-indole (**MTP21**);
5-(Benzyloxy)-1-((3-bromophenyl)sulfonyl)-1*H*-indole (**MTP12**);
1-((3-Bromophenyl)sulfonyl)-5-(3-(piperidin-1-yl)propoxy)-1*H*-indole (**MTP30**);
1-((3-Bromophenyl)sulfonyl)-5-(3-(4-(prop-2-yn-1-yl)piperazin-1-yl)propoxy)-1*H-*indole (**MTP43**);
Ethyl (*E*)-3-(3-((5-methoxy-1*H*-indol-1-yl)sulfonyl)phenyl)acrylate (**MTP25**);
Ethyl (*E*)-3-(3-((5-(benzyloxy)-1-*H*-indol-1-yl)sulfonyl)phenyl)acrylate (**MTP13**);
Ethyl (*E*)-3-(3-((5-(3-(Piperidin-1**-**yl)propoxy)-1*H*-indol-1-yl)sulfonyl)phenyl)acrylate (**MTP41**);
Ethyl (*E*)-3-(4-((5-Methoxy-1H-indol-1-yl)sulfonyl)phenyl)acrylate (**SMD2**);
Ethyl (*E*)-3-(4-((5-(3-(Piperidin-1**-**yl)propoxy)-1*H*-indol-1-yl)sulfonyl)phenyl)acrylate (**APP13**);
(*E*)-3-(3-((5-Methoxy-1*H*-indol-1-yl)sulfonyl)phenyl)acrylic acid (**MTP37**);
(*E*)-3-(3-((5-(Benzyloxy)-1*H*-indol-1-yl)sulfonyl)phenyl)acrylic acid (**MTP153**);
(*E*)-3-(3-((5-(3-(Piperidin-1-yl)propoxy)-1*H*-indol-1-yl)sulfonyl)phenyl)acrylic acid (**MTP146**);
(E)-3-(4-((5-Methoxy-1*H*-indol-1-yl)sulfonyl)phenyl)acrylic acid (**SMD3**);
(*E*)-3-(4-((5-(3-(Piperidin-1-yl)propoxy)-1*H*-indol-1-yl)sulfonyl)phenyl)acrylic acid (**APP15**);
(*E*)-*N*-Hydroxy-3-(3-((5-methoxy-1*H*-indol-1-yl)sulfonyl)phenyl)acrylamide (**MTP142**);
(*E*)-3-(3-((5-(Benzyloxy)-1*H*-indol-1-yl)sulfonyl)phenyl)-*N*-hydroxyacrylamide (**MTP156**);
(*E*)-*N*-Hydroxy-3-(3-((5-(3-(piperidin-1-yl)propoxy)-1*H*-indol-1-yl)sulfonyl)phenyl)acrylamide (**MTP150**);
(*E*)-*N*-Hydroxy-3-(4-((5-methoxy-1*H*-indol-1-yl)sulfonyl)phenyl)acrylamide (**SMD17**);
(*E*)-*N*-Hydroxy-3-(4-((5-(3-(piperidin-1-yl)propoxy)-1*H*-indol-1-yl)sulfonyl)phenyl)acrylamide (**APP19**);
(*E*)-*N*-(2-Aminophenyl)-3-(3-((5-methoxy-1*H-*indol-1-yl)sulfonyl)phenyl)acrylamide (**MTP143**);
(*E*)-*N*-(2-Aminophenyl)-3-(3-((5-(benzyloxy)-1*H-*indol-1-yl)sulfonyl)phenyl)acrylamide (**MTP157**);
(*E*)-*N*-(2-Aminophenyl)-3-(3-((5-(3-(piperidin-1-yl)propoxy)-1H-indol-1-yl)sulfonyl)phenyl)acrylamide (**MTP167**);
(*E*)-*N*-(2-Aminophenyl)-3-(4-((5-methoxy-1*H-*indol-1-yl)sulfonyl)phenyl)acrylamide (**SMD10**);
(*E*)-*N*-(2-Aminophenyl)-3-(4-((5-(3-(piperidin-1-yl)propoxy)-1*H*-indol-1-yl)sulfonyl)phenyl)acrylamide (**APP17**);
Methyl 4-((5-methoxy-1*H-*indol-1-yl)sulfonyl)benzoate (**MTP21**);
Methyl 4-((5-(benzyloxy)-1*H*-indol-1-yl)sulfonyl)benzoate (**MTP12**);
Methyl 4-((5-(3-(piperidin-1-yl)propoxy)-1*H*-indol-1-yl)sulfonyl)benzoate (**MTP30**);
Methyl 4-((5-(3-(4-(prop-2-yn-1-yl)piperazin-1-yl)propoxy)-1*H-*indol-1-yl)sulfonyl) benzoate (**MTP43**);
4-((5-Methoxy-1*H*-indol-1-yl)sulfonyl)benzohydrazide (**MTP86**);
4-((5-(Benzyloxy)-1*H*-indol-1-yl)sulfonyl)benzohydrazide (**MTP90**);
4-((5-(3-(Piperidin-1-yl)propoxy)-1*H*-indol-1-yl)sulfonyl)benzohydrazide (**MTP96**);
4-((5-(3-(4-(Prop-2-yn-1-yl)piperazin-1-yl)propoxy)-1*H*-indol-1-yl)sulfonyl)benzohydrazide (**MTP99**);
4-((5-Methoxy-1*H*-indol-1-yl)sulfonyl)-*N'*-propylbenzohydrazide (**FRB24**);
4-((5-(3-(Piperidin-1-yl)propoxy)-1*H*-indol-1-yl)sulfonyl)-*N'*-propylbenzohydrazide (**FRB44**);
4-((5-(3-(4-(Prop-2-yn-1-yl)piperazin-1-yl)propoxy)-1*H*-indol-1-yl)sulfonyl)-*N'-*propylbenzohydrazide (**FRB56**);
4-((5-Methoxy-1*H*-indol-1-yl)sulfonyl)-*N*',*N*'-dipropylbenzohydrazide (**FRB21**);
*N*-Hydroxy-4-((5-methoxy-1*H*-indol-1-yl)sulfonyl)benzamide (**MTP89**);
4-((5-(Benzyloxy)-1*H*-indol-1-yl)sulfonyl)-*N*-hydroxybenzamide (**MTP98**);
*N*-Hydroxy-4-((5-(3-(piperidin-1-yl)propoxy)-1*H*-indol-1-yl)sulfonyl)benzamide (**MTP100**);
*N*-Hydroxy-4-((5-(3-(4-(prop-2-yn-1-yl)piperazin-1-yl)propoxy)-1*H-*indol-1-yl) sulfonyl)benzamide (**MTP109**);
4-((5-Methoxy-1*H*-indol-1-yl)sulfonyl)benzoic acid (**MTP105**);
4-((5-(Benzyloxy)-1*H*-indol-1-yl)sulfonyl)benzoic (**MTP148**);
4-((5-(3-(Piperidin-1-yl)propoxy)-1*H*-indol-1-yl)sulfonyl)benzoic acid (**MTP162**);
4-((5-(3-(4-(Prop-2-yn-1-yl)piperazin-1-yl)propoxy)-1*H*-indol-1-yl)sulfonyl)benzoic acid (**MTP194**);
3-((5-(3-(Piperidin-1-yl)propoxy)-1*H*-indol-1-yl)sulfonyl)benzoic acid (**APP35**);
3-((5-(3-(4-(Prop-2-yn-1-yl)piperazin-1-yl)propoxy)-1*H*-indol-1-yl)sulfonyl)benzoic acid (**APP37**);
*N*-(2-Aminophenyl)-4-((5-(benzyloxy)-1*H*-indol-1-yl)sulfonyl)benzamide (**MTP155**);
*N*-(2-Aminophenyl)-4-((5-(3-(piperidin-1-yl)propoxy)-1*H*-indol-1-yl)sulfonyl)benzamide (**MTP165**);
*N*-(2-Aminophenyl)-4-((5-(3-(4-(prop-2-yn-1-yl)piperazin-1-yl)propoxy)-1*H*-indol-1-yl)sulfonyl)benzamide (**MTP185**);
Ethyl 5-(benzyloxy)-1-(phenylsulfonyl)-1*H*-indole-2-carboxylate (**MTP127**);
Ethyl 1-(phenylsulfonyl)-5-(3-(piperidin-1-yl)propoxy)-1*H-*indole-2-carboxylate (**MTP132**);
5-(Benzyloxy)-1-(phenylsulfonyl)-1*H*-indole-2-carbohydrazide (**MTP128**);
1-(Phenylsulfonyl)-5-(3-(piperidin-1-yl)propoxy)-1*H*-indole-2-carbohydrazide (**MTP133**);
1-(Phenylsulfonyl)-5-(3-(4-(prop-2-yn-1-yl)piperazin-1-yl)propoxy)-1*H*-indole-2-carbohydrazide (**MTP137**);
5-(Benzyloxy)-1-(phenylsulfonyl)-1*H-*indole-2-carboxylic acid (**MTP136**);
1-(Phenylsulfonyl)-5-(3-(piperidin-1-yl)propoxy)-1*H*-indole-2-carboxylic acid (**MTP186**);
1-(Phenylsulfonyl)-5-(3-(4-(prop-2-yn-1-yl)piperazin-1-yl)propoxy)-1*H*-indole-2-carboxylic acid (**MTP191**);
5-(Benzyloxy)-*N*-hydroxy-1-(phenylsulfonyl)-1*H*-indole-2-carboxamide (**MTP172**);
*N*-(2-Aminophenyl)-5-(benzyloxy)-1-(phenylsulfonyl)-1*H*-indole-2-carboxamide (**MTP140**);
*N*-(2-Aminophenyl)-1-(phenylsulfonyl)-5-(3-(piperidin-1-yl)propoxy)-1*H*-indole-2-carboxamide (**MTP209**);
*N*-(2-Aminophenil)-1-(phenylsulfonyl)-5-(3-(4-(prop-2-yn-1-yl)piperazin-1-yl)propoxy)-1*H-*indole-2-carboxamide (**MTP192**);
*N'*-(Prop-2-yn-1-yl)benzohydrazide (**MTP230**);
5-(Benzyloxy)-*N'*-(prop-2-yn-1-yl)-1*H-*indole-2-carbohydrazide (**MTP229**); and
8-Oxo-*N*-phenyl-8-(2-(prop-2-yn-1-yl)hydrazineyl)octanamide (**MTP234**).

7. A pharmaceutical composition which comprises a compound of formula **I, II, III, IV, V** as defined in any of claims 1 to 6 or a pharmaceutically acceptable salt thereof and one or more pharmaceutically acceptable excipients.

8. A compound of formula **I, II, III, IV** or **V** according to any of claims 1 to 6 or to a pharmaceutical salt thereof, for use as a medicament.

9. A compound of formula **I, II, III, IV** or **V** according to any of claims 1 to 6 or to a pharmaceutical salt thereof, for use in the treatment of cancer.

10. The compound of formula **I, II, III, IV** or **V** or a pharmaceutical salt thereof for the use according to claim 9, wherein the cancer is selected from glioblastoma, pancreatic, colorectal, lung, skin, gastric , breast, liver, prostate, head, and neck.

11. Use of a compound of formula **I, II, III, IV** or **V** or a pharmaceutical composition thereof as defined above, for identifying and evaluating in a robotic manner other compounds which may be useful as a medicament.
